# EUROPEAN PATENT SPECIFICATION

(11) **EP 2 611 466 B1**
(45) Date of publication and mention of the grant of the patent: **12.06.2019**
(21) Application number: 11845534.4
(22) Date of filing: 30.08.2011
(51) Int. Cl.: A61K 47/50, A61K 9/16, A61K 9/51, A61K 38/49, A61P 9/10, A61P 7/02, A61P 7/04

(54) **SHEAR CONTROLLED RELEASE OF THROMBOLYTIC THERAPIES FOR STENOTIC LESIONS**
SCHERUNGSGESTEUERTE FREISETZUNG VON THROMBOLYTISCHEN THERAPIEN FÜR STENOSELÄSIONEN
LIBÉRATION CONTRÔLÉE PAR CISAILLEMENT DE TRAITEMENTS THROMBOLYTIQUES POUR LÉSIONS STÉNOSÉES

(30) Priority: 30.08.2010 US 378057 P; 25.04.2011 US 201161478700 P
(43) Date of publication of application: 10.07.2013
(73) Proprietor: President and Fellows of Harvard College, Cambridge, MA 02138 (US)
(72) Inventor: INGBER, Donald, E., Boston, MA 02118 (US); KORIN, Netanel, Brookline, MA 02246 (US); KANAPATHIPILLAI, Mathumai, Boston, MA 02215 (US)
(74) Representative: Moore, Michael Richard
(86) International application number: PCT/US2011/049691
(87) International publication number: WO 2012/074588

(56) References cited:
- WO-A2-2004/091571
- WO-A2-2009/073193
- US-A1- 2003 082 237
- US-A1- 2007 172 653
- US-A1- 2007 172 653
- US-A1- 2008 317 794
- US-A1- 2010 055 187
- US-A1- 2010 203 142
- US-B1- 6 797 342
- CHUNG ET AL: "Accelerating thrombolysis with chitosan-coated plasminogen activators encapsulated in poly-(lactide-co-glycolide) (PLGA) nanoparticles", BIOMATERIALS, ELSEVIER SCIENCE PUBLISHERS BV., BARKING, GB, vol. 29, no. 2, 22 October 2007 (2007-10-22), pages 228-237, XP022323373, ISSN: 0142-9612, DOI: 10.1016/J.BIOMATERIALS.2007.09.027
- N. KORIN ET AL: "Shear-Activated Nanotherapeutics for Drug Targeting to Obstructed Blood Vessels", SCIENCE, vol. 337, no. 6095, 10 August 2012 (2012-08-10), pages 738-742, XP55179213, ISSN: 0036-8075, DOI: 10.1126/science.1217815
- E. LAVIK ET AL: "Leveraging Shear Stress to Bust Clots with Nanoparticles", SCIENCE, vol. 337, no. 6095, 9 August 2012 (2012-08-09), pages 658-659, XP055273605, US ISSN: 0036-8075, DOI: 10.1126/science.1227097

### SEQUENCE LISTING

<110> INGBER, DONALD E. KORIN, NETANEL KANAPATHIPILLAI, MATHUMAI
<120> SHEAR CONTROLLED RELEASE FOR STENOTIC LESIONS AND THROMBOLYTIC THERAPIES
<130> 002806-068233-US
<140> 13/816,763
   <141> 2013-02-13
<150> PCT/US2011/049691
   <151> 2011-08-30
<150> 61/478,700
   <151> 2011-04-25
<150> 61/378,057
   <151> 2010-08-30
<160> 30
<170> PatentIn version 3.5
<210> 1
   <211> 5
   <212> PRT
   <213> Artificial Sequence
<220>
   <223> Description of Artificial Sequence: Synthetic peptide
<400> 1
   Cys Arg Glu Lys Ala
   1               5
<210> 2
   <211> 9
   <212> PRT
   <213> Artificial Sequence
<220>
   <223> Description of Artificial Sequence: Synthetic peptide
<400> 2
   Cys Arg Lys Arg Leu Asp Arg Asn Lys
   1               5
<210> 3
   <211> 9
   <212> PRT
   <213> Artificial Sequence
<220>
   <223> Description of Artificial Sequence: Synthetic peptide
<400> 3
   Cys His Val Leu Trp Ser Thr Arg Cys
   1               5
<210> 4
   <211> 15
   <212> PRT
   <213> Artificial Sequence
<220>
   <223> Description of Artificial Sequence: Synthetic peptide
<400> 4
   Ala Leu Glu Ala Leu Ala Glu Ala Leu Glu Ala Leu Ala Glu Ala
   1               5                   10                  15
<210> 5
   <211> 10
   <212> PRT
   <213> Artificial Sequence
<220>
   <223> Description of Artificial Sequence: Synthetic peptide
<400> 5
   Lys Phe Phe Lys Phe Phe Lys Phe Phe Lys
   1               5                   10
<210> 6
   <211> 29
   <212> PRT
   <213> Artificial Sequence
<220>
   <223> Description of Artificial Sequence: Synthetic peptide
<400> 6
   Ala Ala Leu Glu Ala Leu Ala Glu Ala Leu Glu Ala Leu Ala Glu Ala
   1               5                   10                  15
   Leu Glu Ala Leu Ala Glu Ala Ala Ala Ala Gly Gly Cys
               20                  25
<210> 7
   <211> 29
   <212> PRT
   <213> Artificial Sequence
<220>
   <223> Description of Artificial Sequence: Synthetic peptide
<400> 7
   Ala Leu Ala Glu Ala Leu Ala Glu Ala Leu Ala Glu Ala Leu Ala Glu
   1               5                   10                  15
   Ala Leu Ala Glu Ala Leu Ala Ala Ala Ala Gly Gly Cys
               20                  25
<210> 8
   <211> 22
   <212> PRT
   <213> Artificial Sequence
<220>
   <223> Description of Artificial Sequence: Synthetic peptide
<400> 8
   Gly Leu Phe Glu Ala Ile Glu Gly Phe Ile Glu Asn Gly Trp Glu Gly
   1               5                   10                  15
   Met Ile Trp Asp Tyr Gly
               20
<210> 9
   <211> 23
   <212> PRT
   <213> Artificial Sequence
<220>
   <223> Description of Artificial Sequence: Synthetic peptide
<400> 9
   Gly Leu Phe Gly Ala Ile Ala Gly Phe Ile Glu Asn Gly Trp Glu Gly
   1               5                   10                  15
   Met Ile Asp Gly Trp Tyr Gly
               20
<210> 10
   <211> 48
   <212> PRT
   <213> Artificial Sequence
<220>
   <223> Description of Artificial Sequence: Synthetic polypeptide
<400> 10
   Gly Leu Phe Glu Ala Ile Glu Gly Phe Ile Glu Asn Gly Trp Glu Gly
   1               5                   10                  15
   Met Ile Asp Gly Trp Tyr Gly Cys Gly Leu Phe Glu Ala Ile Glu Gly
               20                  25                  30
   Phe Ile Glu Asn Gly Trp Glu Gly Met Ile Asp Gly Trp Tyr Gly Cys
           35                  40                  45
<210> 11
   <211> 44
   <212> PRT
   <213> Artificial Sequence
<220>
   <223> Description of Artificial Sequence: Synthetic polypeptide
<400> 11
   Gly Leu Phe Glu Ala Ile Glu Gly Phe Ile Glu Asn Gly Trp Glu Gly
   1               5                   10                  15
   Met Ile Asp Gly Gly Cys Gly Leu Phe Glu Ala Ile Glu Gly Phe Ile
               20                  25                  30
   Glu Asn Gly Trp Glu Gly Met Ile Asp Gly Gly Cys
           35                  40
<210> 12
   <211> 35
   <212> PRT
   <213> Artificial Sequence
<220>
   <223> Description of Artificial Sequence: Synthetic polypeptide
<400> 12
   Gly Leu Phe Gly Ala Leu Ala Glu Ala Leu Ala Glu Ala Leu Ala Glu
   1               5                   10                  15
   His Leu Ala Glu Ala Leu Ala Glu Ala Leu Glu Ala Leu Ala Ala Gly
               20                  25                  30
   Gly Ser Cys
           35
<210> 13
   <211> 34
   <212> PRT
   <213> Artificial Sequence
<220>
   <223> Description of Artificial Sequence: Synthetic polypeptide
<400> 13
   Gly Leu Phe Glu Ala Ile Glu Gly Phe Ile Glu Asn Gly Trp Glu Gly
   1               5                   10                  15
   Leu Ala Glu Ala Leu Ala Glu Ala Leu Glu Ala Leu Ala Ala Gly Gly
               20                  25                  30
   Ser Cys
<210> 14
   <211> 41
   <212> PRT
   <213> Artificial Sequence
<220>
   <223> Description of Artificial Sequence: Synthetic polypeptide
<220>
   <221> MOD_RES
   <222> (17)..(17)
   <223> Norleucine
<220>
   <221> MOD_RES
   <222> (38)..(38)
   <223> Norleucine
<400> 14
   Gly Leu Phe Glu Ala Ile Glu Gly Phe Ile Glu Asn Gly Trp Glu Gly
   1               5                   10                  15
   Leu Ile Asp Gly Lys Gly Leu Phe Glu Ala Ile Glu Gly Phe Ile Glu
               20                  25                  30
   Asn Gly Trp Glu Gly Leu Ile Asp Gly
           35                  40
<210> 15
   <211> 16
   <212> PRT
   <213> Artificial Sequence
<220>
   <223> Description of Artificial Sequence: Synthetic peptide
<400> 15
   Arg Gln Ile Lys Ile Trp Phe Gln Asn Arg Arg Met Lys Trp Lys Lys
   1               5                   10                  15
<210> 16
   <211> 14
   <212> PRT
   <213> Human immunodeficiency virus
<400> 16
   Gly Arg Lys Lys Arg Arg Gln Arg Arg Arg Pro Pro Gln Cys
   1               5                   10
<210> 17
   <211> 27
   <212> PRT
   <213> Artificial Sequence
<220>
   <223> Description of Artificial Sequence: Synthetic peptide
<400> 17
   Gly Ala Leu Phe Leu Gly Trp Leu Gly Ala Ala Gly Ser Thr Met Gly
   1               5                   10                  15
   Ala Trp Ser Gln Pro Lys Lys Lys Arg Lys Val
               20                  25
<210> 18
   <211> 18
   <212> PRT
   <213> Artificial Sequence
<220>
   <223> Description of Artificial Sequence: Synthetic peptide
<400> 18
   Leu Leu Ile Ile Leu Arg Arg Arg Ile Arg Lys Gln Ala His Ala His
   1               5                   10                  15
   Ser Lys
<210> 19
   <211> 25
   <212> PRT
   <213> Artificial Sequence
<220>
   <223> Description of Artificial Sequence: Synthetic peptide
<400> 19
   Trp Thr Leu Asn Ser Ala Gly Tyr Leu Leu Lys Ile Asn Leu Lys Ala
   1               5                   10                  15
   Leu Ala Ala Leu Ala Lys Lys Ile Leu
               20                  25
<210> 20
   <211> 18
   <212> PRT
   <213> Artificial Sequence
<220>
   <223> Description of Artificial Sequence: Synthetic peptide
<400> 20
   Lys Leu Ala Leu Lys Leu Ala Leu Lys Ala Leu Lys Ala Ala Leu Lys
   1               5                   10                  15
   Leu Ala
<210> 21
   <211> 9
   <212> PRT
   <213> Artificial Sequence
<220>
   <223> Description of Artificial Sequence: Synthetic peptide
<400> 21
   Arg Arg Arg Arg Arg Arg Arg Arg Arg
   1               5
<210> 22
   <211> 37
   <212> PRT
   <213> Artificial Sequence
<220>
   <223> Description of Artificial Sequence: Synthetic polypeptide
<400> 22
   Leu Leu Gly Asp Phe Phe Arg Lys Ser Lys Glu Lys Ile Gly Lys Glu
   1               5                   10                  15
   Phe Lys Arg Ile Val Gln Arg Ile Lys Asp Phe Leu Arg Asn Leu Val
               20                  25                  30
   Pro Arg Thr Glu Ser
           35
<210> 23
   <211> 31
   <212> PRT
   <213> Artificial Sequence
<220>
   <223> Description of Artificial Sequence: Synthetic polypeptide
<400> 23
   Ser Trp Leu Ser Lys Thr Ala Lys Lys Leu Glu Asn Ser Ala Lys Lys
   1               5                   10                  15
   Arg Ile Ser Glu Gly Ile Ala Ile Ala Ile Gln Gly Gly Pro Arg
               20                  25                  30
<210> 24
   <211> 30
   <212> PRT
   <213> Artificial Sequence
<220>
   <223> Description of Artificial Sequence: Synthetic polypeptide
<400> 24
   Ala Cys Tyr Cys Arg Ile Pro Ala Cys Ile Ala Gly Glu Arg Arg Tyr
   1               5                   10                  15
   Gly Thr Cys Ile Tyr Gln Gly Arg Leu Trp Ala Phe Cys Cys
               20                  25                  30
<210> 25
   <211> 36
   <212> PRT
   <213> Artificial Sequence
<220>
   <223> Description of Artificial Sequence: Synthetic polypeptide
<400> 25
   Asp His Tyr Asn Cys Val Ser Ser Gly Gly Gln Cys Leu Tyr Ser Ala
   1               5                   10                  15
   Cys Pro Ile Phe Thr Lys Ile Gln Gly Thr Cys Tyr Arg Gly Lys Ala
               20                  25                  30
   Lys Cys Cys Lys
           35
<210> 26
   <211> 42
   <212> PRT
   <213> Artificial Sequence
<220>
   <223> Description of Artificial Sequence: Synthetic polypeptide
<220>
   <223> C-term NH2
<400> 26
   Arg Arg Arg Pro Arg Pro Pro Tyr Leu Pro Arg Pro Arg Pro Pro Pro
   1               5                   10                  15
   Phe Phe Pro Pro Arg Leu Pro Pro Arg Ile Pro Pro Gly Phe Pro Pro
               20                  25                  30
   Arg Phe Pro Pro Arg Phe Pro Gly Lys Arg
           35                  40
<210> 27
   <211> 13
   <212> PRT
   <213> Artificial Sequence
<220>
   <223> Description of Artificial Sequence: Synthetic peptide
<220>
   <223> C-term NH2
<400> 27
   Ile Leu Pro Trp Lys Trp Pro Trp Trp Pro Trp Arg Arg
   1               5                   10
<210> 28
   <211> 16
   <212> PRT
   <213> Artificial Sequence
<220>
   <223> Description of Artificial Sequence: Synthetic peptide
<400> 28
   Ala Ala Val Ala Leu Leu Pro Ala Val Leu Leu Ala Leu Leu Ala Pro
   1               5                   10                  15
<210> 29
   <211> 11
   <212> PRT
   <213> Artificial Sequence
<220>
   <223> Description of Artificial Sequence: Synthetic peptide
<400> 29
   Ala Ala Leu Leu Pro Val Leu Leu Ala Ala Pro
   1               5                   10
<210> 30
   <211> 12
   <212> PRT
   <213> Artificial Sequence
<220>
   <223> Description of Artificial Sequence: Synthetic peptide
<400> 30
   Arg Lys Cys Arg Ile Val Val Ile Arg Val Cys Arg
   1               5                   10

## Description

### FIELD OF THE INVENTION

The present invention relates to compositions and uses thereof in methods for treating or imaging stenosis, stenotic lesions, and thrombolytic therapies. The invention also relates to compositions and methods for treating or imaging internal hemorrhage.

### BACKGROUND OF THE INVENTION

Selective delivery of drugs to defined sites of disease is one of the most promising advantages of nanoscaled drug carriers. Targeting of drugs and imaging agents is based on utilizing abnormal features of disease state such as: elevated pH in tumor, enhanced blood vessel permeability in cancer, decreased oxygen level in hypoxic regions, up-regulated cell surface antigens or molecular affinity of targeting moieties to pathological tissue. Based on these characteristics, different drug delivery schemes have been developed. Physical forces play a major role in tissue functionality and disease, however, targeting strategies based on such parameters have not been proposed.

Fluid shear stress is an important physiological feature of the blood circulation that is tightly regulated under normal physiological conditions. Shear stress has been shown to play a major role in regulating endothelial cell phenotype and gene expression, platelet and red blood cell (RBC) aggregation, arteriogenesis and hemodynamic properties. Stenosis, abnormal narrowing in blood vessels due to blockage, constriction or malformation, significantly alters the characteristics of local blood flow; differing this region from normal physiological conditions. For example, wall shear stress at atherosclerotic stenotic sites may be two orders of magnitude higher than normal physiological shear stress levels. These abnormal shear stresses induce platelet activation and facilitate thrombus formation.

As a driving force, shear can cause morphological and structural changes in single and collective elements at varying length scales. The interaction between shear stress and different forms of potential drug carriers including: nano/microspheres, microcapsules and microgels have been extensively studied. As shear increases single particles deform and eventually break. Shear triggered breakup of microcapsule/nanocapsule is being successfully employed in cosmetic products for active ingredient release upon rubbing against the skin. However, these or alternative approaches have not been suggested or developed for targeted drug delivery to sites of stenosis within the vasculature or other fluid filled channels in the body. US-A-2007/172 653 describes nanoclusters comprising a plurality of nanoparticles which disperse in response to an environmental cue, which can be shearing, for the delivery of active agents to a specific targeted site. Chung et al. discuss in Biomaterials, 29(2), 2007, 228-237, that tPA encapsulated in PLGA nanoparticles, which are optionally coated, accelerates thrombolysis in a clot-occluded tube model. The electrostatic forces between carriers and blood clots allow the nanoparticles to permeate into an intra-blood clot and to release tPA therein.

### SUMMARY OF THE INVENTION

In one aspect, the invention provides a pharmaceutical composition for use in the treatment of a stenosis and/or a stenotic lesion, and/or an occlusive lesion in a subject comprising an aggregate, the aggregate comprising a plurality of nanoparticles, the nanoparticles comprising copolymers of polylactic acid and polyglycolic acid, wherein the aggregate disaggregates above a predetermined shear stress when exposed to said predetermined shear stress at the site of stenosis or occlusion; and the aggregate further comprises a molecule which is a therapeutic agent or pharmaceutically acceptable salt thereof, comprising an antithrombotic and/or thrombolytic agent, and/or a vasodilator.

In another aspect, the invention provides the use of a pharmaceutical composition for the imaging of a stenosis and/or a stenotic lesion, and/or an occlusive lesion, wherein the composition comprises an aggregate, the aggregate comprises a plurality of nanoparticles, the nanoparticles comprise copolymers of polylactic acid and polyglycolic acid, and wherein the aggregate disaggregates above a predetermined shear stress when exposed to said predetermined shear stress at the site of stenosis or occlusion; and
the aggregate further comprises a molecule which is an imaging agent.

The disclosure also provides a method for treating or imaging a blood clot and/or an obstructive lesion in a subject, the method comprising administering to a subject in need thereof an aggregate described herein.

The disclosure also provides a method for treating or imaging internal hemorrhage in a subject, the method comprising administering to a subject in need thereof an aggregate described herein.

The disclosure also provides a kit comprising an aggregate herein or components for making an aggregate described herein.

### BRIEF DESCRIPTION OF THE DRAWINGS

This patent or application file contains at least one drawing executed in color.

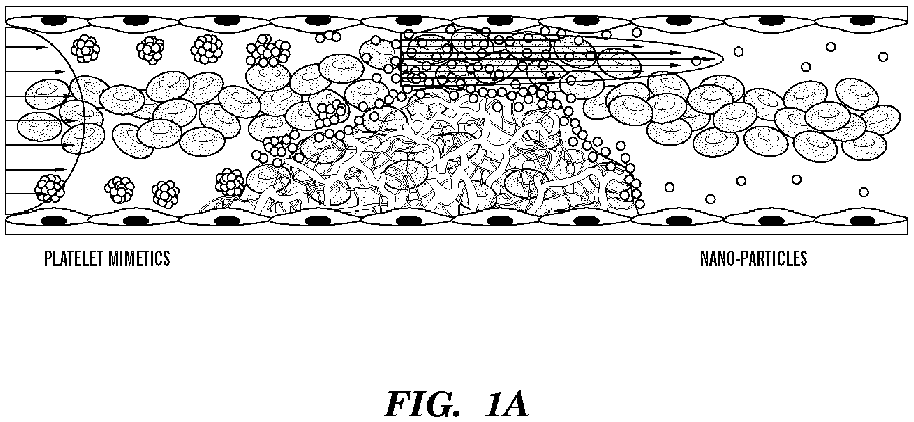

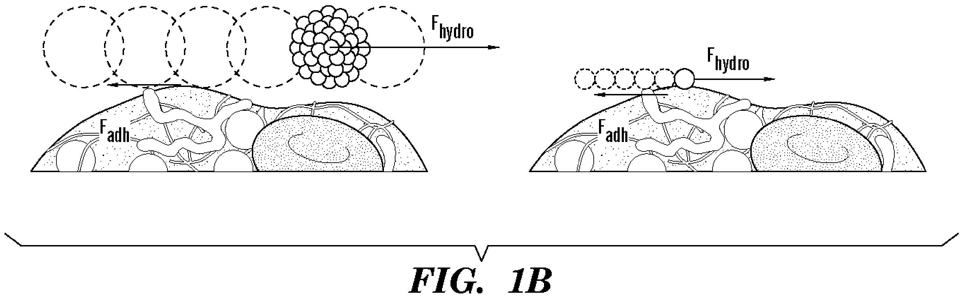

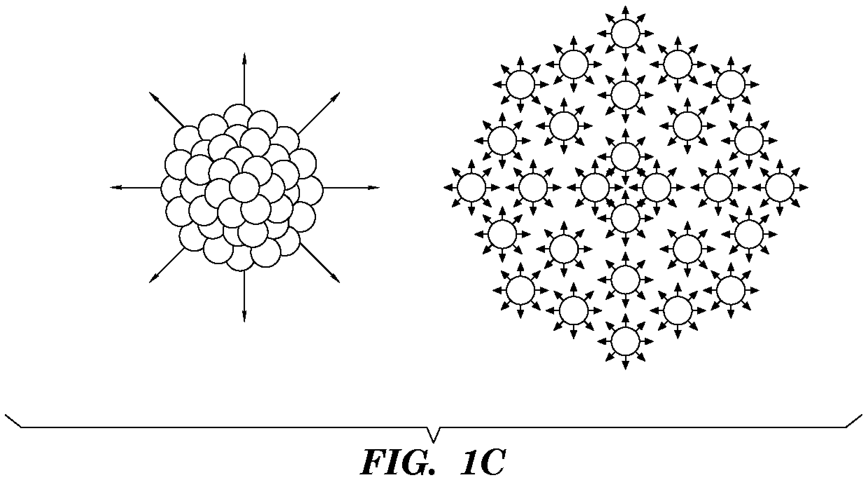

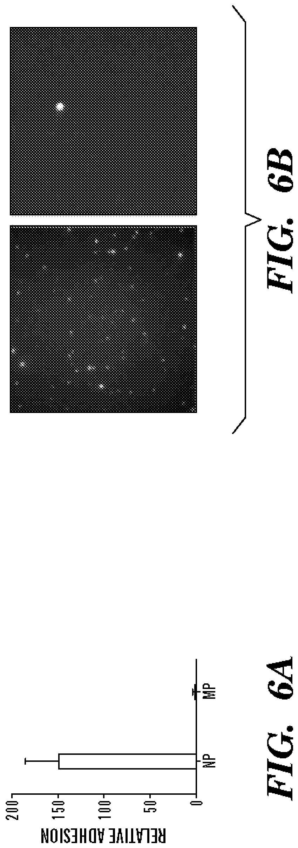

**Figs. 1A-1C** are schematics showing the principle of targeting a stenotic region based on shear disaggregation. **Fig. 1A** shows that shear stress (τ) is a strong function of the vessel diameter (*d*) under Hagen-Poiseuille flow conditions (τ ≈ *d³*), and thus, stenotic or obstructed regions create abnormally high levels of shear stress that are orders of magnitude greater than any normal shear stress. This elevated shear stress physically breaks apart the micron-sized aggregates into their NP components. **Fig. 1B****,** shows that the released NPs experience lower hemodynamic forces (F_{hydro}) due to their smaller size (*F_{hydro}* ≈ *r²*) compared to micron-scale particles, causing them to settle more quickly and adhere more efficiently to the surrounding vascular wall and surface endothelium, while the larger particles are dragged away by fluid flow (also see **Figs. 6A and 6B****).** **Fig. 1C** shows that when the microscale aggregates break up into multiple NPs, the total exposed surface area increases significantly and this can further enhance release or activity of drugs or enzymes that are immobilized on the NPs at the targeting site.

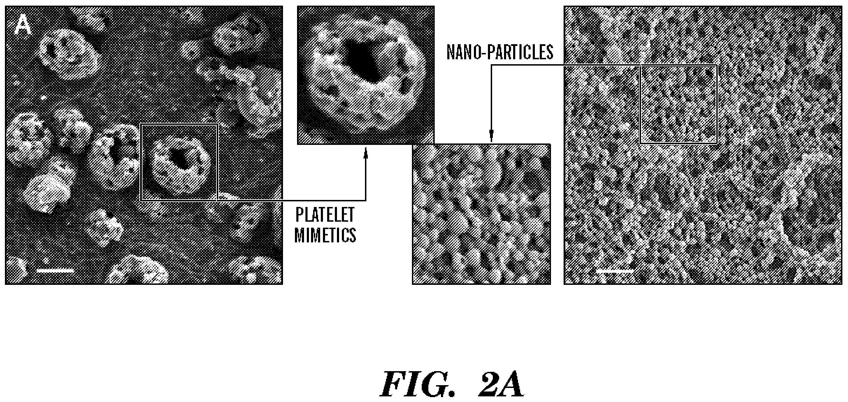

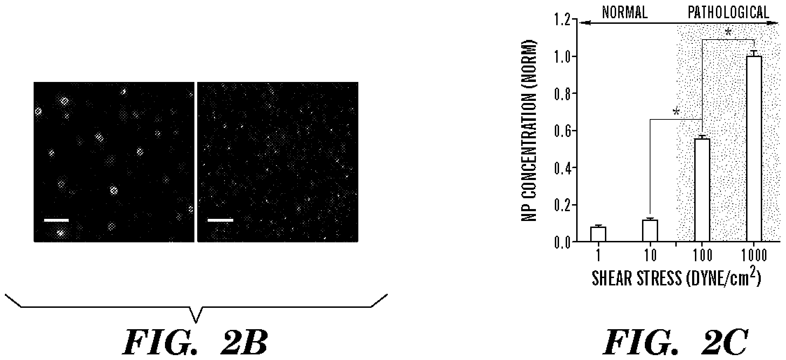

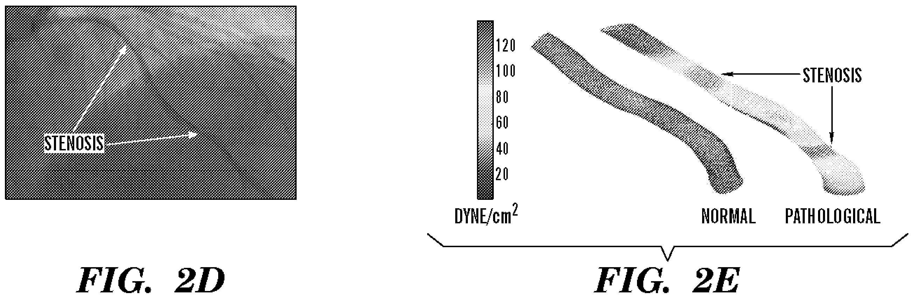

**Figs. 2A -2E** show that microscale platelet mimetics only disperse into nanoparticle aggregates when exposed to pathological shear stresses. **Fig 2A** is a scanning electron micrographs of the microscale (∼2-5 µm) platelet mimetics (left) and the PLGA nanoparticles (∼180 nm) used to produce them (right) (bar, 2 µm) **Fig. 2B** are a fluorescence micrographs demonstrating intact platelet mimetics (left) and nanoparticles dispersed after their exposure to 1,000 dyne/cm² for 10 min using a rheometer (right) (bar, 10 µm). **Fig. 2C** is a bar graph showing quantification of release of fluorescent NPs from the platelet mimetics as a function of shear revealed that exposure to pathological levels of shear (≥100 dyne/cm² for 1 min) caused significant (**p* <0.005) breakup of the microscale aggregates compared to physiological levels of shear (1 or 10 dyne/cm²). **Fig. 2D** is an angiogram of a stenotic left coronary artery in a 63 year old male patient. **Fig. 2E** shows computational fluid dynamics (CFD) simulation comparing flow distributions in a normal coronary artery (left) and the stenotic vessel with a 60% lumen obstruction shown in **Fig. 2D****;** geometries were reconstructed based on intravascular ultrasound scans. Note that the elevated wall shear stress levels (>100 dyne/cm²) in the stenotic regions of the vessel are similar to those required to induced release of NPs from the platelet mimetics shown in **Fig. 2C****.**

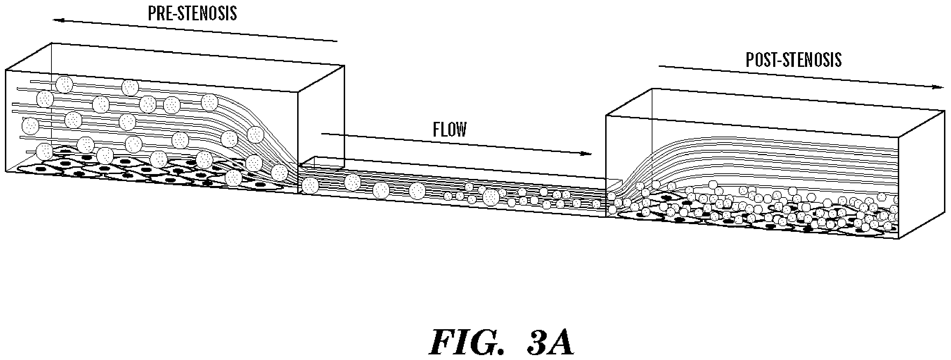

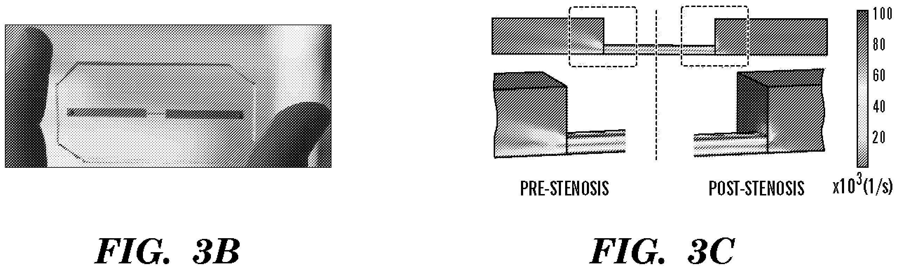

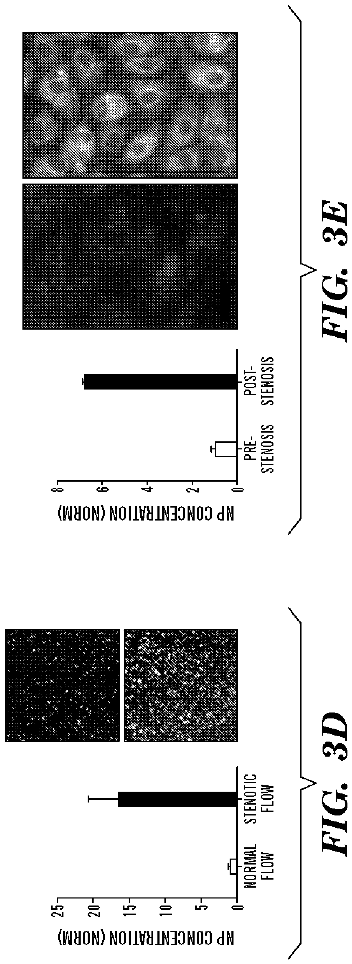

**Figs. 3A-3E** show shear-induced dispersion of platelet mimetics and nanoparticle targeting under hemodynamic conditions in microfluidic devices. **Fig. 3Ab** is a schematic representation of a microfluidic vascular stenosis model. Platelet mimetics (large spheres) are infused through a microfluidic channel that constricts by 90% in cross-sectional area in the central region, mimicking vascular stenosis. The bottom of the channel is lined by a monolayer of cultured bovine arterial endothelial cells. Platelet mimetics remain intact in the pre-stenotic region, but then break up into nanoparticles (smaller spheres) when they flow through the constriction. **Fig. 3B** is a photograph of the stenotic microvessel device fabricated in PDMS. **Fig. 3C** shows the CFD simulations of the micro fluidic device shown in **Fig. 3B** demonstrating that a physiological inlet shear rate of 1,000 (1/s) upstream from the constriction increases to a pathological level of ∼100,000 (1/s) in the stenotic region (90% lumen occlusion); these shear rates correspond to shear stresses of 10 and 1,000 dyne/cm², respectively. **Fig. 3D** is a bar graph showing a significant (**p* <0.005) increase in release of fluorescent NPs from platelet mimetics when they are perfused through the channel shown in **Fig. 3B** compared with flow through an unconstricted channel with a physiological level of shear stress (10 dyne/cm²). Fluorescent micrographs at right compares the NPs collected in the outflow from the unconstricted channel (top) versus the channel with the 90% constriction (bottom). **Fig. 3E** is a bar graph showing the pronounced (p <0.005) accumulation of fluorescent NPs in endothelial cells lining the area downstream (post-stenosis) of the constriction relative to an upstream area (pre-stenosis). Corresponding fluorescence microscopic images of cells from regions before (left) and after (right) the constriction are shown at the right (bar, 20 µm).

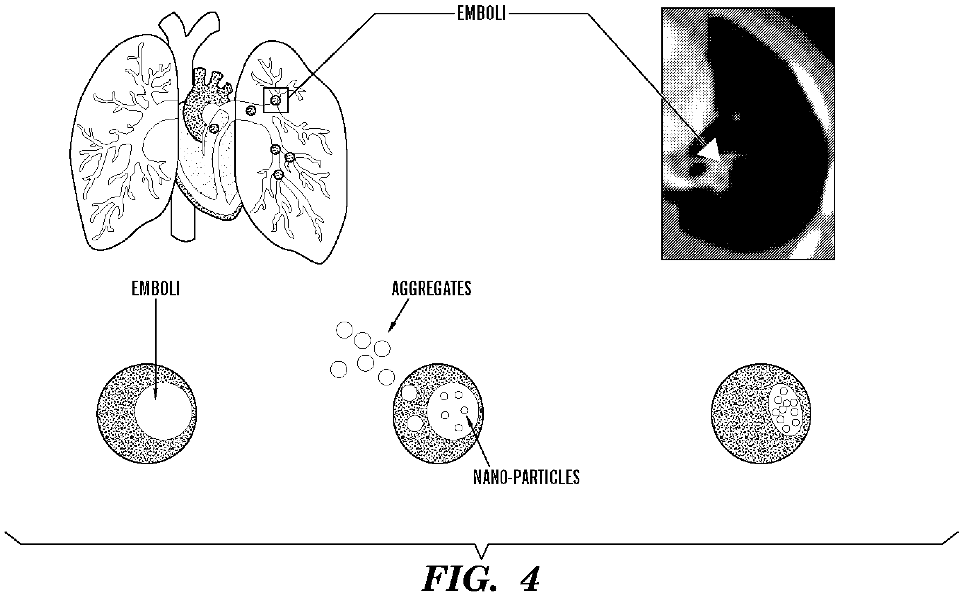

**Fig. 4** is a schematic demonstrating the proposed approach based on thrombolysis of pulmonary emboli shown in schematics (left) and computerized tomographic angiography (CTA) images (right). Bottom panel: NP aggregates coated/loaded with a thrombolytic agent (tissue plasminogen activator; tPA) break apart when flowing through regions with emboli, which partly obstruct flow and thus create elevated shear stress. NPs accumulate at these sites and locally act to dissolve the emboli.

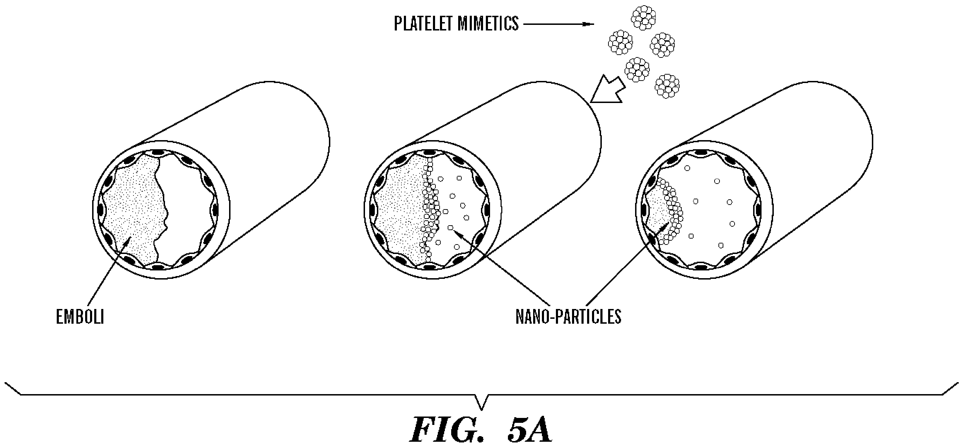

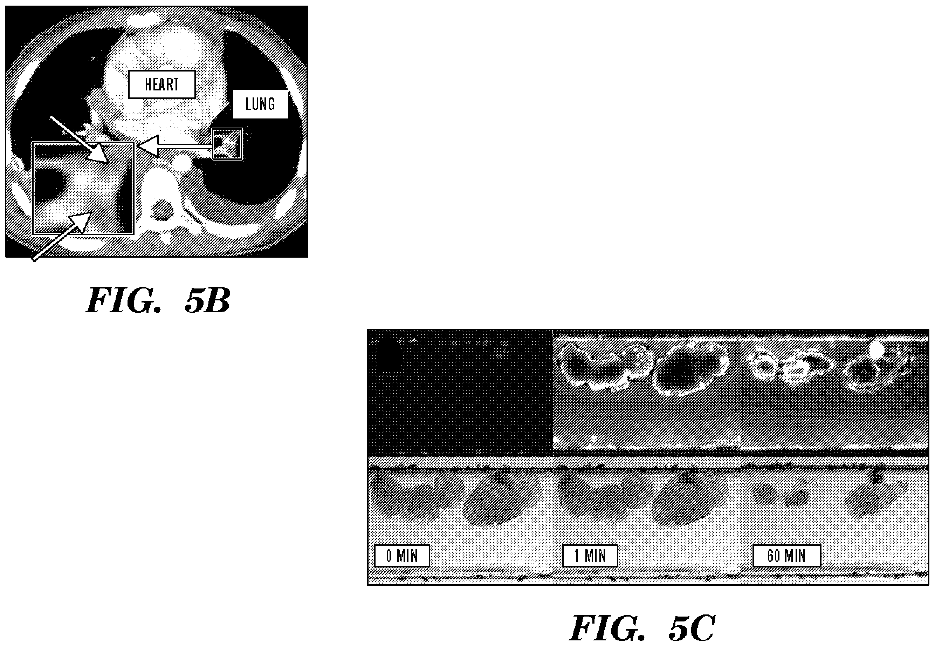

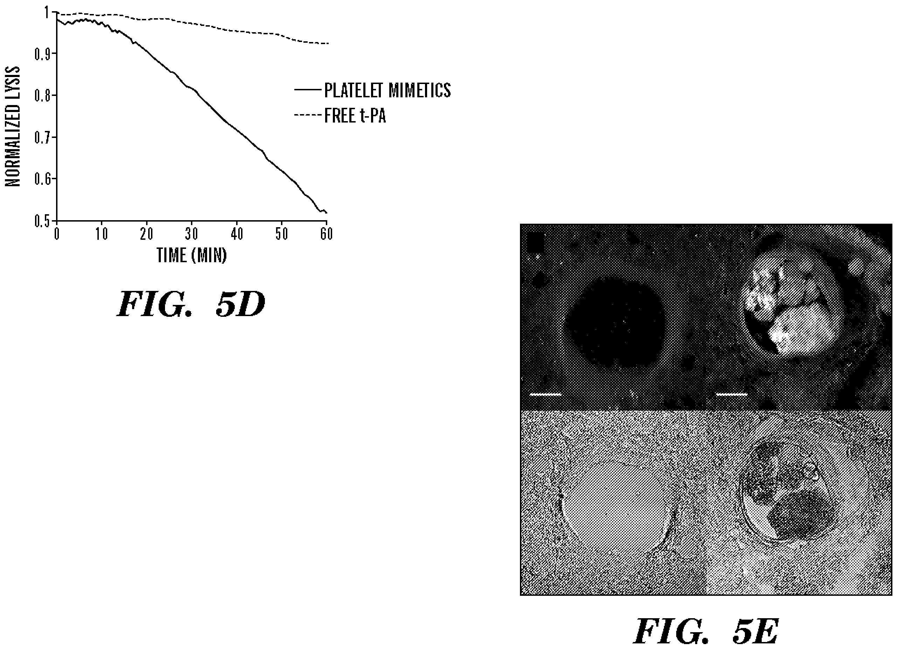

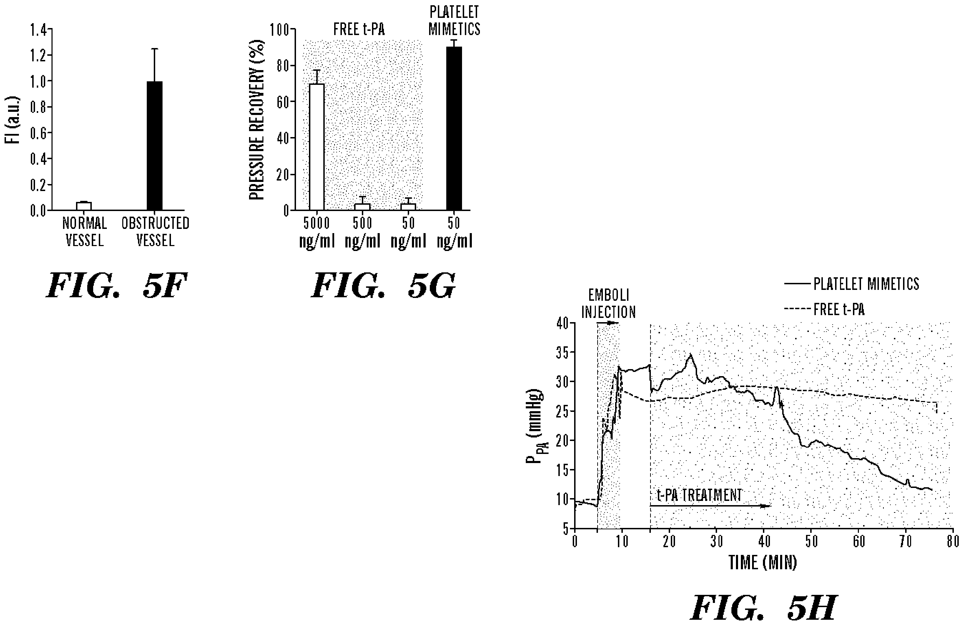

**Figs. 5A-5H** show shear-targeting of a thrombolytic drug. **Fig. 5A** is a schematic of the experimental shear-activated drug targeting approach. Formation of an embolus (lodged blood clot) that partly obstructs blood vessel flow results in local elevated shear stress in the narrowed lumen (left), which should cause the platelet mimetics to break apart and deploy NP components that will accumulate locally (center). If the NPs are coated with a thrombolytic drug, such as tPA, the NPs bound to the clot should dissolve the obstruction (right). **Fig. 5B** is a computerized tomographic angiogram of a pulmonary embolism in a human lung; emboli appear as dark regions that can be seen at higher magnification (arrows in inset) to constrict flow of blood, which appears white due to use of a contrast agent in this view. **Fig. 5C** is a time lapse fluorescence (top) and bright field (bottom) views of artificial microemboli (∼200 µm) obstructing flow in the narrowed microchannel of a microfluidic device before (0 min) and after (1 and 60 min) injection of platelet mimetics coated with t-PA (50 ng/ml). The fluorescent NPs accumulated locally on the surface of the clots within 1 min after injection, which resulted in progressive lysis and shrinkage of the clots over time. **Fig. 5D** is a line graph showing enhanced emboli lysis kinetics when using tPA-coated platelet mimetics compared to soluble t-PA. The platelet mimetics coated with t-PA (50 ng/ml) reduced emboli size by more than 50% within 1 hour (blue line), while the same concentration of free t-PA produced less than a 5% reduction in size (red line). **Fig. 5E** is a fluorescence (top) and phase contrast (bottom) views of histological sections of whole lung in which artificial fibrin clots were injected into the pulmonary artery using a mouse *ex vivo* lung ventilation-perfusion model, showing local accumulation of fluorescent NPs within the obstructing emboli. While emboli are absent from some vessels (left), they can easily be visualized in others (right). **Fig. 5F** is a bar graph showing a major (10- to 20-fold) increase (p <0.005) in accumulation of the fluorescent NPs in regions of obstruction compared to non-obstructed vessels, as detected by microfluorimetry. **Fig. 5G** is a bar graph showing that platelet mimetics coated with 50 ng/ml tPA normalize the pulmonary artery pressure within an hour after intravenous injection in the *ex vivo* pulmonary embolism model, whereas the same concentration of free t-PA or a 10 times higher dose (500 ng/ml) did not reduce the pulmonary artery pressure (N=3; *p <0.005); a 100-fold dose (5000 ng/ml) was required to produce similar effects on thrombolysis. **Fig. 5H** is real-time measurements of pulmonary artery pressure in the lung embolism model showing that the tPA-coated platelet mimetics (blue line) reversed pulmonary hypertension over approximately 1 hour, whereas the same concentration (50 ng/ml) of free t-PA was ineffective (red line).
**Figs. 6A and 6B** show enhanced adhesion of nanoparticles compared to microparticles under flow. **Fig. 6A** is a bar graph showing quantitation of the surface adhesion of PLGA tPA coated nanoparticles (average size 200 nm) and microaggregates (average size 2 mm; both at 100 mg/ml in PBS, tPA coating 0.5 mg/mg) when flowing for 15 min through a fibrin-coated 80 mm channel (10 dyne/cm²). **Fig. 6B** are fluorescence microscopic images showing much higher level of binding of the NPs at the left, compared to the microaggregates at the right.

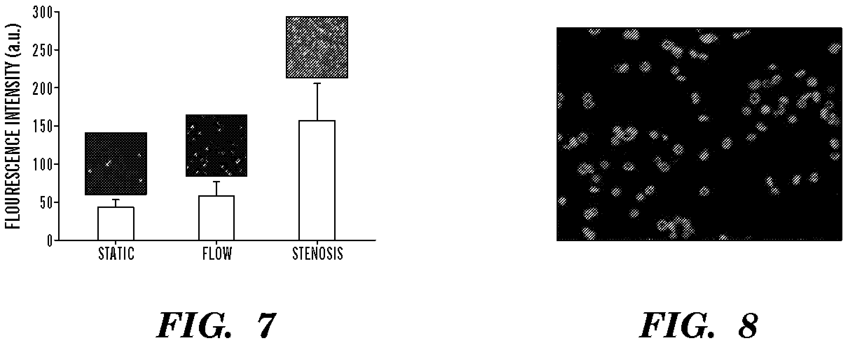

**Fig. 7** is a bar graph showing accelerated breakup of micro-aggregates into nanoparticles when flowing through a stenotic channel with characteristics that mimic a living stenotic blood vessel. A suspension of aggregates was infused through a device without a (640 micron height channel, wall shear stress 10 dyne/cm²) or with a stenotic region (80% stenosis, 80 micron in height). The suspension was then filtered through a 0.22 micron filter removing all micron sized particles and the fluorescence intensity was measured (corresponding fluorescent images shown above each bar). The flow-induced release was eight fold higher with the stenosis compared to without stenosis.
**Fig. 8** is a fluorescence image of RBC ghosts loaded with FITC-dextran (MW 70kDa) taken five days from preparation.

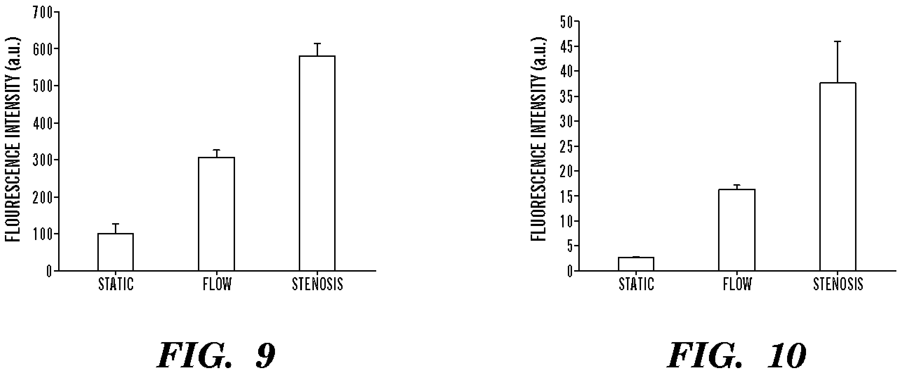

**Fig. 9** is a bar graph showing increased release from RBC ghosts flowing through a stenosis.
**Fig. 10** is a bar graph showing release of FITC-dextran from Pluronic-PEI microcapsules flowing through a stenosis.

### DETAILED DESCRIPTION OF THE INVENTION

In one aspect, the invention provides an aggregate, for use in the treatment of a stenosis and/or a stenotic lesion, and/or an occlusive lesion, comprising a plurality of nanoparticles, wherein the aggregate disaggregates under shear stress. The invention can be used to deliver a compound of interest, e.g., a therapeutic agent and/or an imaging agent, to a localized site where restricted and/or constrained fluid flow at the site results in elevated fluid shear stress. In some embodiments described herein, the aggregate is for biomedical uses. In other embodiments described herein, the aggregate is for non-medical or industrial uses.

In some embodiments of this and other aspects of the invention, the aggregate is a micro sized aggregate. By "micro sized" is meant aggregates that are on the order of 0.1 µm to 1000µm. The aggregate can be a regular or irregular shape. For example, the aggregate can be a spheroid, hollow spheroid, cube, polyhedron, prism, cylinder, rod, disc, or other geometric or irregular shape. Generally, an aggregate of the invention has at least one dimension that is ≥ 1µm (e.g., 1µm or more, 2µm or more, 5 µm or more, 10µm or more, 20µm or more, 30µm or more, 40µm or more, 50µm or more, 60µm or more, 70µm or more, 80µm or more, 90µm or more, 100µm or more, 150µm or more, 200µm or more, 250µm or more, 300µm or more, or 500µm or more). In some embodiments, an aggregate has at least one dimension that is ≤ 500 µm (e.g., 500µm or less, 400µm or less, 300µm or less, 250µm or less, 200µm or less, 150µm or less, 100µm or less, 50µm or less, 25µm or less, 20µm or less, 15µm or less, 10µm or less, or 5µm or less). In some embodiments, the aggregate has one dimension in the range of from about 0.5 µm to about 200µm, preferably in the range of from about 0.75µm to about 50µm, more preferably in the range from about 1µm to about 20µm. In some embodiments, the aggregate is 1µm to 3µm in size. In some embodiments, the aggregate is 2.5µm to 5.5µm in size. In one embodiment, the aggregates is about 3.8 µm in size.

Without wishing to be bound by a theory, because the aggregates of the invention are micro-sized, they can be cleared out easily in bile or, if biodegradable, they can be broken down into chemical components and passed out through the kidney . This can be advantageous for drug delivery in military and/or emergency situations. For example, the aggregates can be used for treating vascular infarction (stroke, heart attack, pulmonary embolism) because rapid occlusion of the vessels by blood clots results in a large increase in shear stress locally. The aggregates also can be used to treat bleeding. Because shear stress is high at bleed sites, due to high volume going through a small hole in vessel wall, the aggregates of the invention will disaggregate at the bleed sites. Thus, delivering pro-coagulants at the bleed site if contained on the NPs.

As used herein, the term "shear stress" refers to the ratio of force to area. A fluid flows in response to the applied shear force. However, when fluid flows through a channel, the fluid adjacent to the walls of the channel tends to adhere to the wall resulting in a velocity gradient as shown on left side of Fig. 1A. The fluid velocity increases as distance from the wall increases. The differences in fluid velocity, as indicated by the velocity gradient, result in a shear stress being applied on cells and particles flowing in the fluid. The shear stress increases as the distance to the wall decreases where the differences in fluid velocity are greater. Shear stress is also a function of radius, and thus it also increases when the channel becomes constricted as shown in the middle of Fig. 1A. As used herein, the term "shear stress conditions" refers to conditions under which a shearing stress is applied by a fluid. The shear stress generated by the flowing fluid can be transferred or applied to molecules, particles and aggregates that may be present in the flowing fluid. These shear stress conditions can occur in a fluid having generally laminar or turbulent flow characteristics. Amount of shear stress an aggregate undergoes is a function of aggregate size.

Generally, in normal blood vessels the wall shear stress is well below 70 dyn/cm² (7Pa) while at the stenosis site shear stress exceeds 70dyn/cm² (AM Malek, S. A. & S. Izumo "Hemodyamic shear stress and its role in atherosclerosis." JAMA, 1999, 282: 2035-2042). Accordingly, the shear stress under which an aggregate described herein disaggregates is 5 to 3000dyn/cm². In some embodiments, the shear stress under which an aggregate described herein disaggregates is ≥ 5dyn/cm², ≥ 6dyn/cm², ≥ 7dyn/cm², ≥ 8dyn/cm², ≥ 9dyn/cm², ≥ 10dyn/cm², ≥ 11dyn/cm², ≥ 12dyn/cm², ≥ 13dyn/cm², ≥ 14dyn/cm², ≥ 15dyn/cm², or ≥ 20dyn/cm². It is to be understood that complete disaggregation of the aggregate is not required.

An aggregate described herein can disaggregate by at least 5%, at least 10%, at least 15%, at least 20%, at least 25%, at least 30%, at least 40%, at least 50%, at least 60%, at least 70%, at least 75%, at least 80%, at least 85%, at least 90%, at least 95%, or100% (i.e. complete disaggregation) under shear stress conditions (e.g., a stenosis site shear stress) as compared to a control shear condition (e.g., normal blood vessel shear stress).

Amount and/or rate of disaggregation can be controlled by modulating the non-covalent association of nanoparticles in the aggregate. As used herein, the term "non-covalent association" refers to an intermolecular interaction between two or more individual molecules without involving a covalent bond. Intermolecular interaction depends on, for example, polarity, electric charge, and/or other characteristics of the individual molecules, and includes, without limitation, electrostatic (e.g., ionic) interactions, dipole-dipole interactions, van der Waal's forces, and combinations of two or more thereof. Accordingly, strength of non-covalent association can be modulated by altering one or more of the above-mentioned intermolecular interactions. For example, surface of nanoparticles can be modified to modulate intermolecular electrostatic interactions, hydrogen bonding interactions, dipole-dipole interactions, hydrophilic interaction, hydrophobic interactions, van der Waal's forces, and any combinations thereof between two or more nanoparticles.

One method of controlling association strength is by including pair of affinity binding pairs on the surface of nanoparticles and modulating the intermolecular association of these affinity binding pairs by modulating one or more of the above-noted intermolecular interactions.

As used herein, the term "hydrophilic interaction" refers to an attraction toward water molecules, wherein a material/compound or a portion thereof may bind with, absorb, and/or dissolve in water. As used herein, the term "hydrophobic interaction" refers to a repulsion against water molecules, wherein a material/compound or a portion thereof does not bind with, absorb, or dissolve in water. Association strength can be controlled by modulating the hydrophilic and/or hydrophobic characteristics of nanoparticle surface. For example, more hydrophobic nanoparticles would cluster together under hydrophilic conditions (e.g. in blood). Conversely, more hydrophilic nanoparticles would not cluster together under hydrophilic conditions.

As used herein, the term "electrostatic interaction" refers to an intermolecular interaction between two or more positively or negatively charged moieties/groups, which may be attractive when two are oppositely charged (i.e., one positive, another negative), repulsive when two charges are of the same sign (i.e., two positive or two negative), or a combination thereof. Electrostatic interaction can be modulated by including positively and negatively charged moieties/groups on the surface of the nanoparticles. By adjusting the ratio of positive to negative charges strength of association of nanoparticles can be modulated; thus, controlling the rate of disaggregation.

As used herein, the term "dipole-dipole interaction" refers an intermolecular attraction between two or more polar molecules, such as a first molecule having an uncharged, partial positive end δ+ (e.g., electropositive head group such as the choline head group of phosphatidylcholine) and a second molecule having an uncharged, partial negative end δ- (e.g., an electronegative atom such as the heteroatom O, N, or S in a polysaccharide). Dipole-dipole interaction also refers to intermolecular hydrogen bonding in which a hydrogen atom serves as a bridge between electronegative atoms on separate molecules and in which a hydrogen atom is held to a first molecule by a covalent bond and to a second molecule by electrostatic forces.

As used herein, the term "hydrogen bond" refers to an attractive force or bridge between a hydrogen atom covalently bonded to a first electronegative atom (e.g., O, N, S) and a second electronegative atom, wherein the first and second electronegative atoms may be in two different molecules (intermolecular hydrogen bonding) or in a single molecule (intramolecular hydrogen bonding). Strength of association between nanoparticles can be modulated by modulating the number of intermolecular hydrogen bonds the nanoparticles can form with each other. More intermolecular hydrogen bonds leading to more stronger association; thus a lower rate of disaggregation. Conversely, less intermolecular hydrogen bonds lead to a less stronger association; thus a higher rate of disaggregation.

As used herein, the term "van der Waal's forces" refers to the attractive forces between non-polar molecules that are accounted for by quantum mechanics. Van der Waal's forces are generally associated with momentary dipole moments induced by neighboring molecules undergoing changes in electron distribution.

In some embodiments of this and other aspects of the invention described herein, one or more compounds, e.g., a compound to be delivered, can be associated with the aggregate. As used herein, with respect to aggregates, the phrase "associated with" means entangled, embedded, incorporated, encapsulated, bound to the surface, or otherwise associated with the aggregate or a nanoparticle constituent of the aggregate. In one aspect of the invention, the aggregate comprises a molecule which is a therapeutic agent or pharmaceutically acceptable salt thereof, comprising an antithrombotic and/or thrombolytic agent, and/or a vasodilator. In another aspect, the aggregate further comprises a molecule which is an imaging agent.

Without wishing to be bound by a theory, the compound can be covalently or non-covalently associated with the nanoparticles in the aggregate. Accordingly, in some embodiments of this and other aspects of the invention described herein, the compound is encapsulated within a nanoparticle.

In some embodiments of this and other aspects of the invention described herein, the compound is absorbed/adsorbed on the surface of a nanoparticle.

In some embodiments of this and other aspects of the invention described herein, the compound is covalently linked with a nanoparticle.

It is to be understood that a compound does not need to be associated with a nanoparticle while the compound is in the aggregate. For example, preformed nanoparticle can be aggregated in the presence of the compound. Without wishing to be bound by a theory, the compound can then be present in the spaces (or cavities) in the aggregate.

In some embodiments of this and other aspects of the disclosure, the aggregate comprises at least two or more therapeutic agents. For a non-limiting example, the aggregate can comprise two or more different therapeutic agents that are known in the art to treat a disease, disorder, or condition.

In some embodiments of this and other aspects of the disclosure, the aggregate comprises an inflammatory agent and another therapeutic agent. The other therapeutic may or may not be an inflammatory agent.

### Nanoparticles

As used herein, the term "nanoparticle" refers to particles that are on the order of 10⁻⁹ or one billionth of a meter and below 10⁻⁶ or 1 millionth of a meter in size. The term "nanoparticle" includes nanospheres; nanorods; nanoshells; and nanoprisms; and these nanoparticles may be part of a nanonetwork. The term "nanoparticles" also encompasses liposomes and lipid particles having the size of a nanoparticle. The particles may be, e.g., monodisperse or polydisperse and the variation in diameter of the particles of a given dispersion may very, e.g., particle diameters of between about 0.1 to 100's of nm.

As used herein, the term "liposome" encompasses any compartment enclosed by a lipid bilayer. Liposomes may be characterized by membrane type and by size. Liposomes are also referred to as lipid vesicles in the art. In order to form a liposome the lipid molecules comprise elongated non-polar (hydrophobic) portions and polar (hydrophilic) portions. The hydrophobic and hydrophilic portions of the molecule are preferably positioned at two ends of an elongated molecular structure. When such lipids are dispersed in water they spontaneously form bilayer membranes referred to as lamellae. The lamellae are composed of two mono layer sheets of lipid molecules with their non-polar (hydrophobic) surfaces facing each other and their polar (hydrophilic) surfaces facing the aqueous medium. The membranes formed by the lipids enclose a portion of the aqueous phase in a manner similar to that of a cell membrane enclosing the contents of a cell. Thus, the bilayer of a liposome has similarities to a cell membrane without the protein components present in a cell membrane.

Liposomes include unilamellar vesicles which are comprised of a single lipid layer and generally have a diameter of 20 to 100 nanometers; large unilamellar vesicles (LUVS) are typically larger than 100nm, which can be produced by subjecting multilamellar liposomes to ultrasound. Preferred liposomes have a diameter in the range of 20-250 nm.

Without limitation, there are at least ten types of nanoparticles that can be used in forming the aggregates described herein: (1) nanoparticles formed from a polymer or other material to which a molecule of interest, e.g., a therapeutic agent, an imaging agent or a ligand, absorbs/adsorbs or forms a drug coating on a nanoparticle core; (2) nanoparticles formed from a core formed by the molecule of interest, e.g., a therapeutic agent, an imaging agent or a ligand, which is coated with a polymer or other material; (3) nanoparticles formed from a polymer or other material to which a molecule of interest, e.g., a therapeutic agent, an imaging agent or a ligand, is covalently linked; (4) nanoparticles formed from molecule of interest (e.g., a therapeutic agent, an imaging agent or a ligand) and other molecules; (5) nanoparticles formed so as to comprise a generally homogeneous mixture of a therapeutic agent, an imaging agent or a ligand with a constituent of the nanoparticle or other non-drug substance; (6) nanoparticles of pure drug or drug mixtures with a coating over a core of a molecule of interest, e.g., a therapeutic agent, an imaging agent or a ligand; (7) nanoparticles without any associated compound of interest; (8) nanoparticles composed entirely of a therapeutic agent, an imaging agent or a biologically active compound; (9) nanoparticle which have a molecule of interest, e.g., a therapeutic agent, an imaging agent or a ligand, permeated in the nanoparticles; and (10) nanoparticles which have a molecule of interest, e.g., a therapeutic agent, an imaging agent or a ligand, adsorbed to the nanoparticles. According to the invention, the nanoparticles comprise copolymers of polylactic acid and polyglycolic acid.

In some embodiments, the compound of interest, e.g., a therapeutic agent, an imaging agent or a ligand, is coated on the outer surface of the aggregate, i.e., a compound of interest forms a coating on the outer surface of the aggregate. Without wishing to be bound by a theory, a subset of the nanoparticles present in the aggregate comprise a compound of interest on the surface (i.e., the surface is coated with the compound of interest) and these nanoparticles are then present the compound of interest on the outer surface of the aggregate.

In some embodiments, the outer surface of the aggregate can be coated with a compound of interest after forming the aggregate with the nanoparticles. For example, ligands and/or chemically reactive groups can be present on the outer surface of the nanoparticles in the aggregate, and these ligands and/or chemical groups can be utilized to couple a compound of interest to the outer surface of the aggregate.

In some embodiments, a compound of interest can be absorbed/adsorbed on the outer surface of a preformed aggregate in order to form a coating of the compound of interest on the outer surface of the aggregate.

It is not necessary for every nanoparticle in the aggregate to comprise a compound of interest. Only a subset of the nanoparticles may comprise a compound of interest. For example, in an aggregate at least 10%, at least 20%, at least 30%, at least 40%, at least 50%, at least 60%, at least 70%, at least 80%, at least 90%, at least 950%, or 100% (i.e. all of the nanoparticles) can comprise a compound of interest.

A skilled artisan is well aware of a wide variety of nanoparticles for drug delivery that are known in the art. Accordingly, nanoparticles amenable to the disclosure include those described, for example, in U.S. Pat. No. 6,645,517; No. 5,543,158; No. 7,348,026; No. 7,265,090; No. 7,541,046; No. 5,578,325; No. 7,371,738; No. 7,651,770; No. 9,801,189; No. 7,329,638; No. 7,601,331; and No. 5,962,566, and U.S. Pat. App. Pub. No. US2006/0280798; No. US2005/0281884; No. US2003/0223938; 2004/0001872; No.2008/0019908; No.2007/0269380; No.2007/0264199; No.2008/0138430; No.2005/0003014; No.2006/0127467; No.2006/0078624; No.2007/0243259; No.2005/0058603; No.2007/0053870; No.2006/0105049; No.2007/0224277; No.2003/0147966; No.2003/0082237; No.2009/0226525; No.2006/0233883; No.2008/0193547; No.2007/0292524; No.2007/0014804; No.2004/0219221; No.2006/0193787; No.2004/0081688; No.2008/0095856; No.2006/0134209; and No. 2004/0247683.

In some embodiments of this disclosure, nanoparticle is a Perflubutane Polymer Microsphere or HDDS™ (Hydrophobic Drug Delivery System) from Acusphere (www.acusphere.com/technology/home.html). Perflubutane Polymer Microspheres are made by creating an emulsion containing PLGA (polylactic-co-glycolic acid), a phospholipid and a pore-forming agent. This emulsion is further processed by spray drying to produce small, porous microspheres containing gas analogous in structure of honeycombs.

Without wishing to be bound by a theory, HDDS™ can convert a broad class of drugs that do not dissolve well in water, or hydrophobic drugs, into microspheres or nanospheres of the drug embedded in small microspheres that can more rapidly dissolve in water. One preferred HDDS™ is AI-850™, which is a reformulation of the hydrophobic drug paclataxel and is bioequivalent to Abraxis Bioscience's ABRAXANE®, a leading cancer drug. This can be delivered to inhibit intimal hyperplasia or vascular constriction due to cell overgrowth.

In some embodiments of this and other aspects of the invention, the nanoparticles have an average diameter of from about 10 nm to about 500 nm. In some embodiments, the nanoparticles have an average diameter of from about 50 nm to about 250 nm. In one embodiment, the nanoparticles have an average diameter of from about 100 nm to about 250 nm. In one embodiment, the nanoparticles have an average diameter of about 180 nm.

Without limitation, nanoparticles amenable to the disclosure can be composed of any material. In some embodiments of this and other aspects of the disclosure, the nanoparticle comprises a polymer, e.g. a biocompatible polymer. The average molecular weight of the polymer, as determined by gel permeation chromatography, can range from 20,000 to about 500,000. According to the invention, the nanoparticles comprise copolymers of polylactic acid and polyglycolic acid.

As used herein, the term "biocompatible" means exhibition of essentially no cytotoxicity or immunogenicity while in contact with body fluids or tissues. As used herein, the term "polymer" refers to oligomers, co-oligomers, polymers and co-polymers, e.g., random block, multiblock, star, grafted, gradient copolymers and combination thereof.

The term "biocompatible polymer" refers to polymers which are non-toxic, chemically inert, and substantially non-immunogenic when used internally in a subject and which are substantially insoluble in blood. The biocompatible polymer can be either non-biodegradable or preferably biodegradable. Preferably, the biocompatible polymer is also noninflammatory when employed *in situ.*

Biodegradable polymers are disclosed in the art. Examples of suitable biodegradable polymers include, but are not limited to, linear-chain polymers such as polylactides, polyglycolides, polycaprolactones, copolymers of polylactic acid and polyglycolic acid, polyanhydrides, polyepsilon caprolactone, polyamides, polyurethanes, polyesteramides, polyorthoesters, polydioxanones, polyacetals, polyketals, polycarbonates, polyorthocarbonates, polydihydropyrans, polyphosphazenes, polyhydroxybutyrates, polyhydroxyvalerates, polyalkylene oxalates, polyalkylene succinates, poly(malic acid), poly(amino acids), polyvinylpyrrolidone, polyethylene glycol, polyhydroxycellulose, polymethyl methacrylate, chitin, chitosan, copolymers of polylactic acid and polyglycolic acid, poly(glycerol sebacate) (PGS), and copolymers, terpolymers, and copolymers including one or more of the foregoing. Other biodegradable polymers include, for example, gelatin, collagen, silk, chitosan, alginate, cellulose, poly-nucleic acids, etc.

Suitable non-biodegradable biocompatible polymers include, by way of example, cellulose acetates (including cellulose diacetate), polyethylene, polypropylene, polybutylene, polyethylene terphthalate (PET), polyvinyl chloride, polystyrene, polyamides, nylon, polycarbonates, polysulfides, polysulfones, hydrogels (e.g., acrylics), polyacrylonitrile, polyvinylacetate, cellulose acetate butyrate, nitrocellulose, copolymers of urethane/carbonate, copolymers of styrene/ maleic acid, poly(ethylenimine), Poloxamers (e.g. Pluronic such as Poloxamers 407 and 188), Hyaluron, heparin, agarose, Pullulan, and copolymers including one or more of the foregoing, such as ethylene/vinyl alcohol copolymers (EVOH).

In some embodiments, the biocompatible polymer is a copolymer of polylactic acid and polyglycolic acid, poly(glycerol sebacate) (PGS), poly(ethylenimine), Pluronic (Poloxamers 407, 188), Hyaluron, heparin, agarose, or Pullulan.

In addition to the molecule of interest, the nanoparticles can comprise additional moieties that can extend the in vivo lifetime of the nanoparticles in the blood. For example, the nanoparticles can comprise functional moieties that enhance the in vivo lifetime of the nanoparticles in the blood. One exemplary moiety for increasing the in vivo lifetime is polyethylene glycol. Accordingly, the aggregate can comprise nanoparticles which are polyethylene glycoated on the surface.

### Red blood cells (not part of the claimed invention)

While various aspects of the invention are discussed in relation to aggregates, one can also use red blood cells (RBCs) in place of the aggregates wherein a compound of interest, e.g., a therapeutic agent and/or an imaging agent, is associated with a red blood cell. Inventors have discovered that compounds encapsulated in red blood cells can be preferentially released from the red blood cells under shear stress. Accordingly, described herein is a method for treating or imaging a stenosis, a stenotic lesion, a blood clot, an obstructive lesion, and/or an internal hemorrhage in a subject, the method comprising administering to a subject in need thereof a red blood cell, wherein the red blood cell comprises a therapeutic agent and/or an imaging agent.

There are two major approaches for the association between a compound and RBCs. The most widely used approach is compound encapsulation in RBCs using one of several encapsulation methods. The second approach is reversible or irreversible compound attachment to RBC membrane. Accordingly, in some embodiments, the compound of interest (e.g., a therapeutic agent and/or an imaging agent) is encapsulated in the RBCs. Thus, there is no covalent linking between the compound of interest and RBC membrane.

Red blood cells are the most common cells of blood, are responsible for oxygen transport and have a typical biconcave shape. Normal human RBCs have a diameter of 7-8 µm and an average volume of 90 fl. In mammals, RBCs are anucleated and lose their organelles during maturation. A human body is commonly endowed with 2-3 x 10¹³ RBCs continuously produced at a rate of 2 million per second. RBCs spent their 100-120 day life-span travelling the circulatory system before being selectively removed by macrophages in the reticuloendothelial system (RES).

The surface area of mature, biconcave RBCs is about 136 µm² but can swell to a sphere of approx 150 fl. It is noteworthy that RBCs can also cross undamaged capillaries of 2-3 µm in diameter. The RBC membrane is strictly connected with the membrane skeletal proteins which are organized in a uniform shell. The RBC shape can undergo a number of reversible transformations. An important determinant of RBC survival is its deformability. Key factors affecting deformability are internal viscosity (mainly contributed by RBC hemoglobin), the surface/volume of the cell and the intrinsic deformability of the membrane. The RBCs have other very interesting properties namely they behave as an osmometer since they shrink when placed into a hypertonic solution or swell when placed into a hypotonic solution. The RBCs can reach a critical haemolytic volume giving rise to holes on the membrane ranging from 10 nm up to 500 nm. These processes are usually reversible and following haemolysis the holes close and the cell resumes its biconcave shape.

Red blood cells are biocompatible carriers because they are completely biodegradable without generation of toxic products and show high biocompatibility especially when autologous erythrocytes are employed. They can be easily handled *ex vivo* by means of several techniques for the encapsulation of different molecules, after which one can obtain loaded erythrocytes with morphological, immunological and biochemical properties similar to those of native cells. Lacking a nucleus and other organelles, most of their volume is available for the encapsulation of drugs. They protect the encapsulated substance from premature inactivation and degradation by endogenous factors and, at the same time, the subject against the toxic effects of the drugs thus avoiding immunological reactions. Potentially a wide variety of chemicals can be encapsulated. They have a longer life-span in circulation as compared to other synthetic carriers and can act as bioreactors due to the presence of several enzymatic activities that can directly affect the loaded molecules and, in the case of loaded prodrugs, give rise to the active drug itself.

Without limitations, red blood cells can include autologous red blood cells, i.e., a cell or cells taken from a subject who is in need of treatment (i.e., the donor and recipient are the same individual). Autologous red blood cells have the advantage of avoiding any immunologically-based rejection of the cells. Alternatively, the cells can be heterologous, e.g., taken from a donor. The second subject can be of the same or different species. Typically, when the cells come from a donor, they will be from a donor who is sufficiently immunologically compatible with the recipient, i.e., will not be subject to transplant rejection, to lessen or remove the need for immunosuppression. In some embodiments, the cells are taken from a xenogeneic source, i.e., a non-human mammal that has been genetically engineered to be sufficiently immunologically compatible with the recipient, or the recipient's species. Methods for determining immunological compatibility are known in the art, and include tissue typing to assess donor-recipient compatibility for HLA and ABO determinants. See, e.g., Transplantation Immunology, Bach and Auchincloss, Eds. (Wiley, John & Sons, Incorporated 1994). In some embodiments, red blood cells are recombinant red blood cells or red blood cell derived vesicles, for example those described in U.S. Pat. No. 7,521,174 and U.S. Pat. App. Pub. No. 2009/0274630.

A number of different methods can be used to load or encapsulate a compound of interest into RBCs. Some of these methods have a physical nature (e.g., osmosis-based and electrical pulse methods) or a chemical nature (e.g., chemical perturbation of the membrane).

The methods most widely used for erythrocyte loading are commonly based on the remarkable property of the RBCs to increase in volume when placed under condition of reduced osmotic pressure, such as in the presence of a hypotonic solution. Accordingly, osmosis-based methods constitute the more standard methods for the encapsulation compounds in red blood cells. Although in terms of methodology there are differences between one method and another, they are all based on the swelling of the cells accompanied by an increase in the permeability of the membrane of the erythrocytes when it is exposed to a hypotonic solution. The encapsulation of the substance is favored because pores appear in the membrane when red cells are under reduced osmotic pressure conditions. There are several variations to these methods, such as hypotonic dilution, hypotonic pre-swelling, the osmotic pulse, hypotonic hemolysis, and hypotonic dialysis, with the latter being the one most commonly used.

Three variations of the hypotonic haemolysis procedures are available: the dilutional, preswell dilutional and dialysis methods. Generally, the hypotonic dialysis method is used because it preserves the biochemical and physiological characteristics of the RBCs resulting from the process and it results in the highest percentage of encapsulation.

In hypotonic dialysis, the suspension of erythrocytes with a suitable hematocrit is placed in a dialysis bag facing a hypo-osmotic buffer at 4 °C with osmolalities that range from 100 mosM/kg in dog erythrocytes to 200-220 mosM/kg in sheep. Generally, for human erythrocytes recommended osmolality is a about 120 mosM/kg. The osmolality of the medium implies a compromise between the efficiency of the encapsulation and the least possible hemolysis of the dialysed erythrocytes. The compound to be encapsulated tends to be included in the suspension of red cells inside the dialysis bag. Although varying in its composition, the hypo-osmotic buffer usually includes NaH₂PO₄, CO₃HNa, glucose, reduced glutathione and ATP at pH 7.4. The ATP and reduced glutathione can be added to the dialysis buffer in order to preserve the cellular energy and reduce the environment inside the red cell, respectively. The time of dialysis can vary between 20 and 180 min. In order to perform hypo-osmotic dialysis, a continuous flow dialysis device as described in C. Ropars, G. Avenard and M. Chassaigne. In: Methods in Enzymology, vol. 149, R. Green and K.J. Widder, Editors,, Academic Press, San Diego (1987), pp. 242-248 can be used.

Subsequently, an annealing process is performed with the loaded erythrocytes in an isoosmotic medium for 10 min at 37 °C. Finally, a resealing of the erythrocytes is performed at 37 °C using a hyperosmotic buffer. The hyperosmotic buffer usually contains adenine, inosine, glucose, pyruvate, NaH₂PO₄ and NaCl at pH 7.4. Upon conclusion of the encapsulation process, the erythrocytes are again suspended in autologous plasma for subsequent administration.

When hypo-osmotic dialysis is used, several factors can affect the performance of the encapsulation, namely the tonicity of the solutions employed, times of dialysis, pH of the medium, temperature, concentration of the drug or peptide in contact with the erythrocytes, etc. The procedure permits the encapsulation of approximately 40-50% of the added compound. The final intracellular concentration of the compound is similar to the extracellular concentration.

Furthermore, ZnCl₂ can be externally added to loaded RBCs. Without wishing to be bound by a theory, this induces the reversible clusterization of the band 3 protein (an anion transporter on the RBC surface). By varying the amount of Zn²⁺ used, it can be possible to modulate the *in vivo* survival of the treated cells by controlling the extension of band 3 clustering.

The osmotic pulse method is a variation of the osmotic-based methods that uses dimethyl sulphoxide (DMSO) to facilitate the access of the substance into the erythrocytes. The mechanism is a transient osmotic gradient across the red cell membrane with a resultant loading of drug into the erythrocyte. Use of osmotic pulse is described, for example, in R. Franco, R. Barker and M. Weiner, Adv. Biosci. (series) 67 (1987), pp. 63-72.

Use of hypotonic hemolysis is described, for example, in S. Jain and N.K. Jain, Indian J. Pharm. Sci. 59 (1997), pp. 275-281; G.M. Ihler and H.C.W. Tsong, Methods Enzymol. (series) 149 (1987), pp. 221-229; and G.M. Ihler, Pharmacol. Ther. 20 (1983), pp. 151-169.

Use of hypotonic dilution is described, for example, in D.A. Lewis and H.O. Alpar, Int. J. Pharm. 22 (1984), pp. 137-146; U. Zimmermann, In: Targeted Drugs, E.P. Goldberg, Editor, John Wiley & Sons, New York (1983), pp. 153-200; V. Jaitely et al., Indian Drugs 33 (1996), pp. 589-594; S.J. Updike and R.T. Wakamiya, J. Lab. Clin. Med. 101 (1983), pp. 679-691; D.A. Lewis, Pharm. J. 233 (1984), pp. 384-385; K. Adriaenssenset al. al., Clin. Chem. 22 (1976), pp. 323-326; R. Baker, Nature 215 (1967), pp. 424-425; G.M. Ihler and H.C.W. Tsong, Methods Enzymol. (series) 149 (1987), pp. 221-229; S.J. Updike, R.T. Wakarniya and E.N. Lightfoot, Science 193 (1976), pp. 681-683; N. Talwar and N.K. Jain, Drug Devel. Ind. Pharm. 18 (1992), pp. 1799-1812; E. Pitt et al., Biochem. Pharmacol. 22 (1983), pp. 3359-3368; G.M. Iher, R.M. Glew and F.W. Schnure, Proc. Natl. Acad. Sci. U. S. A. 70 (1973), pp. 2663-2666; J.R. Deloach and G.M. Ihler, Biochim. Biophys. Acta 496 (1977), pp. 136-145; and S. Bhaskaran and S.S. Dhir, Indian J. Pharm. Sci. 57 (1995), pp. 240-242.

Use of hypotonic dialysis is described, for example, in U. Zimmermann, In: Targeted Drugs, E.P. Goldberg, Editor, John Wiley & Sons, New York (1983), pp. 153-200; V. Jaitely et al., Indian Drugs 33 (1996), pp. 589-594; H.G. Erchler et al., Clin. Pharmacol. Ther. 40 (1986), pp. 300-303; G.M. Ihler and H.C.W. Tsong, Methods Enzymol. (series) 149 (1987), pp. 221-229; U. Benatti et al., Adv. Biosci. (series) 67 (1987), pp. 129-136; R. Kravtozoff et al., J. Pharm. Pharmacol. 42 (1990), pp. 473-476; J.D. Berman, Adv. Biosci. (series) 67 (1987), pp. 145-152; J.R. Deloach et al., Adv. Biosci. (series) 67 (1987), pp. 183-190; J.R. Deloach and G.M. Ihler, Biochim. Biophys. Acta 496 (1977), pp. 136-145; M. Jradeet al., Adv. Biosci. (series) 67 (1987), pp. 29-36; A. Zanella et al., Adv. Biosci. (series) 67 (1987), pp. 17-27; G. Fiorelli et al., Adv. Biosci. (series) 67 (1987), pp. 47-54; and M.I. Garin et al., Pharm. Res. 13 (1996), pp. 869-874.

Use of hypotonic preswelling is described, for example, in V. Jaitely et al., Indian Drugs 33 (1996), pp. 589-594; S. Jain and N.K. Jain, Indian J. Pharm. Sci. 59 (1997), pp. 275-281; H.O. Alpar and W.J. Irwin, Adv. Biosci. (series) 67 (1987), pp. 1-9; N. Talwar and N.K. Jain, J. Control. Release 20 (1992), pp. 133-142; D.J. Jenner et al., Br. J. Pharmacol. 73 (1981), pp. 212P-213P; H.O. Alpar and D.A. Lewis, Biochem. Pharmacol. 34 (1985), pp. 257-261; G.M. Ihler and H.C.W. Tsong, Methods Enzymol. (series) 149 (1987), pp. 221-229; E. Pitt, D.A. Lewis and R. Offord, Biochem. Pharmacol. 132 (1983), pp. 3355-3358; N. Talwar and N.K. Jain, Drug Devel. Ind. Pharm. 18 (1992), pp. 1799-1812; E. Pitt et al., Biochem. Pharmacol. 22 (1983), pp. 3359-3368; S. Jain, S.K. Jain and V.K. Dixit, Drug Devel. Ind. Pharm. 23 (1997), pp. 999-1006; H. Tajerzadeh and M. Hamidi, Drug Devel. Ind. Pharm. 26 (2000), pp. 1247-1257; M. Hamidi et al., Drug Deliv. 8 (2001), pp. 231-237; and J. Bird, R. Best and D.A. Lewis, J. Pharm. Pharmacol. 35 (1983), pp. 246-247.

Compounds can also be encapsulated in red blood cells by exposing the cells to membrane active drugs such as primaquine, hydrocortisone, vinblastine and chlorpromazine, which are known to induce stomatocyte formation in the cell membrane. Use of chemical perturbation is described, for example, in U. Zimmermann, In: Targeted Drugs, E.P. Goldberg, Editor, John Wiley & Sons, New York (1983), pp. 153-200; J. Connor and A.J. Schroit, Adv. Biosci. (series) 67 (1987), pp. 163-171; I. Ben-Bassat, K.G. Bensch and S.L. Schrier, J. Clin. Invest. 51 (1972), pp. 1833-1844; L.M. Matovcik, I.G. Junga and S.L. Schrier, Drug-induced endocytosis of neonatal erythrocytes. Blood 65 (1985), pp. 1056-1063; S.L. Schrier, A. Zachowski and P.F. Devaux, Blood 79 (1992), pp. 782-786; and M. Tonetti et al., Eur. J. Cancer 27 (1991), pp. 947-948.

Electroporation is based on inducing pores in the red blood cell membrane by exposing the cells to a strong external electrical field. These pores are able to admit compounds of different size. This method of encapsulation is a good alternative to other commonly employed techniques and has been used in the encapsulation of enzymes such as alcohol and aldehyde dehydrogenase and drugs such as diclofenac sodium. Use of electroporation dilution is described, for example, in D.A. Lewis and H.O. Alpar, Int. J. Pharm. 22 (1984), pp. 137-146; U. Zimmermann, In: Targeted Drugs, E.P. Goldberg, Editor, John Wiley & Sons, New York (1983), pp. 153-200; V. Jaitely et al., Indian Drugs 33 (1996), pp. 589-594; D.A. Lewis, Pharm. J. 233 (1984), pp. 384-385; K. Kinosita and T.Y. Tsong, Nature 272 (1978), pp. 258-260; S. Jain, S.K. Jain and V.K. Dixit, Indian Drugs 32 (1995), pp. 471-476; C.A. Kruse et al., Adv. Biosci. (series) 67 (1987), pp. 137-144; U. Zimmermann, F. Riemann and G. Pilwat, Biochim. Biophys. Acta 436 (1976), pp. 460-474; D.H. Mitchell, G.T. James and C.A. Kruse, Biotechnol. Appl. Biochem. 12 (1990) (3), pp. 264-275; T.Y. Tsong, Biophys. J. 60 (1991), pp. 297-306; C. Lizano et al., Biochim. Biophys. Acta 1425 (1998), pp. 328-336; C. Lizano, M.T. Perez and M. Pinilla, Life Sci. 68 (2001), pp. 2001-2016; Q. Dong and W. Jin, Electrophoresis 22 13 (2001), pp. 2786-2792; M. Haritou et al., Clin. Hemorheol. Microcirc. 19 (1988), pp. 205-217; and P.C. Mangal and A. Kaur, Indian J. Biochem. Biophys. 28 (1991), pp. 219-221.

Methods for encapsulating a compound of interest in RBCs are also described, for example, in L. Rossi, S. Serafini and M. Magnani, In: M. Magnani, Editor, Erythrocytes Engineering for Drug Delivery and Targeting, M. Magnani, Editor, Kluwer Academic/Plenum Publishers, New York (2003), pp. 1-18; C. Lizano, M.T. Perez and M. Pinilla, Life Sci. 68 (2001), pp. 2001-2016; R.S. Franco et al., Transfusion 30 (1990), pp. 196-200; M. Ihler, Bibl. Haematol. 51 (1985), pp. 127-133; G.M. Ihler and H.C.W. Tsong, Methods Enzymol. (series) 149 (1987), pp. 221-229; S.E. Mulholland et al., Pharm. Res. 16 (1999) (4), pp. 514-518; and L.A. Lotero, G. Olmos and J.C. Diez, Biochim. Biophys. Acta 1620 (2003) (1-3), pp. 160-166.

A number of active substances have been encapsulated into RBCs. See for example, M. Magnan et al., Drug Deliv. 2 (1995), pp. 57-61; U. Benatti et al., Biochem. Biophys. Res. Commun. 220 (1996), pp. 20-25; A. Fraternale, L. Rossi and M. Magnani, Biochem. Biophys. Acta 1291 (1996), pp. 149-154; L. Rossi et al., AIDS Res. Hum. Retroviruses 15 (1999), pp. 345-353; M. Magnani et al., Proc. Natl. Acad. Sci. U. S. A. 93 (1996), pp. 4403-4408; L. Rossi et al., AIDS Res. Hum. Retroviruses 14 (1998), pp. 435-444; L. Rossi et al., J. Antimicrob. Chemother. 47 (2001), pp. 819-827; P. Franchetti et al., Antivir. Chem. Chemother. 12 (2001), pp. 151-159; P. Franchetti et al., Antivir. Res. 47 (2000), pp. 149-158; M. D'Ascenzo et al., In: Erythrocytes as Drug Carriers in Medicine, U. Sprandel and J.L. Way, Editors, Plenum Press, New York (1997), pp. 81-88; R. Crinelli et al., Blood Cells Mol. Diseases 26 (2000), pp. 211-222; L. Rossi et al., Biotechnol. Appl. Biochem. 33 (2001), pp. 85-89; L. Rossi et al., Blood Cells Mol. Diseases 33 (2004), pp. 57-63; R. Kravtzoff et al., In: Advances in the Biosciences, R. Green and J.R. De Loach, Editors, Pergamon Press, Oxford (1991), pp. 127-137; M. Magnani et al., Biotechnol. Appl. Biochem. 18 (1993), pp. 217-226; M. Magnani et al., Alcohol Clin. Exp. Res. 13 (1989), p. 849; L. Rossi et al., In: Resealed Erythrocytes as Carriers and Bioreactors, R. Green and J.R. De Loach, Editors, Pergamon Press, Oxford (1991), pp. 169-179; L. Rossi et al., J. Antimicrob. Chemother. 53 (2004), pp. 863-866; C. De Chastellier, T. Lang and L. Thilo, Eur. J. Cell. Biol. 68 (1995), pp. 167-182; A. Antonell et al., Br. J. Haematol. 104 (1999), pp. 475-481; A. Fraternale et al., Antivir. Res. 56 (2002), pp. 263-272; A.T. Palamara et al., AIDS Res. Hum. Retroviruses 12 (1996), pp. 1373-1381; A. Fraternale et al., J. Antimicrob. Chemother. 52 (2003), pp. 551-554; R. Buhl, et al., Lancet 2 (1989), pp. 1294-1298; F.J. Staal et al., AIDS Res. Hum. Retroviruses 8 (1992), pp. 305-311; S. Mihm et al., FASEB J. 9 (1995), pp. 246-252; E. Garaci et al., Biochem. Biophys. Res. Commun. 188 (1992), pp. 1090-1096; A.T. Palamara et al., Antivir. Res. 27 (1995), pp. 237-253; A.T. Palamara et al., AIDS Res. Hum. Retroviruses 12 (1996), pp. 1537-1541; M. Magnani et al., AIDS Res. Hum. Retroviruses 13 (1997), pp. 1093-1099; Y. Murata et al., Int. Immunol. 14 (2002), pp. 201-212; M. Egholm et al., Nature 365 (1993), pp. 566-568; P. Wittung et al., FEBS Lett. 365 (1995), pp. 27-29; L. Chiarantini et al., Biochemistry 41 (2002), pp. 8471-8477; and H. Arima et al., J. Pharm. Sci. 86 (1997), pp. 1079-1084. In most of these references the drug is encapsulated as a nondiffusible pro-drug that is converted into a diffusible drug by RBC resident enzymes and released in circulation. Alternatively the drug is maintained into the RBCs until these are targeted to and phagocytised by macrophages where their content is released. In some instances, RBCs are used as circulating bioreactors for the degradation of metabolites or xenobiotics. In this case an enzyme is encapsulated into RBCs where it remains catalytically active as long as the cell circulates. These modified RBCs are able to perform as circulating bioreactors when a metabolite, and/or a xenobiotic able to cross the RBC membrane reach the enzyme within the cell.

In addition to association with a compound of interest, e.g., a therapeutic agent and/or an imaging agent, a wide variety of entities, e.g., ligands, can also be coupled to the red blood cells. These ligands can be attached to the red blood cell membrane using methods known in the art. For example, coupling of a ligand to RBC can be using a non-specific chemical cross-linkers such as tannic acid and chromium chloride. See, for example, V.R. Muzykantov et al., Anal Biochem. (1993) 208:338-342; V.R. Muzykantov et al., Am J Pathol. (1987) 128:276-285; and L. Chiarantini et al., Biotechnol Appl Biochem. (1992), 15:171-184. Alternatively, coupling of a ligand to RBC can be using specific cross-linkers for coupling to defined reactive groups on RBC membrane. In particular, controlled biotinylation of RBC lysine residues using NHS esters of biotin is one of the most popular means for conjugation cargoes to RBC surface for a wide variety of applications *in vitro* and *in vivo.* Use of specific cross-linkers for linking molecules to RBC is described, for example, in G.A. Orr GA, J Biol Chem. (1981) 256:761-766; W. Godfrey et al., Exp Cell Res. (1981) 135:137-145; E. Roffman et al., Biochem Biophys Res Commun. (1986) 136:80-85; E.A. Bayer et al., Anal Biochem. (1987) 161:262-271; M. Wilchek et al., Biochem Biophys Res Commun. (1986) 138:872-879; G.P. Samokhin et al., FEBS Lett. (1983) 154:257-261; V.R. Muzykantov et al., J Immunol Methods. (1993) 158:183-190; M.D. Smirnov et al., Biochem Biophys Res Commun. (1983) 116:99-105; V.R. Muzykantov et al., FEBS Lett. (1985) 182:62-66; M. Magnaniet al., Biotechnol Appl Biochem. (994) 20(Pt 3):335-345; V.R. Muzykantov et al., Anal Biochem. (1994) 223:142-148; and H. Cowley et al., Transfusion (1999) 39:163-168.

Conjugation of highly hydrophilic polyethylene glycol (PEG) with the chain length in the range MW 3-10kD has evolved as a universal "stealth" technology, prolonging circulation and masking from defense systems in the body of liposomes, nanoparticles, polymer nanocarriers, proteins, other drug carriers, and drugs themselves. Accordingly, the red blood cells described herein can by PEGylated. Without wishing to be bound by a theory, PEG-coated RBC are less effectively opsonized, taken up by phagocytes and recognized by antibodies to RBC antigens. Methods of coupling PEG to RBCs are well known in the art and described, for example, in A. J. Bradley et al., Transfusion 41 (2001) pp:1225-1233; D. Sabolovic et al., Electrophoresis 21 (2000) pp: 301-306; P. Nacharaju et al., Transfusion 45 (2005) pp:374-383; P. Nacharaju et al. Artif Cells Blood Substit Immobil Biotechnol 35 (2007) pp:107-118; H.A. Chunget al., J Biomed Mater Res A 70 (2004) pp 179-185; M.D. Scott et al., Proc Natl Acad Sci USA 94 (1997) pp:7566-7571; J.K. Leach, A. Hinman and E.A. O'Rear, Biomed Sci Instrum 38 (2002) pp:333-338; and S. Hashemi-Najafabadi et al., Bioconjug Chem 17 (2006) pp:1288-1293. One can also modify RBCs by Pluronic, a tri-block copolymer combining two PEG chains at the ends of a less hydrophilic moiety as described in J.K. Armstrong et al., Biorheology 38 (2001) pp:239-247.

In some embodiments, a RBC comprises at least one therapeutic agent and at least one imaging agent.

### Microcapsules (not part of the claimed invention)

A compound of interest, e.g., a therapeutic agent and/or an imaging agent, can also be encapsulated in a microcapsule for delivery to a stenosis site. Accordingly,described herein is a method for treating or imaging a stenosis, a stenotic lesion, a blood clot, an obstructive lesion, and/or an internal hemorrhage in a subject, the method comprising administering to a subject in need thereof a microcapsule, wherein the microcapsule comprises a therapeutic agent and/or an imaging agent. Without wishing to be bound by a theory, the microcapsule breaks apart under the elevated shear stress at the elevated shear stress at the stenosis site and releases the compound of interest (e.g., a therapeutic agent or an imaging agent).

As used herein, the term "microcapsule" means a spheroid, cube, polyhedron, prism, cylinder, rod, disc, or other geometric or irregular shape structure ranging in size from on the order of about 1 micron to about 5,000 microns composed of a distinct polymer shell, which serves as a wall-forming material, surrounding encapsulated media, e.g., a compound of interest, located within the shell. This term is distinct from microspheres, which consist of spherical homogeneous granules of a compound of interest dispersed in a polymer and are, in strict sense, spherically empty particles.

A microcapsule can be a single-layer microcapsule or a multi-layer microcapsule. As used herein the term "single-layer microcapsule" refers to a microcapsule consisting of a single polymeric shell and the encapsulated compound located within the shell in the center of the microcapsule. The term "multi-layer microcapsule" refers to a microcapsule consisting of an inner core microcapsule and one or more outer polymeric shells. The term "double-layer microcapsule" refers to a microcapsule consisting of the inner core microcapsule coated with a second polymeric shell. In the course of the microencapsulation, the core microcapsules are introduced to the polymer-plasticizer solution or polymer-mineral dispersion, and promote the formation of "embryo" shells, which are converted to a structured solid shell of double-layer microcapsules.

As used herein, the term "inner core microcapsule" refers to a single-layer microcapsule as defined above when within a double-layer or multi-layer microcapsule.

The term "wall-forming polymer" typically refers to a polymer or a combination of two or more different polymers as defined herein, which form a component of the external wall or layer or shell of the microcapsules. In some embodiments, the wall-forming polymer is a biocompatible polymer.

In some embodiments, the wall-forming polymer is a poloxamer. Poloxamers are nonionic triblock copolymers composed of a central hydrophobic chain of polyoxypropylene (poly(propylene oxide)) flanked by two hydrophilic chains of polyoxyethylene (poly(ethylene oxide)). Poloxamers are also known by the trade name Pluronic or Pluronics. Because the lengths of the polymer blocks can be customized, many different poloxamers exist that have slightly different properties. For the generic term "poloxamer", these copolymers are commonly named with the letter "P" (for poloxamer) followed by three digits, the first two digits x 100 give the approximate molecular mass of the polyoxypropylene core, and the last digit x 10 gives the percentage polyoxyethylene content (e.g., P407 = Poloxamer with a polyoxypropylene molecular mass of 4,000 g/mol and a 70% polyoxyethylene content) . For the Pluronic tradename, coding of these copolymers starts with a letter to define its physical form at room temperature (L = liquid, P = paste, F = flake (solid)) followed by two or three digits, The first digit (two digits in a three-digit number) in the numerical designation, multiplied by 300, indicates the approximate molecular weight of the hydrophobe; and the last digit x 10 gives the percentage polyoxyethylene content (e.g., L61 = Pluronic with a polyoxypropylene molecular mass of 1,800 g/mol and a 10% polyoxyethylene content). In some embodiment, the poloxamer is Pluronic F127.

As used herein, the term "polymer shell" refers to a polymer layer containing the wall-forming polymer and, optionally, further components such as a plasticizer and/or a mineral.

Numerous techniques for forming microcapsules are available depending on the nature of the encapsulated substance and on the type of wall-forming polymer used. A widely used method for encapsulation of water insoluble substances such as some vitamins, drugs and oils within water insoluble polymers is the solvent removal method. Generally in such a process the desired wall-forming polymer is dissolved in a suitable organic solvent. This action is followed by addition of the desired compound to be encapsulated. This compound is either dissolved or dispersed in the organic solvent. The resulting organic solution or dispersion is dispersed in an aqueous phase to obtain an oil-in-water emulsion where oily microparticles are dispersed in the aqueous phase. Upon complete removal of the solvent from the microparticles, the microcapsules are formed. A basic prerequisite for this process is the use of a solvent that is able to efficiently dissolve the compound to be encapsulated as well as the wall-forming material. This solvent has to be only partially soluble in water, giving rise to emulsion of an organic phase in a continuous water phase. Chlorinated solvents such as dichloromethane and chloroform as well as glycols or their mixtures with other solvents have been widely used since they facilitate the microencapsulation process.

Without limitations, solvent can be removed by vacuum distillation, evaporation, or extraction with water. Exemplary methods of solvent removal are described, for example, in U.S. Pat. No. 4,384,975 and No. 3,891,570.

Methods of forming microcapsules are described, for example, in U.S. Pat. No. 3,173,878; No. 3,460,972; No. 3,516,941; No. 4,089,802; No. 4,093,556; No. 4,105,823; No. 4,140,516; No. 4,157,983; No. 4,219,604; No. 4,219,631; No. 4,221,710; No. 4,272,282; No. 4,534,783; No. 4,557,755; No. 4,574,110; No. 4,601,863; No. 4,711,749; No. 4,753,759; No. 4,898,696; No. 4,936,916; No. 4,956,129; No. 4,957,666; No. 5,011,634; No. 5,061,410; No. 5,160,529; No. 5,204,185; No. 5,236,782; No. 5,401,577; No. 5,529,877; No. 5,603,986; No. 5,650,173; No. 5,654,008; No. 5,733,561; No. 5,837,653; No. 5,861,360; No. 5,86,9424; No. 6,099,864; No. 6,197,789; No. 6,248,364; No. 6,251,920; No. 6,270,836; No. 6,524,763; No. 6,534,091; No. 6,733,790; No. 6,818,296; No. 6,951,836; No. 6,969,530; No. 6,974,592; No. 7,041,277; No. 7,736,695; No. 7,803,422; No. 7,833,640; and No. 7897,555, and U.S. Pat. Pub. No. 2003/0118822; No. 2004/0115280; No. 2004/0170693; No. 2006/0040844; No. 2007/0042184; No. 2006/0256423; No. 2009/0289216; and No. 2010/0009893. Methods of preparing multi-wall microspheres are described, for example, in U.S. No. 3,429,827; No. 4,861,627; No. 5,795,570; No. 5,985,354; No. 6,511,749; and No. 6,528,035; and U.S. Pat. App. Pub. No. 2003/0222378.

The shear stress under which a microcapsule described herein can break apart is 5 to 3000dyn/cm². In some embodiments, the shear stress under which a microcapsule described herein breaks apart is ≥ 5dyn/cm², ≥ 6dyn/cm², ≥ 7dyn/cm², ≥ 8dyn/cm², ≥ 9dyn/cm², ≥ 10dyn/cm², ≥ 11dyn/cm², ≥ 12dyn/cm², ≥ 13dyn/cm², ≥ 14dyn/cm², ≥ 15dyn/cm², or ≥ 0dyn/cm².

As used herein, "breaking apart" refers to breaking of the polymeric shell of the microcapsule into smaller pieces. It is to be understood that complete breakup of the polymeric shell is not required. Accordingly, in some embodiments, a microcapsule can break apart such that at least 5%, at least 10%, at least 15%, at least 20%, at least 25%, at least 30%, at least 40%, at least 50%, at least 60%, at least 70%, at least 75%, at least 80%, at least 85%, at least 90%, at least 95%, or 100% (i.e. complete breakup) of the polymeric shell is broken into smaller pieces under shear stress conditions (e.g., a stenosis site shear stress) as compared to a control shear condition (e.g., normal blood vessel shear stress).

Under elevated shear stress, the rate of release of an encapsulated compound from the microcapsule is at least 10%, at least 20%, at least 30%, at least 40%, at least 50%, at least 60%, at least 70%, at least 80%, at least 90%, at least 1-fold, at least 2-fold, at least 5-fold, at least 10-fold, at least 20-fold, at least 30-fold, at least 40-fold, at least 50-fold, or at least 100-fold or higher, relative to release under non-elevated shear stress (i.e., normal blood vessel shear stress).

In some embodiments, the amount of an encapsulated compound released from the microcapsule is at least 10%, at least 20%, at least 30%, at least 40%, at least 50%, at least 60%, at least 70%, at least 80%, at least 90%, at least 1-fold, at least 2-fold, at least 5-fold, at least 10-fold, at least 20-fold, at least 30-fold, at least 40-fold, at least 50-fold, or at least 100-fold or higher under shear stress conditions (e.g., a stenosis site shear stress) as compared to a control shear condition (e.g., normal blood vessel shear stress).

Exemplary microcapsules include those described, for example, in U.S. Pat. No. 3,173,878; No. 3,429,827; No. 3,460,972; No. 3,516,941; No. 4,089,802; No. 4,093,556; No. 4,105,823; No. 4,140,516; No. 4,157,983; No. 4,219,604; No. 4,219,631; No. 4,221,710; No. 4,272,282; No. 4,534,783; No. 4,557,755; No. 4,574,110; No. 4,601,863; No. 4,711,749; No. 4,753,759; No. 4,861,627; No. 4,898,696; No. 4,936,916; No. 4,956,129; No. 4,957,666; No. 5,011,634; No. 5,061,410; No. 5,160,529; No. 5,204,185; No. 5,236,782; No. 5,401,577; No. 5,529,877; No. 5,603,986; No. 5,650,173; No. 5,654,008; No. 5,733,561; No. 5,795,570; No. 5,837,653; No. 5,861,360; No. 5,86,9424; No. 5,985,354; No. 6,099,864; No. 6,197,789; No. 6,248,364; No. 6,251,920; No. 6,270,836; 6,511,749; No. 6,524,763; No. 6,528,035; No. 6,534,091; No. 6,733,790; No. 6,818,296; No. 6,951,836; No. 6,969,530; No. 6,974,592; No. 7,041,277; No. 7,736,695; No. 7,803,422; No. 7,833,640; and No. 7897,555, and U.S. Pat. Pub. No. 2003/0118822; No. 2003/0222378No. 2004/0115280; No. 2004/0170693; No. 2006/0040844; No. 2007/0042184; No. 2006/0256423; No. 2009/0289216; and No. 2010/0009893.

In some embodiments, a microcapsule comprises at least one therapeutic agent and at least one imaging agent.

### Compounds of interest

A wide variety of compounds can be associated with aggregates, red blood cells and microcapsules. Accordingly, without limitation, the compound of interest can be selected from the group consisting of small or large organic or inorganic molecules, monosaccharides, disaccharides, trisaccharides, oligosaccharides, polysaccharides, biological macromolecules, e.g., peptides, proteins, peptide analogs and derivatives thereof, peptidomimetics, nucleic acids, nucleic acid analogs and derivatives, polynucleotides, oligonucleotides, enzymes, an extract made from biological materials such as bacteria, plants, fungi, or animal cells or tissues, naturally occurring or synthetic compositions, particulates, non-aggregating nanoparticles, or any combinations thereof. The compound can be hydrophobic, hydrophilic, or amphiphilic.

As used herein, the term "particulate" refers to a particle, powder, flake, etc., that inherently exists in a relatively small form and may be formed by, for example, grinding, shredding, fragmenting, pulverizing, atomizing, or otherwise subdividing a larger form of the material into a relatively small form.

As used herein, the term "non-aggregating nanoparticle" refers to nanoparticles that do not aggregate under the conditions for aggregation described herein.

As used herein, the term "small molecule" can refer to compounds that are "natural product-like," however, the term "small molecule" is not limited to "natural product-like" compounds. Rather, a small molecule is typically characterized in that it contains several carbon-carbon bonds, and has a molecular weight of less than 5000 Daltons (5 kD), preferably less than 3 kD, still more preferably less than 2 kD, and most preferably less than 1 kD. In some cases it is highly preferred that a small molecule have a molecular mass equal to or less than 700 Daltons.

In one embodiment, the compound is a peptide or a protein. As used herein, the term "peptide" is used in its broadest sense to refer to compounds containing two or more amino acids, amino acid equivalents or other non-amino groups joined to each other by peptide bonds or modified peptide bonds. Peptide equivalents can differ from conventional peptides by the replacement of one or more amino acids with related organic acids (such as PABA), amino acids or the like or the substitution or modification of side chains or functional groups. A peptide can be of any size so long; however, in some embodiments, peptides having twenty or fewer total amino acids are preferred. Additionally, the peptide can be linear or cyclic. Peptide sequences specifically recited herein are written with the amino terminus on the left and the carboxy terminus on the right.

In addition, the term "peptide" broadly includes proteins, which generally are polypeptides. As used herein, the term "protein" is used to describe proteins as well as fragments thereof. Thus, any chain of amino acids that exhibits a three dimensional structure is included in the term "protein", and protein fragments are accordingly embraced.

A peptidomimetic is a molecule capable of folding into a defined three-dimensional structure similar to a natural peptide As used herein, the term "nucleic acid" refers to a polymers (polynucleotides) or oligomers (oligonucleotides) of nucleotide or nucleoside monomers consisting of naturally occurring bases, sugars and intersugar linkages. The term "nucleic acid" also includes polymers or oligomers comprising non-naturally occurring monomers, or portions thereof, which function similarly. Such modified or substituted nucleic acids are often preferred over native forms because of properties such as, for example, enhanced cellular uptake and increased stability in the presence of nucleases.

A nucleic acid can be single-stranded or double-stranded. A single-stranded nucleic acid can have double-stranded regions and a double-stranded nucleic acid can have single-stranded regions. Exemplary nucleic acids include, but are not limited to structural genes, genes including control and termination regions, self-replicating systems such as viral or plasmid DNA, single-stranded and double-stranded siRNAs and other RNA interference reagents (RNAi agents or iRNA agents), short-hairpin RNAs (shRNA), antisense oligonucleotides, ribozymes, microRNAs, microRNA mimics, aptamers, antimirs, antagomirs, triplex-forming oligonucleotides, RNA activators, immuno-stimulatory oligonucleotides, and decoy oligonucleotides.

In some embodiments of this and other aspects of the invention described herein, the compound is biologically active or has biological activity.

As used herein, the term "biological activity" or "bioactivity" refers to the ability of a compound to affect a biological sample. Biological activity can include, without limitation, elicitation of a stimulatory, inhibitory, regulatory, toxic or lethal response in a biological assay at the molecular, cellular, tissue or organ levels. For example, a biological activity can refer to the ability of a compound to exhibit or modulate the effect/activity of an enzyme, block a receptor, stimulate a receptor, modulate the expression level of one or more genes, modulate cell proliferation, modulate cell division, modulate cell morphology, or any combination thereof. In some instances, a biological activity can refer to the ability of a compound to produce a toxic effect in a biological sample, or it can refer to an ability to chemical modify a target molecule or cell.

In aspects of the invention, the compound is a therapeutic agent. As used herein, the term "therapeutic agent" refers to a biological or chemical agent used for treatment, curing, mitigating, or preventing deleterious conditions in a subject. The term "therapeutic agent" also includes substances and agents for combating a disease, condition, or disorder of a subject, and includes drugs, diagnostics, and instrumentation. "Therapeutic agent" also includes anything used in medical diagnosis, or in restoring, correcting, or modifying physiological functions. The terms "therapeutic agent" and "pharmaceutically active agent" are used interchangeably herein.

The therapeutic agent is selected according to the treatment objective and biological action desired. General classes of therapeutic agents include anti-microbial agents such as adrenergic agents, antibiotic agents or antibacterial agents, antiviral agents, anthelmintic agents, anti-inflammatory agents, antineoplastic agents, antioxidant agents, biological reaction inhibitors, botulinum toxin agents, chemotherapy agents, diagnostic agents, gene therapy agents, hormonal agents, mucolytic agents, radioprotective agents, radioactive agents including brachytherapy materials, tissue growth inhibitors, tissue growth enhancers, vasoactive agents, thrombolytic agents (i.e., clot busting agents), inducers of blood coagulation, and inhibitors of RBC aggregation in Sickle Cell Disease.

According to the invention, the aggregate comprises a molecule which is a therapeutic agent or pharmaceutically acceptable salt thereof, comprising an antithrombotic and/or thrombolytic agent, and/or a vasodilator. The therapeutic agent may be selected from any class suitable for the therapeutic objective. For example, if the objective is treating a disease or condition associated stenosis, the therapeutic agent may include antithrombotic or thrombolytic agent or fibrinolytic agents. By way of further example, if the desired treatment objective is treatment of cancer, the therapeutic agent may include radioactive material in the form of radioactive seeds providing radiation treatment directly into the tumor or close to it. Further, the therapeutic agent may be selected or arranged to provide therapeutic activity over a period of time.

Exemplary pharmaceutically active compound include, but are not limited to, those found in Harrison's Principles of Internal Medicine, 13th Edition, Eds. T.R. Harrison McGraw-Hill N.Y., NY; Physicians Desk Reference, 50th Edition, 1997, Oradell New Jersey, Medical Economics Co.; Pharmacological Basis of Therapeutics, 8th Edition, Goodman and Gilman, 1990; United States Pharmacopeia, The National Formulary, USP XII NF XVII, 1990; current edition of Goodman and Oilman's The Pharmacological Basis of Therapeutics; and current edition of The Merck Index*.*

In some embodiments of this and other aspects of the disclosure, the therapeutic agent is an antithrombotic or thrombolytic agent or fibrinolytic agent selected from the group consisting of anticoagulants, anticoagulant antagonists, antiplatelet agents, thrombolytic agents, thrombolytic agent antagonists, and any combinations thereof.

In some embodiments of this disclosure, the therapeutic agent is thrombogenic agent selected from the group consisting of thrombolytic agent antagonists, anticoagulant antagonists, pro-coagulant enzymes, pro-coagulant proteins, and any combinations thereof. Some exemplary thrombogenic agents include, but are not limited to, protamines, vitamin K1, amiocaproic acid (amicar), tranexamic acid (amstat), anagrelide, argatroban, cilstazol, daltroban, defibrotide, enoxaparin, fraxiparine, indobufen, lamoparan, ozagrel, picotamide, plafibride, tedelparin, ticlopidine, triflusal, collagen, and collagen-coated particles.

In some embodiments of this and other aspects of the invention, the therapeutic agent is a thrombolytic agent. As used herein, the term "thrombolytic agent" refers to any agent capable of inducing reperfusion by dissolving, dislodging or otherwise breaking up a clot, e.g., by either dissolving a fibrin-platelet clot, or inhibiting the formation of such a clot. Reperfusion occurs when the clot is dissolved and blood flow is restored. Exemplary thrombolytic agents include, but are not limited to, tissue-type plasminogen activator (t-PA), streptokinase (SK), prourokinase, urokinase (uPA), alteplase (also known as Activase®, Genentech, Inc.), reteplase (also known as r-PA or retavase®, Centocor, Inc.), tenecteplase (also known as TNKTM, Genentech, Inc.), Streptase® (AstraZeneca, LP), lanoteplase (Bristol-Myers Squibb Company), monteplase (Eisai Company, Ltd.), saruplase (also known as r-scu-PA and rescupaseTM, Grunenthal GmbH, Corp.), staphylokinase, and anisoylated plasminogen-streptokinase activator complex (also known as APSAC, Anistreplase and Eminase®, SmithKline Beecham Corp.). Thrombolytic agents also include other genetically engineered plasminogen activators. The invention can additionally employ hybrids, physiologically active fragments or mutant forms of the above thrombolytic agents. The term "tissue-type plasminogen activator" as used herein is intended to include such hybrids, fragments and mutants, as well as both naturally derived and recombinantly derived tissue-type plasminogen activator.

The term "anticoagulant" is meant to refer to any agent capable of prolonging the prothrombin and partial thromboplastin time tests and reducing the levels of prothrombin and factors VII, IX and X. Anticoagulants typically include cormarin derivatives and heparin as well as aspirin, which may also be referred to as an antiplatelet agent.

In some embodiments of this disclosure, the pharmaceutically active agent include those agents known in the art for treatment of inflammation or inflammation associated disorders, or infections. Exemplary anti-inflammatory agents include, but are not limited to, non-steroidal anti-inflammatory drugs (NSAIDs - such as aspirin, ibuprofen, or naproxen), coricosteroids (such as presnisone), anti-malarial medication (such as hydrochloroquine), methotrexrate, sulfasalazine, leflunomide, anti-TNF medications, cyclophosphamise, mycophenolate, dexamethasone, rosiglitazone, prednisolone, corticosterone, budesonide, estrogen, estrodiol, sulfasalazine, fenfibrate, provastatin, simvastatin, proglitazone, acetylsalicylic acid, mycophenolic acid, mesalamine, hydroxyurea, and analogs, derivatives, prodrugs, and pharmaceutically acceptable salts thereof.

In some embodiments of this and other aspects of the invention, the pharmaceutically active agent is a vasodilator. A vasodilator can be selected from the group consisting of alpha-adrenoceptor antagonists (alpha-blockers), agiotensin converting enzyme (ACE) inhibitors, angiotensin receptor blockers (ARBs), beta2-adrenoceptor agonists (β2-agonists), calcium-channel blockers (CCBs), centrally acting sympatholytics, direct acting vasodilators, endothelin receptor antagonists, ganglionic blockers, nitrodilators, phosphodiesterase inhibitors, potassium-channel openers, renin inhibitors, and any combinations thereof. Exemplary vasodilator include, but are not limited to, prazosin, terazosin, doxazosin, trimazosin, phentolamine, phenoxybenzamine, benazepril, captopril, enalapril, fosinopril, lisinopril, moexipril, quinapril, ramipril, candesartan, eprosartan, irbesartan, losartan, olmesartan, telmisartan, valsartan, Epinephrine, Norepinephrine, Dopamine, Dobutamine, Isoproterenol, amlodipine, felodipine, isradipine, nicardipine, nifedipine, nimodipine, nitrendipine, clonidine, guanabenz, guanfacine, α-methyldopa, hydralazine, Bosentan, trimethaphan camsylate, isosorbide dinitrate, isosorbide mononitrate, nitroglycerin, erythrityl tetranitrate, pentaerythritol tetranitrate, sodium nitroprusside, milrinone, inamrinone (formerly amrinone), cilostazol, sildenafil, tadalafil, minoxidil, aliskiren, and analogs, derivatives, prodrugs, and pharmaceutically acceptable salts thereof.

In some embodiments of this disclosure, the pharmaceutically active agent is a vasoconstrictor. As used herein, the term "vasoconstrictor" refers to compounds or molecules that narrow blood vessels and thereby maintain or increase blood pressure, and/or decrease blood flow. There are many disorders that can benefit from treatment using a vasoconstrictor. For example, redness of the skin (e.g., erythema or cuperose), which typically involves dilated blood vessels, benefit from treatment with a vasoconstrictor, which shrinks the capillaries thereby decreasing the untoward redness. Other descriptive names of the vasoconstrictor group include vasoactive agonists, vasopressor agents and vasoconstrictor drugs. Certain vasoconstrictors act on specific receptors, such as vasopressin receptors or adrenoreceptors. Exemplary vasoconstrictors include, but are not limited to, alpha-adrenoreceptor agonists, chatecolamines, vasopressin, vasopressin receptor modualors, calcium channel agonists, and other endogenous or exogenous vasoconstrictors.

In some embodiments, the vasoconstrictor is selected from the group consisting of aluminum sulfate, amidephrine, amphetamines, angiotensin, antihistamines, argipressin, bismuth subgallate, cafaminol, caffeine, catecholamines, cyclopentamine, deoxyepinephrine, dopamine, ephedrine, epinephrine, felypressin, indanazoline, isoproterenol, lisergic acid diethylamine, lypressin (LVP), lysergic acid, mephedrone, methoxamine, methylphenidate, metizoline, metraminol, midodrine, naphazoline, nordefrin, norepinephrine, octodrine, ornipressin, oxymethazoline, phenylefhanolamine, phenylephrine, phenylisopropylamines, phenylpropanolamine, phenypressin, propylhexedrine, pseudoephedrine, psilocybin, tetrahydralazine, tetrahydrozoline, tetrahydrozoline hydrochloride, tetrahydrozoline hydrochloride with zinc sulfate, tramazoline, tuaminoheptane, tymazoline, vasopressin, vasotocin, xylometazoline, zinc oxide, and the like.

In some embodiments, the vasoactive agent is a substance derived or extracted from a herbal source, selected from the group including ephedra sinica (ma huang), *polygonum* bistorta (bistort root), hamamelis virginiana (witch hazel), hydrastis canadensis (goldenseal), lycopus virginicus (bugleweed), aspidosperma quebracho (quebracho bianco), cytisus scoparius (scotch broom), cypress and salts, isomers, analogs and derivatives thereof.

In some embodiments of this disclosure, the pharmaceutically active agents is an anti-neoplastic, anti-proliferative, and/or anti-miotic agent. Exemplary anti-neoplastic/anti-proliferative/anti-miotic agents include, but are not limited to, paclitaxel, 5-fluorouracil, doxorubicin, daunorubicin, cyclosporine, cisplatin, vinblastine, vincristine, epothilones, methotrexate, azathioprine, adriamycin and mutamycin; endostatin, angiostatin and thymidine kinase inhibitors, cladribine, taxol, trapidil, halofuginone, plasmin, and analogs, derivatives, prodrugs, and pharmaceutically acceptable salts thereof. In some embodiments, the pharmaceutically active agent has a very short half-life in blood or serum. For example, the pharmaceutically active agent has a half-life of 1 minute, 2 minutes, 3 minutes, 4 minutes, 5 minutes, 5 minutes, 10 minutes, 20 minutes, 30 minutes, 40 minutes, 50 minutes, 1 hour, 2 hours, 3 hours, 4 hours, 5 hours, 6 hours, 7 hours, 8 hours, 8 hours, 9 hours, 10 hours, 11 hours, or 12 hours or less, in blood or serum. These short lifetime agents can have a local effect.

In some embodiments of this disclosure, the therapeutic agent is selected from the group consisting of aspirin, wafarin (coumadin), acenocoumarol, ancrod, anisindione, bromindione, clorindione, coumetarol, cyclocumarol, dextran, dextran sulfate sodium, dicumarol, diphenadione, ethyl biscoumacetate, ethylidene dicoumarol, fluindione, heparin, hirudin, lyapolate sodium, oxazidione, pentosan polysulfate, phenindione, phenprocoumon, phosvitin, picotamide, tioclomarol, dipyridamole (persantin), sulfinpyranone (anturane), ticlopidine (ticlid), tissue plasminogen activator (activase), plasmin, pro-urokinase, urokinase (abbokinase) streptokinase (streptase), and anistreplase/APSAC (eminase), and analogs, derivatives, prodrugs, and pharmaceutically acceptable salts thereof.

The therapeutic agent can be a radioactive material. Suitable radioactive materials include, for example, of ⁹⁰yttrium, ¹⁹²iridium, ¹⁹8gold, ¹²iodine, ¹³⁷cesium, ⁶⁰cobalt, ⁵⁵cobalt, ⁵⁶cobalt, ⁵⁷cobalt, ⁵⁷magnesium, ⁵⁵iron, ³²phosphorous, ⁹⁰strontium, ⁸¹rubidium, ²⁰⁶bismuth, ⁶⁷gallium, ⁷⁷bromine, ¹²⁹cesium, ⁷³selenium, ⁷²selenium, ⁷²arsenic, ¹⁰³palladium, ¹²³lead, ¹¹¹Indium, ⁵²iron, ¹⁶⁷thulium, ⁵⁷nickel, ⁶²zinc, ⁶²copper, ²⁰¹thallium and ¹²³iodine. Without wishing to be bound by a theory, aggregates comprising a radioactive material can be used to treat diseased tissue such as tumors, arteriovenous malformations, and the like.

The aggregates, red blood cells and microcapsules described herein can be used as in vivo imaging agents of tissues and organs in various biomedical applications including, but not limited to, imaging of blood vessel occlusions, tumors, tomographic imaging of organs, monitoring of organ functions, coronary angiography, fluorescence endoscopy, laser guided surgery, photoacoustic and sonofluorescence methods, and the like. The aggregates, red blood cells and microcapsules described herein are useful for detection and/or diagnosis of atherosclerotic plaques, or blood clots. When used in imaging applications, the aggregates, red blood cells and microcapsules described herein typically comprise an imaging agent, which can be covalently or noncovalently attached to the aggregate.

Accordingly, in some embodiments, the compound is an imaging agent. As used herein, the term "imaging agent" refers to an element or functional group in a molecule that allows for the detection, imaging, and/or monitoring of the presence and/or progression of a condition(s), pathological disorder(s), and/or disease(s). The imaging agent may be an echogenic substance (either liquid or gas), non-metallic isotope, an optical reporter, a boron neutron absorber, a paramagnetic metal ion, a ferromagnetic metal, a gamma-emitting radioisotope, a positron-emitting radioisotope, or an x-ray absorber.

Suitable optical reporters include, but are not limited to, fluorescent reporters and chemiluminescent groups. A wide variety of fluorescent reporter dyes are known in the art. Typically, the fluorophore is an aromatic or heteroaromatic compound and can be a pyrene, anthracene, naphthalene, acridine, stilbene, indole, benzindole, oxazole, thiazole, benzothiazole, cyanine, carbocyanine, salicylate, anthranilate, coumarin, fluorescein, rhodamine or other like compound. Suitable fluorescent reporters include xanthene dyes, such as fluorescein or rhodamine dyes, including, but not limited to, Alexa Fluor® dyes (InvitrogenCorp.; Carlsbad, Calif), fluorescein, fluorescein isothiocyanate (FITC), Oregon GreenTM, rhodamine, Texas red, tetrarhodamine isothiocynate (TRITC), 5-carboxyfluorescein (FAM), 2'7'-dimethoxy-4'5'-dichloro-6-carboxyfluorescein (JOE), tetrachlorofluorescein (TET), 6-carboxyrhodamine (R6G), N,N,N,N'-tetramefhyl-6-carboxyrhodamine (TAMRA), 6-carboxy-X-rhodamine (ROX). Suitable fluorescent reporters also include the naphthylamine dyes that have an amino group in the alpha or beta position. For example, naphthylamino compounds include 1-dimethylamino-naphthyl-5-sulfonate, 1-anilino-8-naphthalene sulfonate, 2-p-toluidinyl-6-naphthalene sulfonate, and 5-(2'-aminoethyl)aminonaphthalene-1-sulfonic acid (EDANS). Other fluorescent reporter dyes include coumarins, such as 3-phenyl-7-isocyanatocoumarin; acridines, such as 9-isothiocyanatoacridine and acridine orange; N-(p(2-benzoxazolyl)phenyl)maleimide; cyanines, such as Cy2, indodicarbocyanine 3 (Cy3), indodicarbocyanine 5 (Cy5), indodicarbocyanine 5.5 (Cy5.5), 3-(-carboxy-pentyl)-3'ethyl-5,5'-dimethyloxacarbocyanine (CyA); 1H,5H,11H, 15H-Xantheno[2,3,4-ij:5,6,7-i'j']diquinolizin-18-ium, 9-[2(or 4)-[[[6-[2,5-dioxo-1-pyrrolidinyl)oxy]-6-oxohexyl]amino]sulfonyl]-4(or 2)-sulfophenyl]-2,3,6,7,12,13,16,17octahydro-inner salt (TR or Texas Red); BODIPYTM dyes; benzoxadiazoles; stilbenes; pyrenes; and the like. Many suitable forms of these fluorescent compounds are available and can be used.

Examples of fluorescent proteins suitable for use as imaging agents include, but are not limited to, green fluorescent protein, red fluorescent protein (e.g., DsRed), yellow fluorescent protein, cyan fluorescent protein, blue fluorescent protein, and variants thereof (see, e.g., U.S. Pat. Nos. 6,403, 374, 6,800,733, and 7,157,566). Specific examples of GFP variants include, but are not limited to, enhanced GFP (EGFP), destabilized EGFP, the GFP variants described in Doan et al, Mol. Microbiol, 55:1767-1781 (2005), the GFP variant described in Crameri et al, Nat. Biotechnol., 14:315319 (1996), the cerulean fluorescent proteins described in Rizzo et al, Nat. Biotechnol, 22:445 (2004) and Tsien, Annu. Rev. Biochem., 67:509 (1998), and the yellow fluorescent protein described in Nagal et al, Nat. Biotechnol., 20:87-90 (2002). DsRed variants are described in, e.g., Shaner et al, Nat. Biotechnol., 22:1567-1572 (2004), and include mStrawberry, mCherry, mOrange, mBanana, mHoneydew, and mTangerine. Additional DsRed variants are described in, e.g., Wang et al, Proc. Natl. Acad. Sci. U.S.A., 101:16745-16749 (2004) and include mRaspberry and mPlum. Further examples of DsRed variants include mRFPmars described in Fischer et al, FEBS Lett., 577:227-232 (2004) and mRFPruby described in Fischer et al, FEBS Lett, 580:2495-2502 (2006).

Suitable echogenic gases include, but are not limited to, a sulfur hexafluoride or perfluorocarbon gas, such as perfluoromethane, perfluoroethane, perfluoropropane, perfluorobutane, perfluorocyclobutane, perfluropentane, or perfluorohexane.

Suitable non-metallic isotopes include, but are not limited to, ¹¹C, ¹⁴C, ¹³N, ¹⁸F, ¹²³I, ¹²⁴I, and ¹²⁵I.

Suitable radioisotopes include, but are not limited to, ⁹⁹mTc, ⁹⁵Tc, ¹¹¹In, ⁶²Cu, ⁶⁴Cu, Ga, ⁶⁸Ga, and ¹⁵³Gd.

Suitable paramagnetic metal ions include, but are not limited to, Gd(III), Dy(III), Fe(III), and Mn(II).

Suitable X-ray absorbers include, but are not limited to, Re, Sm, Ho, Lu, Pm, Y, Bi, Pd, Gd, La, Au, Au, Yb, Dy, Cu, Rh, Ag, and Ir.

In some embodiments, the radionuclide is bound to a chelating agent or chelating agent-linker attached to the aggregate. Suitable radionuclides for direct conjugation include, without limitation, ¹⁸F, ¹²⁴I, ¹²⁵I, ¹³¹I, and mixtures thereof. Suitable radionuclides for use with a chelating agent include, without limitation, ⁴⁷Sc, ⁶⁴Cu, ⁶⁷Cu, ⁸⁹Sr, ⁸⁶Y, ⁸⁷Y, ⁹⁰Y, ¹⁰⁵Rh, ¹¹¹Ag, ¹¹¹In, ¹¹⁷mSn, ¹⁴⁹Pm, ¹⁵³Sm, ¹⁶⁶Ho, ¹⁷⁷Lu, ¹⁸⁶Re, ¹⁸⁸Re, ²¹¹At, ²¹²Bi, and mixtures thereof. Suitable chelating agents include, but are not limited to, DOTA, BAD, TETA, DTPA, EDTA, NTA, HDTA, their phosphonate analogs, and mixtures thereof. One of skill in the art will be familiar with methods for attaching radionuclides, chelating agents, and chelating agent-linkers to the nanoparticles.

A detectable response generally refers to a change in, or occurrence of, a signal that is detectable either by observation or instrumentally. In certain instances, the detectable response is fluorescence or a change in fluorescence, e.g., a change in fluorescence intensity, fluorescence excitation or emission wavelength distribution, fluorescence lifetime, and/or fluorescence polarization. One of skill in the art will appreciate that the degree and/or location of labeling in a subject or sample can be compared to a standard or control (e.g., healthy tissue or organ). In certain other instances, the detectable response the detectable response is radioactivity (i.e., radiation), including alpha particles, beta particles, nucleons, electrons, positrons, neutrinos, and gamma rays emitted by a radioactive substance such as a radionuclide.

Specific devices or methods known in the art for the in vivo detection of fluorescence, e.g., from fluorophores or fluorescent proteins, include, but are not limited to, in vivo near-infrared fluorescence (see, e.g., Frangioni, Curr. Opin. Chem. Biol, 7:626-634 (2003)), the MaestroTM in vivo fluorescence imaging system (Cambridge Research & Instrumentation, Inc.; Woburn, Mass.), in vivo fluorescence imaging using a flying-spot scanner (see, e.g., Ramanujam et al, IEEE Transactions on Biomedical Engineering, 48:1034-1041 (2001), and the like. Other methods or devices for detecting an optical response include, without limitation, visual inspection, CCD cameras, video cameras, photographic film, laser-scanning devices, fluorometers, photodiodes, quantum counters, epifluorescence microscopes, scanning microscopes, flow cytometers, fluorescence microplate readers, or signal amplification using photomultiplier tubes.

Any device or method known in the art for detecting the radioactive emissions of radionuclides in a subject is suitable for use in the present invention. For example, methods such as Single Photon Emission Computerized Tomography (SPECT), which detects the radiation from a single photon gamma-emitting radionuclide using a rotating gamma camera, and radionuclide scintigraphy, which obtains an image or series of sequential images of the distribution of a radionuclide in tissues, organs, or body systems using a scintillation gamma camera, may be used for detecting the radiation emitted from a radiolabeled aggregate. Positron emission tomography (PET) is another suitable technique for detecting radiation in a subject.

One of skill in the art will understand that the methods described herein for attaching ligands to the nanoparticles can be also be used for attaching imaging agents to the nanoparticles. In addition, an ordinarily skilled artisan will also be familiar with other methods for attaching imaging agents to nanoparticle and/or fabricating nanoparticles that comprise an imaging agent.

In some embodiments, the aggregate comprises at least one therapeutic agent and at least one imaging agent.

Without wishing to be bound by a theory, aggregation of nanoparticles into an aggregate reduces the rate of release and/or amount released of the compound(s) associated with the aggregate or prevents the compound(s) from coming in contact with the cells that would absorb or adsorb the compound(s). This can be due to the reduction in the surface area of aggregate relative to the total surface area of the individual nanoparticle. Accordingly, in some embodiments, the associated compound is released at a higher rate and/or amount from a disaggregated aggregate relative to release from to a non-disaggregated aggregate. For example, the rate of release from a disaggregated aggregate is at least 10%, at least 20%, at least 30%, at least 40%, at least 50%, at least 60%, at least 70%, at least 80%, at least 90%, at least 1-fold, at least 2-fold, at least 5-fold, at least 10-fold, at least 20-fold, at least 30-fold, at least 40-fold, at least 50-fold, or at least 100-fold or higher, relative to release from a non-disaggregated aggregate.

Because aggregation of nanoparticles into an aggregate reduces the rate of release and/or amount released of the compound(s) associated with the aggregate, the aggregates described herein can be used as slow release drug carriers to prolong circulating half-life of therapeutic agents. For example, aggregates that only undergo partial disaggregation under normal blood vessel shear stress will not release, or release very little, of the nanoparticles and a molecule associated therewith. This can increase circulation life of the nanoparticle and the associated therapeutic agent. Thus, aggregates that disaggregate partially, e.g., less than 20%, less than 15%, less than 10%, less than 5%, less than 4%, less than 3%, or less than 2% under normal blood vessel shear stress (e.g., less than 70 dyne/cm², less than 60 dyne/cm², less than 50 dyne/cm², less than 40 dyne/cm², less than 30 dyne/cm², less than 25 dyne/cm², less than 20 dyne/cm², or less than 15 dyne/cm²) can be used as slow release drug carriers to increase circulating half-life of therapeutic agents.

In some embodiments, the aggregates that disaggregate partially, e.g., less than 20%, less than 15%, less than 10%, less than 5%, less than 4%, less than 3%, or less than 2% in the shear stress range of from about 1dyne/cm² to about 25dyne/cm², from about 2dyne/cm² to about 20dyne/cm², or from about 5dyne/cm² to about 15dyne/cm² can be use as slow release drug carriers to increase the circulating half-life of therapeutic agents.

Additionally, this slow release can occur throughout the entire vasculature over time. This can be useful in long term targeting of endothelium under physiological shear stress conditions.

### Ligands

A wide variety of entities can be coupled to the nanoparticles, red blood cells and microcapsules. Preferred moieties are ligands, which are coupled, preferably covalently, either directly or indirectly via an intervening tether. In preferred embodiments, a ligand alters the distribution, targeting or lifetime of the nanoparticle, red blood cell or microcapsule into which it is incorporated. In preferred embodiments a ligand provides an enhanced affinity for a selected target, e.g., molecule, cell or cell type, compartment, e.g., a cellular or organ compartment, tissue, organ or region of the body, as, e.g., compared to a species absent such a ligand. Ligands providing enhanced aggregation are termed aggregating ligands herein.

Ligands providing enhanced affinity for a selected target are also termed targeting ligands herein. As used herein, the term "targeting ligand" refers to a molecule that binds to or interacts with a target molecule. Typically the nature of the interaction or binding is noncovalent, e.g., by hydrogen, electrostatic, or van der waals interactions, however, binding may also be covalent.

A ligand can be selected from the group consisting of peptides, polypeptides, proteins, peptidomimetics, glycoproteins, lectins, nucleosides, nucleotides, nucleic acids, monosaccharides, disaccharides, trisaccharides, oligosaccharides, polysaccharides, lipopolysaccharides, vitamins, steroids, hormones, cofactors, receptors, receptor ligands, and analogs and derivatives thereof. According to the invention, the ligand is as defined in claim 9.

In some embodiments of this and other aspects of the invention, the ligand is selected from the group consisting of polylysine (PLL), poly L-aspartic acid, poly L-glutamic acid, styrene-maleic acid anhydride copolymer, poly(L-lactide-co-glycolied) copolymer, divinyl ether-maleic anhydride copolymer, N-(2-hydroxypropyl)methacrylamide copolymer (HMPA), polyethylene glycol (PEG), polyvinyl alcohol (PVA), polyurethane, poly(2-ethylacryllic acid), N-isopropylacrylamide polymers, polyphosphazine, polyethylenimine, cspermine, spermidine, polyamine, pseudopeptide-polyamine, peptidomimetic polyamine, dendrimer polyamine, arginine, amidine, protamine, thyrotropin, melanotropin, lectin, surfactant protein A, mucin, transferrin, bisphosphonate, polyglutamate, polyaspartate, an aptamer, asialofetuin, hyaluronan, procollagen, insulin, transferrin, albumin, acridines, cross- psoralen, mitomycin C, TPPC4, texaphyrin, Sapphyrin, polycyclic aromatic hydrocarbons (e.g., phenazine, dihydrophenazine), bile acids, cholesterol, cholic acid, adamantane acetic acid, 1-pyrene butyric acid, dihydrotestosterone, 1,3-Bis-O(hexadecyl)glycerol, geranyloxyhexyl group, hexadecylglycerol, borneol, menthol, 1,3-propanediol, heptadecyl group, palmitic acid, myristic acid, O3-(oleoyl)lithocholic acid, O3-(oleoyl)cholenic acid, dimethoxytrityl, or phenoxazine), RGD peptide, radiolabeled markers, haptens, naproxen, aspirin, dinitrophenyl, HRP, AP, lectins, vitamin A, vitamin E, vitamin K, vitamin B, folic acid, B12, riboflavin, biotin, pyridoxal, taxon, vincristine, vinblastine, cytochalasin, nocodazole, japlakinolide, latrunculin A, phalloidin, swinholide A, indanocine, myoservin, tumor necrosis factor alpha (TNFalpha), interleukin-1 beta, gamma interferon, GalNAc, galactose, mannose, mannose-6P, clusters of sugars such as GalNAc cluster, mannose cluster, galactose cluster, an aptamer, integrin receptor ligands, chemokine receptor ligands, serotonin receptor ligands, PSMA, endothelin, GCPII, somatostatin, and any combinations thereof.

In some embodiments of this and other aspects of the invention, the ligand is a peptide selected from the group consisting of, SEQ ID NO: 1 (CREKA), SEQ ID NO: 2 (CRKRLDRNK), SEQ ID NO: 3 (CHVLWSTRC), SEQ ID NO: 4 (ALEALAEALEALAEA), SEQ ID NO: 5 (KFFKFFKFFK (Bacterial cell wall permeating peptide)), SEQ ID NO: 6 (AALEALAEALEALAEALEALAEAAAAGGC (GALA)), SEQ ID NO: 7 (ALAEALAEALAEALAEALAEALAAAAGGC (EALA)), SEQ ID NO: 8 (GLFEAIEGFIENGWEGMIWDYG (INF-7)), SEQ ID NO: 9 (GLFGAIAGFIENGWEGMIDGWYG (Inf HA-2)), SEQ ID NO: 10 (GLF EAI EGFI ENGW EGMI DGWYGC GLF EAI EGFI ENGW EGMI DGWYGC (diINF-7)), SEQ ID NO: 11 (GLF EAI EGFI ENGW EGMI DGGC GLF EAI EGFI ENGW EGMI DGGC (diINF-3)), SEQ ID NO: 12 (GLFGALAEALAEALAEHLAEALAEALEALAAGGSC (GLF)), SEQ ID NO: 13 (GLFEAIEGFIENGWEGLAEALAEALEALAAGGSC (GALA-INF3)), SEQ ID NO: 14 (GLF EAI EGFI ENGW EGnI DG K GLF EAI EGFI ENGW EGnI DG (INF-5, n is norleucine)), SEQ ID NO: 15 (RQIKIWFQNRRMKWKK (penetratin)), SEQ ID NO: 16 (GRKKRRQRRRPPQC (Tat fragment 48-60)), SEQ ID NO: 17 (GALFLGWLGAAGSTMGAWSQPKKKRKV (signal sequence based peptide)), SEQ ID NO: 18 (LLIILRRRIRKQAHAHSK (PVEC)), SEQ ID NO: 19 (WTLNSAGYLLKINLKALAALAKKIL (transportan)), SEQ ID NO: 20 (KLALKLALKALKAALKLA (amphiphilic model peptide)), SEQ ID NO: 21 (RRRRRRRRR (Arg9)), SEQ ID NO: 22 (LLGDFFRKSKEKIGKEFKRIVQRIKDFLRNLVPRTES (LL-37)), SEQ ID NO: 23 (SWLSKTAKKLENSAKKRISEGIAIAIQGGPR (cecropin PI)), SEQ ID NO: 24 (ACYCRIPACIAGERRYGTCIYQGRLWAFCC (α-defensin)), SEQ ID NO: 25 (DHYNCVSSGGQCLYSACPIFTKIQGTCYRGKAKCCK (β-defensin)), SEQ ID NO: 26 (RRRPRPPYLPRPRPPPFFPPRLPPRIPPGFPPRFPPRFPGKR-NH2 (PR-39)), SEQ ID NO: 27 ILPWKWPWWPWRR-NH2 (indolicidin)), SEQ ID NO: 28 (AAVALLPAVLLALLAP (RFGF)), SEQ ID NO: 29 (AALLPVLLAAP (RFGF analogue)), SEQ ID NO: 30 (RKCRIVVIRVCR (bactenecin)), cecropins, lycotoxins, paradaxins, buforin, CPF, bombinin-like peptide (BLP), cathelicidins, ceratotoxins, S. *clava* peptides, hagfish intestinal antimicrobial peptides (HFIAPs), magainines, brevinins-2, dermaseptins, melittins, pleurocidin, H₂A peptides, Xenopus peptides, esculentinis-1, caerins, and any analogs and derivatives thereof.

In some embodiments of this and other aspects of the invention, the ligand is an aggregating ligand. Without wishing to be bound by a theory, an aggregating ligand can decrease the rate of disaggregation by at least 10%, at least 20%, at least 30%, at least 40%, at least 50%, at least 60%, at least 70%, at least 80%, or at least 90% or more, relative to a control.

In some embodiments, the ligand is fluorescent reporter or a chemiluminescent molecule.

In some embodiments, of this and other aspects of the invention, a nanoparticle comprises both a targeting ligand and the target molecule. Without wishing to be bound by a theory, binding of the targeting ligand on one nanoparticle to a target molecule on a second nanoparticle enhances aggregation.

### Linking of molecules

A molecule (e.g. a compound or a ligand) can be conjugated to a nanoparticle, red blood cell, or microcapsule using any of a variety of methods known to those of skill in the art. The molecule can be coupled or conjugated to the nanoparticle, red blood cell, or microcapsule covalently or non-covalently. The covalent linkage between the molecule and the nanoparticle, red blood cell, or microcapsule can be mediated by a linker. The non-covalent linkage between the molecule and the nanoparticle, red blood cell, or microcapsule can be based on ionic interactions, van der Waals interactions, dipole-dipole interactions, hydrogen bonds, electrostatic interactions, and/or shape recognition interactions.

As used herein, the term "linker" means an organic moiety that connects two parts of a compound. Linkers typically comprise a direct bond or an atom such as oxygen or sulfur, a unit such as NH, C(O), C(O)NH, SO, SO₂, SO₂NH or a chain of atoms, such as substituted or unsubstituted C₁-C₆ alkyl, substituted or unsubstituted C₂-C₆ alkenyl, substituted or unsubstituted C₂-C₆ alkynyl, substituted or unsubstituted C₆-C₁₂ aryl, substituted or unsubstituted C₅-C₁₂ heteroaryl, substituted or unsubstituted C₅-C₁₂ heterocyclyl, substituted or unsubstituted C₃-C₁₂ cycloalkyl, where one or more methylenes can be interrupted or terminated by O, S, S(O), SO₂, NH, C(O).

The molecule can be conjugated with the nanoparticle, red blood cell, or microcapsule by an affinity binding pair. The term "affinity binding pair" or "binding pair" refers to first and second molecules that specifically bind to each other. One member of the binding pair is conjugated with the molecule while the second member is conjugated with the nanoparticle, red blood cell, or microcapsule. As used herein, the term "specific binding" refers to binding of the first member of the binding pair to the second member of the binding pair with greater affinity and specificity than to other molecules.

Exemplary binding pairs include any haptenic or antigenic compound in combination with a corresponding antibody or binding portion or fragment thereof (e.g., digoxigenin and anti-digoxigenin; mouse immunoglobulin and goat antimouse immunoglobulin) and nonimmunological binding pairs (e.g., biotin-avidin, biotin-streptavidin, hormone [e.g., thyroxine and cortisol-hormone binding protein, receptor-receptor agonist, receptor-receptor antagonist (e.g., acetylcholine receptor-acetylcholine or an analog thereof), IgG-protein A, lectin-carbohydrate, enzyme-enzyme cofactor, enzyme-enzyme inhibitor, and complementary oligonucleoitde pairs capable of forming nucleic acid duplexes), and the like. The binding pair can also include a first molecule which is negatively charged and a second molecule which is positively charged.

One example of using binding pair conjugation is the biotin-avidin or biotin-streptavidin conjugation. In this approach, one of the molecule or nanoparticle, red blood cell, or microcapsule is biotinylated and the other is conjugated with avidin or streptavidin. Many commercial kits are also available for biotinylating molecules, such as proteins.

Another example of using binding pair conjugation is the biotin-sandwich method. *See, e.g.,* example Davis et al., Proc. Natl. Acad. Sci. USA, 103: 8155-60 (2006). The two molecules to be conjugated together are biotinylated and then conjugated together using tetravalent streptavidin as a linker.

Still another example of using binding pair conjugation is double-stranded nucleic acid conjugation. In this approach, one of the molecule or nanoparticle, red blood cell, or microcapsule is conjugated with a first strand of the double-stranded nucleic acid and the other is conjugated with the second strand of the double-stranded nucleic acid. Nucleic acids can include, without limitation, defined sequence segments and sequences comprising nucleotides, ribonucleotides, deoxyribonucleotides, nucleotide analogs, modified nucleotides and nucleotides comprising backbone modifications, branchpoints and nonnucleotide residues, groups or bridges.

### Aggregate Fabrication

Also envisioned is a method for preparing an aggregate described herein, the method comprising: (i) fabricating a plurality of nanoparticles; (ii) aggregating said plurality of nanoparticle into micron sized particles. The fabricated nanoparticles may also be further subjected to centrifugation to decrease the concentration of single unbound nanoparticles in the aggregate. The fabricated nanoparticles may also be further subjected to centrifugation to decrease the concentration of single unbound nanoparticles in the aggregate.

After aggregation, particles of desired size can be selected by employing various techniques well known to a skilled artisan, such as size exclusion chromatography or use of track etched filters In one non-limiting example, aggregated particles can be filtered using a filter with appropriate pore size. In another non-limiting example, aggregated particles can be subjected to density gradient centrifugation.

Accordingly, in some embodiments, the method further comprises the step of selecting aggregated particles ≥ 1µm, ≥ 2µm, ≥ 3µm, ≥ 4µm, ≥ 5µm, ≥ 6µm, ≥ 7µm, ≥ 8µm, ≥ 8µm, or ≥ 10µm in size.

In some embodiments, the method further comprises the step of selecting aggregated particles ≤ 20µm, ≤ 15µm, ≤ 10µm, or ≤ 5µm in size.

In some embodiments, the method further comprises selecting aggregated particles of a certain size range, e.g., from 1µm to 50µm, from 1µm to 25µm, from 1µm to 20µm, from 1µm to 10µm, or from 0.5µm to 5µm. This can be accomplished by first selecting particles of size less than the upper size limit and then from those particles selecting particles of size greater than the lower size limit or vice-versa.

Various methods can be employed to fabricate nanoparticles of suitable size for aggregation. These methods include vaporization methods (e.g., free jet expansion, laser vaporization, spark erosion, electro explosion and chemical vapor deposition), physical methods involving mechanical attrition (e.g., the pearlmilling technology developed by Elan Nanosystems of Dublin, Ireland), and interfacial deposition following solvent displacement.

The solvent displacement method is relatively simple to implement on a laboratory or industrial scale and can produce nanoparticles able to pass through a 0.22 µm filter. The size of nanoparticles produced by this method is sensitive to the concentration of polymer in the organic solvent, to the rate of mixing, and to the surfactant employed in the process. Although use of the solvent displacement method with the surfactant sodium dodecyl sulfate (SDS) has yielded small nanoparticles (<100 nm), SDS is not ideal for a pharmaceutical formulation. However, similar natural surfactants (e.g., cholic acid or taurocholic acid salts) can be substituted for SDS to obtain similarly sized nanoparticles. Taurocholic acid, the conjugate formed from cholic acid and taurine, is a fully metabolizable sulfonic acid with very similar amphipathic solution chemistry to SDS. An analog of taurocholic acid, tauroursodeoxycholic acid (TUDCA), is not toxic and is actually known to have neuroprotective and anti-apoptotic properties. TUDCA is a naturally occurring bile acid and is a conjugate of taurine and ursodeoxycholic acid (UDCA). UDCA is an approved drug (ACTIGALL®, Watson Pharmaceuticals) for the treatment of gallbladder stone dissolution. Other naturally occurring anionic surfactants (e.g., galactocerebroside sulfate), neutral surfactants (e.g., lactosylceramide) or zwitterionic surfactants (e.g., sphingomyelin, phosphatidyl choline, palmitoyl carnitine) can be used in place of SDS or other surfactants that have been commonly employed in nanoparticle formulation studies. Other excipients that are generally recognized as safe, such as those used to solubilize the basic form of gacyclidine, can also be used to prepare nanoparticles. Such excipients include a polyoxyethylene fatty acid ester (e.g., polysorbate 80 (e.g., TWEEN 80®)), a polyglycol mono or diester of 12-hydroxy steric acid (e.g., SOLUTOL® HS 15), and CAPTISOL®. Poloxamers such as (but not limited to) poloxamer 407 can also be used.

A sampling of various surfactants can be used in order to determine the optimal surfactants for small (e.g., <200 nm), non-toxic drug-containing nanoparticles. Surfactant concentrations also affect the formation of the nanoparticles, their density and their size. A surfactant concentration can be optimized for each polymer composition, desired drug concentration, and intended use.

Of the various organic solvents previously employed in nanoparticle formulation, acetone is attractive because of its prior use in preparing filterable nanoparticles, its low toxicity, and its ease of handling. Various polymers composed of Land D,L-lactic acid (PLA) or mixtures of lactic acid and glycolic acid (poly(lactide-co-glycolide)) (PLGA) are soluble in acetone, with the exception of 100% L-PLA and 100% glycolic acid (PGA). Polymers composed of 100% L-PLA will dissolve in methylene chloride and polymers composed of either 100% L-PLA or 100% PGA will dissolve in hexafluoroisopropanol (HFIP).

Rapid mixing can be employed when preparing nanoparticles using the solvent displacement method. In some such embodiments, a stirring rate of 500 rpm or greater is typically employed. Slower solvent exchange rates during mixing result in larger particles. Fluctuating pressure gradients are used to produce high Reynolds numbers and efficient mixing in fully developed turbulence. Use of high gravity reactive mixing has produced small nanoparticles (10 nm) by achieving centrifugal particle acceleration similar to that achieved by turbulent mixing at high Reynolds numbers.

Sonication is one method that can provide turbulent mixing. Sonication is the method most commonly employed with the double emulsion nanoparticle fabrication method, but is less suited to the solvent displacement method. Sonication can be performed by mixing two liquid streams (e.g. one stream having dissolved particle polymeric material and the other stream having a drug and/or combination of drugs that will cause the particles to come out of solution and solidify) passing through a tube with an inline ultrasonic vibrating plate at the point of stream intersection. Formation of very small liquid droplets by vibrational atomization has also been employed in the fabrication of nanoparticles. For example, the DMP-2800 MEMS-based piezoelectric micropump (inkjet) system produced by the Spectra Printing Division (Lebanon, N.H.) of Dimatix, Inc. (Santa Clara, Calif.) forms a 10-50 pL (1-5 x 10⁻¹¹ liter) sized liquid droplet at 100,000 pL/s. Micropumps (inkjet systems) offer uniform mixing and the ability to reliably translate the process from lab to production scale, but production of nanoparticles smaller than 200 nm will still rely on mixing dynamics (i.e., the solidification timing of the precipitated solid or liquid intermediates produced on mixing) when piezoelectric micropumps are used to produce small, polymer-laden droplets. Temperature, surfactant and solvent composition are important variables in using this approach, as they modify the solidification dynamics and the density of the produced nanoparticle.

The nanoparticles can be induced to form aggregates by a wide variety of methods available and well known to the skilled artisan. Many hydrophobic nanoparticles, such PLGA based nanoparticles, can self-aggregate in aqueous solution. See for example, C.E. Astete and C.M. Sabliov, J. Biomater. Sci, Polymer Ed. 17:247 (2006). Accordingly, a concentrated solution comprising the nanoparticles can be stored at room temperature or lower temperature for a period of time. In some embodiments, the storage temperature is 4°C or lower. Without limitation, the storage period can last from minutes to days or weeks. For example, the storage period is 1-day, 2-days, 3-days, 4-days, 5-days, 6-days, 1-week, 2-week or more.

Alternatively, a concentrated solution of nanoparticles can be spray dried to form aggregates. See for example, Sung, et al., Pharm. Res. 26:1847 (2009) and Tsapis, et al., Proc. Natl. Acad. Sci. USA, 99:12001 (2002).

The concentrated solution can comprise 2mg/ml, 3 mg/ml, 4mg/ml, 5mg/ml, 6 mg/ml, 7mg/ml, 8mg/ml, 9mg/ml, 10mg/ml, 11mg/ml, 12mg/ml, 13mg/ml, 14mg/ml, 15mg/ml, 16mg/ml, 17mg/ml, 18mg/ml, 19mg/ml, 20mg/ml or more of the nanoparticles.

Other methods of forming aggregates include, but are not limited to, the w/o/w emulsion method and the simple solvent displacement method.

In one non-limiting example, the nanoparticles are fabricated from PLGA polymers. The PLGA polymer may be conjugated with PEG and/or a ligand. Accordingly, in some embodiments of this and other aspects of the invention, the nanoparticles are fabricated from PEG-PLGA polymers to which the peptide CREKA (SEQ ID NO: 1), CRKRLDRNK (SEQ ID NO: 2), or CHVLWSTRC (SEQ ID NO: 3). The CREKA (SEQ ID NO: 1) peptide is known to home in to a wide variety of tumors. Without wishing to be bound by a theory, the CREKA (SEQ ID NO: 1) peptide recognizes clotted blood, which is present in the lining of tumor vessels but not in vessels of normal tissues. Additionally, CREKA (SEQ ID NO: 1)peptide is used to target fibrin located on the luminal surface of atherosclerotic plaque.

The CRKRLDRNK (SEQ ID NO: 2) peptide is a known peptide targeting inflamed endothelium.

The CHVLWSTRC (SEQ ID NO: 3) peptide is a known peptide, which targets islet endothelial cells.

### Treatment of stenosis

In another aspect, the invention provides a pharmaceutical composition for use in a method for treating stenosis and/or a stenotic lesion in a subject, the method comprising administering to a subject in need thereof an aggregate according to the invention as described herein.

As used herein, the term "stenosis" refers to narrowing or stricture of a hollow passage (e.g., a duct or canal) in the body. The term "vascular stenosis" refers to occlusion or narrowing of a canal or lumen of the circulatory system. Vascular stenosis often results from fatty deposit (as in the case of atherosclerosis), excessive migration and proliferation of vascular smooth muscle cells and endothelial cells, acute narrowing due to clot formation, or as a result of vascular malformation. As used herein, the term "vascular stenosis" includes occlusive lesions. Arteries are particularly susceptible to stenosis. The term "stenosis" as used herein specifically includes initial stenosis and restenosis. Typical examples of blockages within a canal or lumen include *in situ* or embolized atheromatous material or plaques, aggregations of blood components, such as platelets, fibrin and/or other cellular components, in clots resulting from disease or injury or at the site of wound healing. Clot-forming conditions include thrombosis, embolisms and in an extreme case, abnormal coagulation states. Other vascular blockages include blockages resulting from an infection by a microorganism or macroorganism within the circulatory system, such as fungal or heartworm infections. Sickle cell disease also can result in vessel obstruction as a result of RBC sickling and stacking into structures that are larger than the lumen of the microvessel. Thus, during sickle cell crisis, RBC change shape/stiffness and can occlude blood vessel. This phenomena is also present during crisis stages of malaria.

The term "restenosis" refers to recurrence of stenosis after treatment of initial stenosis with apparent success. For example, "restenosis" in the context of vascular stenosis, refers to the reoccurrence of vascular stenosis after it has been treated with apparent success, e.g. by removal of fatty deposit by balloon angioplasty. One of the contributing factors in restenosis is intimal hyperplasia. The term "intimal hyperplasia", used interchangeably with "neointimal hyperplasia" and "neointimal formation", refers to thickening of the inner most layer of blood vessels, intimal, as a consequence of excessive proliferation and migration of vascular smooth muscle cells and endothelial cells. The various changes taking place during restenosis are often collectively referred to as "vascular wall remodeling."

The terms "balloon angioplasty" and "percutaneous transluminal coronary angioplasty" (PTCA) are often used interchangeably, and refer to a non-surgical catheter-based treatment for removal of plaque from the coronary artery. Stenosis or restenosis often lead to hypertension as a result of increased resistance to blood flow.

The term "hypertension" refers to abnormally high blood pressure, i.e. beyond the upper value of the normal range.

Some exemplary causes of stenosis and/or stenotic lesion include, but are not limited to, trauma or injury, atherosclerosis, birth defects, diabetes, iatrogenic, infection, inflammation, ischemia, neoplasm, vasospasm, coronary vasospasm, Raynaud's phenomenon, stroke, blood clotting, Moyamoya disease, Takayasu's disease, polyarteritis nodosa, disseminated lupus erythematous, rheumatoid arthritis, tumors of the spine, Paget's disease of bone, fluorosis, extracorporeal devices (e.g., hemodialysis, blood pumps, etc.), thrombotic and/or embolic disorders, Sickle Cell Disease, and any combinations thereof.

As used herein, the term "thrombotic and/or embolic disorders" means acute or chronic pathological states or conditions resulting from occlusion or partial occlusion of a blood vessel due to thrombus or embolus. Similarly, the term "thrombotic or embolic occlusion" means occlusion or partial occlusion of a blood vessel due to thrombus or embolus. Examples of thrombotic and embolic disorders include, but are not limited to cerebral thrombotic and embolic disorders such as cerebral infarct (stroke), transient ischemic attack and vascular dementia; thrombotic and embolic disorders of the heart such as myocardial infarct, acute coronary syndrome, unstable angina and ischemic sudden death; pulmonary embolism; pulmonary or renal infarcts, peripheral circulatory disorders and deep vein thrombosis.

In some embodiments of this and other aspects of the invention, stenosis or stenotic lesion is selected from the group consisting of intermittent claudication (peripheral artery stenosis), angina (coronary artery stenosis), carotid artery stenosis (leads to strokes and transient ischaemic episodes), aortic stenosis, buttonhole stenosis, calcific nodular stenosis, coronary ostial stenosis, double aortic stenosis, fish-mouth mitral stenosis, idiopathic hypertrophic subaortic stenosis, infundibular stenosis, mitral stenosis, muscular subaortic stenosis, pulmonary stenosis, subaortic stenosis, subvalvar stenosis, supravalvar stenosis, tricuspid stenosis, renal artery stenosis, pyloric stenosis (gastric outflow obstruction), obstructive jaundice (biliary tract stenosis), bowel obstruction, phimosis, hydrocephalus, stenosing tenosynovitis, spinal stenosis, subglottic stenosis (SGS), and any combinations thereof.

### Treatment of internal hemorrhage (not part of the claimed invention)

As used herein, the term "internal hemorrhage" refers to bleeding that is occurring inside the body. Such bleeding can be a serious depending on wherein it occurs (e.g., brain, stomach, lungs), and can potentially cause death and cardiac arrest if proper medical treatment is not quickly received. Accordingly, described herein is a method for treating internal hemorrhage or a hemorrhagic disorder in a subject, the method comprising administering to a subject in need thereof an aggregate described herein. Depending on the nature of hemorrhage, the shear stress can be high at or near the bleed site.

Internal hemorrhage can result from a trauma, blood vessel rupture from high blood pressure, infection (e.g., Ebola, Marburg), cancer, scurvy, hepatoma, autoimmune thrombocytopenia, ectopic pregnancy, malignant hypothermia, ovarian cysts, liver cancer, vitamin K deficiency, hemophilia, or adverse effect of a medication.

As used herein the term "hemorrhagic disorder" means acute or chronic pathological state or condition resulting from bleeding from damaged blood vessel. Examples of hemorrhagic disorders include, but are not limited to, cerebral hemorrhages such as intracerebral hemorrhage (ICH), subarachnoid hemorrhage (SAH) and hemorrhagic stroke.

The aggregates described herein can also be used in extracorporeal devices, such as hemodialysis devices (possibly also artificial blood vessel/valves etc) - these can cause elevated shear stress that induce shear activation of platelets etc. Aggregates described herein can be added to the extracorporeal device to release anti-platelets drug when elevated shear stress exists in these extracorporeal devices.

The aggregates can also be used for detection of abnormal flow in the body/extracorporeal devices. For example by measuring the release rate and/or amount of a label molecule.

Additionally, the aggregates can also be used in combination with embolization treatments. Embolization refers to the introduction of various substances into the circulation to occlude vessels, either to arrest or prevent hemorrhaging; to devitalize a structure, tumor, or organ by occluding its blood supply; or to reduce blood flow to an arteriovenous malformation. Thus, embolization includes selective occlusion of blood vessels by purposely introducing emboli in the blood vessels. Embolization is used to treat a wide variety of conditions affecting different organs of the human body, including arterivenous malformations, cerebral aneurysm, gastrointestinal bleeding, epistaxis, primary post-partum hemorrhage, surgical hemorrhage, slow or stop blood supply thus reducing the size of a tumor, liver lesions, kidney lesions and uterine fibroids.

### Pharmaceutical Compositions

For administration to a subject, the aggregates can be provided in pharmaceutically acceptable compositions. These pharmaceutically acceptable compositions comprise an aggregate, formulated together with one or more pharmaceutically acceptable carriers (additives) and/or diluents. As described in detail below, the pharmaceutical compositions of the present invention can be specially formulated for administration in solid or liquid form, including those adapted for the following: (1) oral administration, for example, drenches (aqueous or non-aqueous solutions or suspensions), gavages, lozenges, dragees, capsules, pills, tablets (e.g., those targeted for buccal, sublingual, and systemic absorption), boluses, powders, granules, pastes for application to the tongue; (2) parenteral administration, for example, by subcutaneous, intramuscular, intravenous or epidural injection as, for example, a sterile solution or suspension, or sustained-release formulation; (3) topical application, for example, as a cream, ointment, or a controlled-release patch or spray applied to the skin; (4) intravaginally or intrarectally, for example, as a pessary, cream or foam; (5) sublingually; (6) ocularly; (7) transdermally; (8) transmucosally; or (9) nasally. Additionally, compounds can be implanted into a patient or injected using a drug delivery system. See, for example, Urquhart, et al., Ann. Rev. Pharmacol. Toxicol. 24: 199-236 (1984); Lewis, ed. "Controlled Release of Pesticides and Pharmaceuticals" (Plenum Press, New York, 1981); U.S. Pat. No. 3,773,919; and U.S. Pat. No. 35 3,270,960.

As used here, the term "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used here, the term "pharmaceutically-acceptable carrier" means a pharmaceutically-acceptable material, composition or vehicle, such as a liquid or solid filler, diluent, excipient, manufacturing aid (e.g., lubricant, talc magnesium, calcium or zinc stearate, or steric acid), or solvent encapsulating material, involved in carrying or transporting the subject compound from one organ, or portion of the body, to another organ, or portion of the body. Each carrier must be "acceptable" in the sense of being compatible with the other ingredients of the formulation and not injurious to the patient. Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, methylcellulose, ethyl cellulose, microcrystalline cellulose and cellulose acetate; (4) powdered tragacanth; (5) malt; (6) gelatin; (7) lubricating agents, such as magnesium stearate, sodium lauryl sulfate and talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol (PEG); (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) pH buffered solutions; (21) polyesters, polycarbonates and/or polyanhydrides; (22) bulking agents, such as polypeptides and amino acids (23) serum component, such as serum albumin, HDL and LDL; (22) C₂-C₁₂ alchols, such as ethanol; and (23) other non-toxic compatible substances employed in pharmaceutical formulations. Wetting agents, coloring agents, release agents, coating agents, sweetening agents, flavoring agents, perfuming agents, preservative and antioxidants can also be present in the formulation. The terms such as "excipient", "carrier", "pharmaceutically acceptable carrier" or the like are used interchangeably herein.

In some embodiments of this and other aspects of the invention, a therapeutically effective amount of therapeutic agent is administered to the subject. The phrase "therapeutically-effective amount" as used herein means that amount of a therapeutic agent which is effective for producing some desired therapeutic effect in at least a sub-population of cells in an animal at a reasonable benefit/risk ratio applicable to any medical treatment. For example, an amount of a therapeutic agent administered to a subject that is sufficient to produce a statistically significant, measurable modulation of stenosis.

Determination of a therapeutically effective amount is well within the capability of those skilled in the art. Generally, a therapeutically effective amount can vary with the subject's history, age, condition, sex, as well as the severity and type of the medical condition in the subject, and administration of other pharmaceutically active agents.

As used herein, the term "administer" refers to the placement of a composition into a subject by a method or route which results in at least partial localization of the composition at a desired site such that desired effect is produced. Routes of administration suitable for the methods of the invention include both local and systemic administration. Generally, local administration results in more of the therapeutic agent being delivered to a specific location as compared to the entire body of the subject, whereas, systemic administration results in delivery of the therapeutic agent to essentially the entire body of the subject.

Administration to a subject can be by any appropriate route known in the art including, but not limited to, oral or parenteral routes, including intravenous, intramuscular, subcutaneous, transdermal, airway (aerosol), pulmonary, nasal, rectal, and topical (including buccal and sublingual) administration.

Exemplary modes of administration include, but are not limited to, injection, infusion, instillation, inhalation, or ingestion. "Injection" includes, without limitation, intravenous, intramuscular, intraarterial, intrathecal, intraventricular, intracapsular, intraorbital, intracardiac, intradermal, intraperitoneal, transtracheal, subcutaneous, subcuticular, intraarticular, sub capsular, subarachnoid, intraspinal, intracerebro spinal, and intrasternal injection and infusion. In some embodiments of the aspects described herein, administration is by intravenous infusion or injection.

As used herein, a "subject" means a human or animal. Usually the animal is a vertebrate such as a primate, rodent, domestic animal or game animal. Primates include chimpanzees, cynomologous monkeys, spider monkeys, and macaques, e.g., Rhesus. Rodents include mice, rats, woodchucks, ferrets, rabbits and hamsters. Domestic and game animals include cows, horses, pigs, deer, bison, buffalo, feline species, e.g., domestic cat, canine species, e.g., dog, fox, wolf, avian species, e.g., chicken, emu, ostrich, and fish, e.g., trout, catfish and salmon. Patient or subject includes any subset of the foregoing, e.g., all of the above, but excluding one or more groups or species such as humans, primates or rodents. In certain embodiments of the aspects described herein, the subject is a mammal, e.g., a primate, e.g., a human. The terms, "patient" and "subject" are used interchangeably herein. The terms, "patient" and "subject" are used interchangeably herein. A subject can be male or female.

Preferably, the subject is a mammal. The mammal can be a human, non-human primate, mouse, rat, dog, cat, horse, or cow, but are not limited to these examples. Mammals other than humans can be advantageously used as subjects that represent animal models of disorders associated with autoimmune disease or inflammation. In addition, the methods and compositions described herein can be used to treat domesticated animals and/or pets.

A subject can be one who has been previously diagnosed with or identified as suffering from or having a disease or disorder characterized with stenosis or stenotic lesion, or a hemodynamic disorder or condition.

A subject can be one who is currently being treated for stenosis, stenotic lesion, a disease or disorder characterized with stenosis or stenotic lesion, or a hemodynamic disorder or condition.

A subject can be one who has been previously diagnosed with or identified as suffering from or having internal bleeding.

A subject can be one who is being treated for internal bleeding.

In some embodiments of the aspects described herein, the method further comprising diagnosing a subject for stenosis, stenotic lesion, internal bleeding, or a hemodynamic disorder or condition before onset of the treatment according to methods of the invention.

In some embodiments of the aspects described herein, the method further comprising selecting a subject with stenosis, stenotic lesion, internal bleeding, or a hemodynamic disorder or condition before onset of the treatment according to methods of the invention.

Toxicity and therapeutic efficacy can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compositions that exhibit large therapeutic indices, are preferred.

As used herein, the term ED denotes effective dose and is used in connection with animal models. The term EC denotes effective concentration and is used in connection with in vitro models.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized.

The therapeutically effective dose can be estimated initially from cell culture assays. A dose may be formulated in animal models to achieve a circulating plasma concentration range that includes the IC50 (i.e., the concentration of the therapeutic which achieves a half-maximal inhibition of symptoms) as determined in cell culture. Levels in plasma may be measured, for example, by high performance liquid chromatography. The effects of any particular dosage can be monitored by a suitable bioassay.

The dosage may be determined by a physician and adjusted, as necessary, to suit observed effects of the treatment. Generally, the compositions are administered so that therapeutic agent is given at a dose from 1 µg/kg to 150 mg/kg, 1 µg/kg to 100 mg/kg, 1 µg/kg to 50 mg/kg, 1 µg/kg to 20 mg/kg, 1 µg/kg to 10 mg/kg, 1µg/kg to 1mg/kg, 100 µg/kg to 100 mg/kg, 100 µg/kg to 50 mg/kg, 100 µg/kg to 20 mg/kg, 100 µg/kg to 10 mg/kg, 100µg/kg to 1mg/kg, 1 mg/kg to 100 mg/kg, 1 mg/kg to 50 mg/kg, 1 mg/kg to 20 mg/kg, 1 mg/kg to 10 mg/kg, 10 mg/kg to 100 mg/kg, 10 mg/kg to 50 mg/kg, or 10 mg/kg to 20 mg/kg. It is to be understood that ranges given here include all intermediate ranges, for example, the range 1 mg/kg to 10 mg/kg includes 1mg/kg to 2 mg/kg, 1mg/kg to 3 mg/kg, 1mg/kg to 4 mg/kg, 1mg/kg to 5 mg/kg, 1mg/kg to 6 mg/kg, 1mg/kg to 7 mg/kg, 1mg/kg to 8 mg/kg, 1mg/kg to 9 mg/kg, 2mg/kg to 10mg/kg, 3mg/kg to 10mg/kg, 4mg/kg to 10mg/kg, 5mg/kg to 10mg/kg, 6mg/kg to 10mg/kg, 7mg/kg to 10mg/kg,8mg/kg to 10mg/kg, 9mg/kg to 10mg/kg, and the like. It is to be further understood that the ranges intermediate to the given above are also within the scope of this invention, for example, in the range 1mg/kg to 10 mg/kg, dose ranges such as 2mg/kg to 8 mg/kg, 3mg/kg to 7 mg/kg, 4mg/kg to 6mg/kg , and the like.

In some embodiments, the compositions are administered at a dosage so that therapeutic agent or a metabolite thereof has an *in vivo* concentration of less than 500nM, less than 400nM, less than 300 nM, less than 250 nM, less than 200 nM, less than 150 nM, less than 100 nM, less than 50 nM, less than 25 nM, less than 20, nM, less than 10 nM, less than 5nM, less than 1 nM, less than 0.5 nM, less than 0.1nM, less than 0.05, less than 0.01, nM, less than 0.005 nM, less than 0.001 nM after 15 mins, 30 mins, 1 hr, 1.5 hrs, 2 hrs, 2.5 hrs, 3 hrs, 4 hrs, 5 hrs, 6 hrs, 7 hrs, 8 hrs, 9 hrs, 10 hrs, 11 hrs, 12 hrs or more of time of administration.

With respect to duration and frequency of treatment, it is typical for skilled clinicians to monitor subjects in order to determine when the treatment is providing therapeutic benefit, and to determine whether to increase or decrease dosage, increase or decrease administration frequency, discontinue treatment, resume treatment or make other alteration to treatment regimen. The dosing schedule can vary from once a week to daily depending on a number of clinical factors, such as the subject's sensitivity to the therapeutic agent. The desired dose can be administered everyday or every third, fourth, fifth, or sixth day. The desired dose can be administered at one time or divided into subdoses, e.g., 2-4 subdoses and administered over a period of time, e.g., at appropriate intervals through the day or other appropriate schedule. Such sub-doses can be administered as unit dosage forms. In some embodiments of the aspects described herein, administration is chronic, e.g., one or more doses daily over a period of weeks or months. Examples of dosing schedules are administration daily, twice daily, three times daily or four or more times daily over a period of 1 week, 2 weeks, 3 weeks, 4 weeks, 1 month, 2 months, 3 months, 4 months, 5 months, or 6 months or more.

### Non-medical uses (not part of the claimed invention)

The aggregates described herein are also useful in industrial applications. For example, the aggregates can be used for clearing a clogged pipe and/or to repair leaks in a pipe. Without limitation, a pipe can be of any diameter and any substance can be flowing through the pipe, e.g., chemicals, water, oil, gas, etc. As used herein, the term "pipe" is intended to include any type of apparatus through which a fluid can flow. Examples include chemical feed systems, municipal services, and supply pipelines, such as water, gas, and oil. As used herein the term "fluid" refers to a material that can flow. Accordingly, the term "fluid" includes liquid, gaseous, and semi-solid materials.

Without wishing to be bound by a theory, the shear stress in the clogged area is higher than in the unclogged area. Thus, the aggregate will disaggregate near or at the clogged area releasing agents that can clear the clog. Accordingly, in some embodiments of this disclosure, the aggregate includes an agent that can unclog a pipe. Agents for unclogging a pipe can include, but are not limited to, agents capable of producing an exothermic reaction, producing an oxidation reaction, producing an enzymatic reaction, and any combinations thereof.

Agents that can produce an exothermic reaction can include a combination of a base and metal. The base and the metal can be formulated in separate aggregates, and an exothermic reaction takes place upon release of the base and metal at the clog, which can clear the clog. In some embodiments, base is sodium hydroxide. In some embodiments, the metal is aluminum.

Agents that can produce an oxidation reaction can include peroxygens, such as sodium percarbonate, sodium persulfate, and sodium perborate; and halogen-containg oxidizing compounds, such as calcium hypochlorite, alkali earth metal hypochlorites, alkaline earth metal hypochlorites, sodium dichloro-striazinetrione, chlorinated isocyanurates, 1,3-dibromo and 1,3-dichloro-5-isobutylhydantoin. In some embodiments, the oxidation agent includes a combination of a peroxygen and an organic substance, e.g., a carbohydrate.

Agents that can produce an enzymatic reaction can include bacterium. The bacterium can be a lignin-degrading bacterium. In some embodiments of this and other aspects of the invention, the bacterium produces at least one of a lipase, an amylase, a cellulase, or a protease.

In addition to the anti-clogging agent, the aggregate can include a compound selected from the group consisting of surfactants, slip agents, foam suppressants, anti-caking agents, binding agents, abrasive agents, corrosion inhibitors, defoamers, and any combinations thereof.

As discussed above, flow thorough a leak can also lead to high shear stress. Dep [ending on the nature of the leak, shear stress can be high at or near the leak. Accordingly, in some embodiments of this and other aspects of the invention, the aggregate includes a sealing material. Exemplary sealing materials include, but are not limited to, alginates, particlulates, mineral oils, silicone rubber, thermoplastic or thermosetting resins (vinyl acetate resins, or atactic polypropylene), rubber latexes, non-silicone type rubber (natural rubber (MR), isoprene rubber (IR), butadiene rubber (BR), poly(1, 2-butadiene) (1,2-BR), styrene-butadiene rubber (SBR), chloroprene rubber (CR), nitrile rubber (NBR), butyl rubber (TfR), ethylene-propylene rubber (EPM, EPDM), chlorosulfonated polyethylene (CSM) and acryl rubber (ACM, ANM).

### Kits (not part of the claimed invention)

Also described herein is a kit comprising an aggregate, a formulation comprising an aggregate, components for making an aggregate or a formulation comprising an aggregate described herein.

In addition to the above mentioned components, the kit can include informational material. The informational material can be descriptive, instructional, marketing or other material that relates to the methods described herein and/or the use of the aggregates for the methods described herein. For example, the informational material describes methods for administering the aggregate to a subject. The kit can also include a delivery device.

In one embodiment, the informational material can include instructions to administer the formulation in a suitable manner, e.g., in a suitable dose, dosage form, or mode of administration (e.g., a dose, dosage form, or mode of administration described herein). In another embodiment, the informational material can include instructions for identifying a suitable subject, e.g., a human, e.g., an adult human. The informational material of the kits is not limited in its form. In many cases, the informational material, e.g., instructions, is provided in printed matter, e.g., a printed text, drawing, and/or photograph, e.g., a label or printed sheet. However, the informational material can also be provided in other formats, such as Braille, computer readable material, video recording, or audio recording. In another embodiment, the informational material of the kit is a link or contact information, e.g., a physical address, email address, hyperlink, website, or telephone number, where a user of the kit can obtain substantive information about the formulation and/or its use in the methods described herein. Of course, the informational material can also be provided in any combination of formats.

In some embodiments the individual components of the formulation can be provided in one container. Alternatively, it can be desirable to provide the components of the formulation separately in two or more containers, e.g., one container for an oligonucleotide preparation, and at least another for a carrier compound. The different components can be combined, e.g., according to instructions provided with the kit. The components can be combined according to a method described herein, *e.g.,* to prepare and administer a pharmaceutical composition.

In addition to the formulation, the composition of the kit can include other ingredients, such as a solvent or buffer, a stabilizer or a preservative, and/or a second agent for treating a condition or disorder described herein. Alternatively, the other ingredients can be included in the kit, but in different compositions or containers than the formulation. In such embodiments, the kit can include instructions for admixing the formulation and the other ingredients, or for using the oligonucleotide together with the other ingredients.

The formulation can be provided in any form, e.g., liquid, dried or lyophilized form. It is preferred that the formulation be substantially pure and/or sterile. When the formulation is provided in a liquid solution, the liquid solution preferably is an aqueous solution, with a sterile aqueous solution being preferred. When the formulation is provided as a dried form, reconstitution generally is by the addition of a suitable solvent. The solvent, e.g., sterile water or buffer, can optionally be provided in the kit.

In some embodiments, the kit contains separate containers, dividers or compartments for the formulation and informational material. For example, the formulation can be contained in a bottle, vial, or syringe, and the informational material can be contained in a plastic sleeve or packet. In other embodiments, the separate elements of the kit are contained within a single, undivided container. For example, the formulation is contained in a bottle, vial or syringe that has attached thereto the informational material in the form of a label.

In some embodiments, the kit includes a plurality, e.g., a pack, of individual containers, each containing one or more unit dosage forms of the formulation. For example, the kit includes a plurality of syringes, ampules, foil packets, or blister packs, each containing a single unit dose of the formulation. The containers of the kits can be air tight and/or waterproof.

### Definitions

Unless stated otherwise, or implicit from context, the following terms and phrases include the meanings provided below. Unless explicitly stated otherwise, or apparent from context, the terms and phrases below do not exclude the meaning that the term or phrase has acquired in the art to which it pertains. The definitions are provided to aid in describing particular embodiments of the aspects described herein, and are not intended to limit the claimed invention, because the scope of the invention is limited only by the claims. Further, unless otherwise required by context, singular terms shall include pluralities and plural terms shall include the singular.

As used herein the term "comprising" or "comprises" is used in reference to compositions, methods, and respective component(s) thereof, that are essential to the invention, yet open to the inclusion of unspecified elements, whether essential or not.

As used herein the term "consisting essentially of' refers to those elements required for a given embodiment. The term permits the presence of additional elements that do not materially affect the basic and novel or functional characteristic(s) of that embodiment of the invention.

The term "consisting of' refers to compositions, methods, and respective components thereof as described herein, which are exclusive of any element not recited in that description of the embodiment.

Other than in the operating examples, or where otherwise indicated, all numbers expressing quantities of ingredients or reaction conditions used herein should be understood as modified in all instances by the term "about." The term "about" when used in connection with percentages can mean ±1%.

The singular terms "a," "an," and "the" include plural referents unless context clearly indicates otherwise. Similarly, the word "or" is intended to include "and" unless the context clearly indicates otherwise.

Although methods and materials similar or equivalent to those described herein can be used in the practice or testing of this disclosure, suitable methods and materials are described below. The term "comprises" means "includes." The abbreviation, "e.g." is derived from the Latin exempli gratia, and is used herein to indicate a non-limiting example. Thus, the abbreviation "e.g." is synonymous with the term "for example."

The terms "decrease" , "reduced", "reduction" , "decrease" or "inhibit" are all used herein generally to mean a decrease by a statistically significant amount. However, for avoidance of doubt, "reduced", "reduction" or "decrease" or "inhibit" means a decrease by at least 10% as compared to a reference level, for example a decrease by at least about 20%, or at least about 30%, or at least about 40%, or at least about 50%, or at least about 60%, or at least about 70%, or at least about 80%, or at least about 90% or up to and including a 100% decrease (e.g. absent level as compared to a reference sample), or any decrease between 10-100% as compared to a reference level.

The terms "increased" "increase" or "enhance" or "activate" are all used herein to generally mean an increase by a statically significant amount; for the avoidance of any doubt, the terms "increased", "increase" or "enhance" or "activate" means an increase of at least 10% as compared to a reference level, for example an increase of at least about 20%, or at least about 30%, or at least about 40%, or at least about 50%, or at least about 60%, or at least about 70%, or at least about 80%, or at least about 90% or up to and including a 100% increase or any increase between 10-100% as compared to a reference level, or at least about a 2-fold, or at least about a 3-fold, or at least about a 4-fold, or at least about a 5-fold or at least about a 10-fold increase, or any increase between 2-fold and 10-fold or greater as compared to a reference level.

As used herein, the term "treating" and "treatment" refers to administering to a subject an effective amount of a composition so that the subject as a reduction in at least one symptom of the disease or an improvement in the disease, for example, beneficial or desired clinical results. For purposes of this invention, beneficial or desired clinical results include, but are not limited to, alleviation of one or more symptoms, diminishment of extent of disease, stabilized (*e.g*., not worsening) state of disease, delay or slowing of disease progression, amelioration or palliation of the disease state, and remission (whether partial or total), whether detectable or undetectable. In some embodiments, treating can refer to prolonging survival as compared to expected survival if not receiving treatment. Thus, one of skill in the art realizes that a treatment may improve the disease condition, but may not be a complete cure for the disease. As used herein, the term "treatment" includes prophylaxis. Alternatively, treatment is "effective" if the progression of a disease is reduced or halted. In some embodiments, the term "treatment" can also mean prolonging survival as compared to expected survival if not receiving treatment. Those in need of treatment include those already diagnosed with a disease or condition, as well as those likely to develop a disease or condition due to genetic susceptibility or other factors which contribute to the disease or condition, such as a non-limiting example, weight, diet and health of a subject are factors which may contribute to a subject likely to develop diabetes mellitus. Those in need of treatment also include subjects in need of medical or surgical attention, care, or management. The subject is usually ill or injured, or at an increased risk of becoming ill relative to an average member of the population and in need of such attention, care, or management.

The term "statistically significant" or "significantly" refers to statistical significance and generally means a two standard deviation (2SD) above or below a reference level. The term refers to statistical evidence that there is a difference. It is defined as the probability of making a decision to reject the null hypothesis when the null hypothesis is actually true. The decision is often made using the p-value.

The term "nanosphere" means a nanoparticle having an aspect ratio of at most 3:1. The term "aspect ratio" means the ratio of the longest axis of an object to the shortest axis of the object, where the axes are not necessarily perpendicular.

The term "longest dimension" of a nanoparticle means the longest direct path of the nanoparticle. The term "direct path" means the shortest path contained within the nanoparticle between two points on the surface of the nanoparticle. For example, a helical nanoparticle would have a longest dimension corresponding to the length of the helix if it were stretched out into a straight line.

The term "nanorod" means a nanoparticle having a longest dimension of at most 200 nm, and having an aspect ratio of from 3:1 to 20:1.

The term "nanoprism" means a nanoparticle having at least two non-parallel faces connected by a common edge.

The "length" of a nanoparticle means the longest dimension of the nanoparticle.

The "width" of a nanoparticle means the average of the widths of the nanoparticle; and the "diameter" of a nanoparticle means the average of the diameters of the nanoparticle.

The "average" dimension of a plurality of nanoparticles means the average of that dimension for the plurality. For example, the "average diameter" of a plurality of nanospheres means the average of the diameters of the nanospheres, where a diameter of a single nanosphere is the average of the diameters of that nanosphere.

As used herein, the term "pharmaceutically-acceptable salts" refers to the conventional nontoxic salts or quaternary ammonium salts of a compound, e.g., from nontoxic organic or inorganic acids. These salts can be prepared *in situ* in the administration vehicle or the dosage form manufacturing process, or by separately reacting a purified compound in its free base or acid form with a suitable organic or inorganic acid or base, and isolating the salt thus formed during subsequent purification. Conventional nontoxic salts include those derived from inorganic acids such as sulfuric, sulfamic, phosphoric, nitric, and the like; and the salts prepared from organic acids such as acetic, propionic, succinic, glycolic, stearic, lactic, malic, tartaric, citric, ascorbic, palmitic, maleic, hydroxymaleic, phenylacetic, glutamic, benzoic, salicyclic, sulfanilic, 2-acetoxybenzoic, fumaric, toluenesulfonic, methanesulfonic, ethane disulfonic, oxalic, isothionic, and the like. See, for example, Berge et al., "Pharmaceutical Salts", J. Pharm. Sci. 66:1-19 (1977).

In some embodiments of the aspects described herein, representative salts include the hydrobromide, hydrochloride, sulfate, bisulfate, phosphate, nitrate, acetate, succinate, valerate, oleate, palmitate, stearate, laurate, benzoate, lactate, phosphate, tosylate, citrate, maleate, fumarate, succinate, tartrate, napthylate, mesylate, glucoheptonate, lactobionate, and laurylsulphonate salts and the like.

As used herein, a "prodrug" refers to compounds that can be converted via some chemical or physiological process (e.g., enzymatic processes and metabolic hydrolysis) to an active compound. Thus, the term "prodrug" also refers to a precursor of a biologically active compound that is pharmaceutically acceptable. A prodrug may be inactive when administered to a subject, i.e. an ester, but is converted *in vivo* to an active compound, for example, by hydrolysis to the free carboxylic acid or free hydroxyl. The prodrug compound often offers advantages of solubility, tissue compatibility or delayed release in an organism. The term "prodrug" is also meant to include any covalently bonded carriers, which release the active compound *in vivo* when such prodrug is administered to a subject. Prodrugs of an active compound may be prepared by modifying functional groups present in the active compound in such a way that the modifications are cleaved, either in routine manipulation or in vivo, to the parent active compound. Prodrugs include compounds wherein a hydroxy, amino or mercapto group is bonded to any group that, when the prodrug of the active compound is administered to a subject, cleaves to form a free hydroxy, free amino or free mercapto group, respectively. Examples of prodrugs include, but are not limited to, acetate, formate and benzoate derivatives of an alcohol or acetamide, formamide and benzamide derivatives of an amine functional group in the active compound and the like. See Harper, "Drug Latentiation" in Jucker, ed. Progress in Drug Research 4:221-294 (1962); Morozowich et al, "Application of Physical Organic Principles to Prodrug Design" in E. B. Roche ed. Design of Biopharmaceutical Properties through Prodrugs and Analogs, APHA Acad. Pharm. Sci. 40 (1977); Bioreversible Carriers in Drug in Drug Design, Theory and Application, E. B. Roche, ed., APHA Acad. Pharm. Sci. (1987); Design of Prodrugs, H. Bundgaard, Elsevier (1985); Wang et al. "Prodrug approaches to the improved delivery of peptide drug" in Curr. Pharm. Design. 5(4):265-287 (1999); Pauletti et al. (1997) Improvement in peptide bioavailability: Peptidomimetics and Prodrug Strategies, Adv. Drug. Delivery Rev. 27:235-256; Mizen et al. (1998) "The Use of Esters as Prodrugs for Oral Delivery of (3-Lactam antibiotics," Pharm. Biotech. 11,:345-365; Gaignault et al. (1996) "Designing Prodrugs and Bioprecursors I. Carrier Prodrugs," Pract. Med. Chem. 671-696; Asgharnejad, "Improving Oral Drug Transport", in Transport Processes in Pharmaceutical Systems, G. L. Amidon, P. I. Lee and E. M. Topp, Eds., Marcell Dekker, p. 185-218 (2000); Balant et al., "Prodrugs for the improvement of drug absorption via different routes of administration", Eur. J. Drug Metab. Pharmacokinet., 15(2): 143-53 (1990); Balimane and Sinko, "Involvement of multiple transporters in the oral absorption of nucleoside analogues", Adv. Drug Delivery Rev., 39(1-3): 183-209 (1999); Browne, "Fosphenytoin (Cerebyx)", Clin. Neuropharmacol. 20(1): 1-12 (1997); Bundgaard, "Bioreversible derivatization of drugs-principle and applicability to improve the therapeutic effects of drugs", Arch. Pharm. Chemi 86(1): 1-39 (1979); Bundgaard H. "Improved drug delivery by the prodrug approach", Controlled Drug Delivery 17: 179-96 (1987); Bundgaard H. "Prodrugs as a means to improve the delivery of peptide drugs",Arfv. Drug Delivery Rev. 8(1): 1-38 (1992); Fleisher et al. "Improved oral drug delivery: solubility limitations overcome by the use of prodrugs", Drug Delivery Rev. 19(2): 115-130 (1996); Fleisher et al. "Design of prodrugs for improved gastrointestinal absorption by intestinal enzyme targeting", Methods Enzymol. 112 (Drug Enzyme Targeting, Pt. A): 360-81, (1985); Farquhar D, et al., "Biologically Reversible Phosphate-Protective Groups", Pharm. Sci., 72(3): 324-325 (1983); Freeman S, et al., "Bioreversible Protection for the Phospho Group: Chemical Stability and Bioactivation of Di(4-acetoxy-benzyl) Methylphosphonate with Carboxyesterase," Chem. Soc., Chem. Commun., 875-877 (1991); Friis and Bundgaard, "Prodrugs of phosphates and phosphonates: Novel lipophilic alphaacyloxyalkyl ester derivatives of phosphate- or phosphonate containing drugs masking the negative charges of these groups", Eur. J. Pharm. Sci. 4: 49-59 (1996); Gangwar et al., "Pro-drug, molecular structure and percutaneous delivery", Des. Biopharm. Prop. Prodrugs Analogs, [Symp.] Meeting Date 1976, 409-21. (1977); Nathwani and Wood, "Penicillins: a current review of their clinical pharmacology and therapeutic use", Drugs 45(6): 866-94 (1993); Sinhababu and Thakker, "Prodrugs of anticancer agents", Adv. Drug Delivery Rev. 19(2): 241-273 (1996); Stella et al., "Prodrugs. Do they have advantages in clinical practice?", Drugs 29(5): 455-73 (1985); Tan et al. "Development and optimization of anti-HIV nucleoside analogs and prodrugs: A review of their cellular pharmacology, structure-activity relationships and pharmacokinetics", Adv. Drug Delivery Rev. 39(1-3): 117-151 (1999); Taylor, "Improved passive oral drug delivery via prodrugs", Adv. Drug Delivery Rev., 19(2): 131-148 (1996); Valentino and Borchardt, "Prodrug strategies to enhance the intestinal absorption of peptides", Drug Discovery Today 2(4): 148-155 (1997); Wiebe and Knaus, "Concepts for the design of anti-HIV nucleoside prodrugs for treating cephalic HIV infection", Adv. Drug Delivery Rev.: 39(1-3):63-80 (1999); Waller et al., "Prodrugs", Br. J. Clin. Pharmac. 28: 497-507 (1989).

The term "analog" as used herein refers to a compound that results from substitution, replacement or deletion of various organic groups or hydrogen atoms from a parent compound. As such, some monoterpenoids can be considered to be analogs of monoterpenes, or in some cases, analogs of other monoterpenoids, including derivatives of monoterpenes. An analog is structurally similar to the parent compound, but can differ by even a single element of the same valence and group of the periodic table as the element it replaces.

The term "derivative" as used herein refers to a chemical substance related structurally to another, i.e., an "original" substance, which can be referred to as a "parent" compound.. A "derivative" can be made from the structurally-related parent compound in one or more steps. The phrase "closely related derivative" means a derivative whose molecular weight does not exceed the weight of the parent compound by more than 50%. The general physical and chemical properties of a closely related derivative are also similar to the parent compound.

The following examples illustrate some embodiments and aspects of the invention.

### EXAMPLES

### Example 1:

PEG-PLGA (50:50 MW 4:17 kDA) or PLGA (50:50 MW 17kDa) nanoparticles were prepared as follows. The COOH-PEG-PLGA or COOH-PLGA polymer was conjugated with the binding peptides (CREKA (SEQ ID NO: 1) or CRKRLDRNK (SEQ ID NO: 3)) using a maleimide-PEG-NH2 linker. First, the cysteine ends of the peptides were conjugated to the maleimide end of the PEG linker in PBS buffer, at pH 6.5. Second, carboxyl functionalized PEG-PLGA polymer was pre-activated with 1-ethyl-3-(3-dimethylaminopropyl) (EDC) and N-hydroxysuccinimide (NHS) and subsequently reacted with the corresponding PEG modified peptides in PBS/DMSO mixture at room temperature for about 4 hours. The final products were purified by dialysis and a lyophilized powder was obtained. The conjugation reaction was confirmed by ¹H NMR. The peptide conjugated polymer was then dissolved with coumarin a hydrophobic dye either in ethyl acetate or DMSO and the nano particles were prepared by w/o/w emulsion or simple solvent displacement method.

Nanoparticles aggregates were prepared based on enhancing spontaneous self aggregation by storing a 5mg/ml concentrated solution at 4°C for a period of more than three days. Aggregates suspensions were filtered through 5 micron filters to sort out any oversized aggregate. Additionally, centrifugation (x2000g, 5 minute) followed by washing was used to decrease the concentration of single unbound nanoparticles.

COOH-PEG-PLGA (50:50 MW 4:17 kDA) polymer was conjugated with the binding peptides (CHVLWSTRC) using the standard N- hydroxysuccinimide (NHS) ester chemistry. Briefly, carboxyl functionalized PEG-PLGA polymer was pre-activated with 1-ethyl-3-(3-dimethylaminopropyl) (EDC) and NHS and subsequently reacted with corresponding peptides in DMSO at room temperature for about 4 hours. The products were purified by dialysis and a lyophilized powder was obtained. This binding peptide targets islet endothelial cells. The peptide conjugated polymer was then dissolved with coumarin a hydrophobic dye in DMSO and the nanoparticles were prepared by simple solvent displacement method. The nanoparticle were prepared in 5 mg/ml solution and allowed to aggregate as described above.

Microchannels mimicking stenosis were prepared from PDMS using conventional soft lithography. The microchannel height was 300 micron and a 90% stenotic segment (30 micron in height) separated between the pre and post-stenotic areas. The PDMS channels were sealed with a glass micro-slide (170 micron in thickness) using plasma bonding. The channels were sterilized using oxygen plasma and coated with fibronectin (50ug/ml @30 min) to improve cell adhesion. Mouse Islet Endothelial cells were introduced to the microchannel and allowed to adhere under static conditions (2hr in the incubator). Tubing was connected to the channel, and the channel was placed in a tissue culture incubator and medium was infused using a syringe pump. Islet endothelial cells were cultured in the channel for 3- 4 day till a monolayer was formed.

A solution containing nanoparticle aggregate (100ug/ml) was infused through the channels at a flow rate, which corresponds to an estimated wall shear stress of 1 dyne/cm² in the main channel for 10 minutes. Following this step, in order to wash unattached particles, water was infused through the channels at the same flow rate for 5 minutes. Phase images of cells and fluorescence images of the nanoparticles at the pre and post-stenosis sites were acquired using a Zeiss microscope. The fluorescence image of the nanoparticles was imposed on top of the phase image to visualize localization of the nanoparticle (usually throughout the cell but excluding the nucleus area). More nanoparticle accumulated (adhered and endocytosed) in the post-stenosis site compare to the pre-stenosis site (data not shown). This demonstrates that nanoparticle aggregate breaks up in the stenosis site, disperse into single nanoparticles and adhere to the target site.

### Example 2: Shear activated platelet mimetics for drug targeting to obstructed blood vessels

### Materials and Methods

*Nanoparticle preparation:* Nanoparticles (NPs) were prepared from PLGA (50:50, 17kDa, acid terminated; Lakeshore Biomaterials, AL) using a simple solvent displacement method (C. E. Astete &C. M. Sabliov, Journal of Biomaterials Science, Polymer Edition 17, 247 (2006)). The fluorescent hydrophobic dye, coumarin, was included in the NPs to enable visualization and quantitation in this study. Briefly, 1 mg/ml of polymer was dissolved with coumarin in dimethyl sulfoxide (DMSO, Sigma, MO), dialyzed against water at room temperature, and the nanoparticles were allowed to form by solvent displacement. The size distribution and morphology of the formed NPs were characterized using Dynamic Light Scattering (DLS), Scanning Electron Microscopy (SEM) and Transmission Electron Microscopy (TEM).

*Fabrication of Platelet Mimetics* - The PLGA NPs were centrifuged and concentrated to a 10 mg/ml suspension in water and 1 mg/ml L-leucine (Spectrum Chemicals & Laboratory Products, CA) was added. NP aggregates (platelet mimetics) were prepared by a spray-drying technique using a Mobile Minor spray dryer (Niro, Inc.; Columbia, MD). The aqueous leucine-NP suspension was infused separately from the organic phase (ethanol) at a ratio of 1.5:1 and mixed in-line immediately prior to atomization (J. C. Sung et al., Pharmaceutical research 26, 1847 (2009)). The inlet temperature was 80°C and the liquid feed rate was 50 ml/min; gas flow rate was set at 25 g/min and nozzle pressure was 40 psi. Spray dried powders were collected in a container at the outlet of the cyclone. Platelet mimetic suspensions were formed by reconstituting the powders in water at desired concentrations. Aggregate suspensions were filtered through 5µm filters to sort out any oversized aggregates; centrifugation (2000g for 5 min) followed by washing also was used to remove any single unbound nanoparticles. DLS was used to determine the size of the NPs in dilute solutions using a zeta particle size analyzer (Malvern instruments, UK) operating with a HeNe laser, 173° back scattering detector. Samples were prepared at 1 mg/ml concentration in PBS buffer at pH 7.4. Data collection and analysis was performed with Malvern instrument software.

*Functionalization with tPA* - NP aggregates (1 mg/ml) were pre-activated with 1-ethyl-3-(3-dimethylaminopropyl) (EDC) and N-hydroxysuccinimide (NHS) at 1:5:10 (PLGA:EDC:NHS) molar ratio and subsequently reacted with linker NH2-PEG-biotin (Thermofisher Scientific, Rockford, IL) at a 1:10 molar ratio in PBS, pH 7.4 at room temperature for 2 hours. The aggregates were then centrifuged and washed twice and reacted with streptavidin (Thermofisher Scientific) for 15 minutes at room temperature. The aggregates were purified by repeated centrifugation and washing to remove unreacted reagents. Separately, human tissue plasminogen activator (tPA, Cell Sciences, MA) was functionalized with biotin using the NHS-PEG-biotin in PBS at room temperature for 2 hours at a 1:10 molar ratio (J. C. Murciano et al., Nature biotechnology 21, 891 (2003)). The functionalized tPA was then reacted with the strepatvidin-biotin-aggregates for 30 min at room temperature. The tPA functionalized NP aggregates functionalized with tPA were then purified by centrifugation and washing; the amount of tPA conjugated to the aggregates was determined by fluorescence spectrometry. Briefly, aggregates were dissolved in 1M NaOH under stirring at 37°C for ∼ 6h until a clear polymer solution was obtained. The amount of tPA (TRITC-labeled, Cell Sciences, MA) in the polymer solution was then measured at 594 nm. Activity of t-PA coated particles was confirmed using a flourimetric tPA activity assay (SensoLyte,AnaSpec,CA). SEM of the aggregated nanoparticles was performed using a Zeiss FESEM Supra55vP (Center for Nanosystems (CNS), Harvard University). Samples were mounted on carbon tape adhesive substrates and coated with gold under vacuum using a sputter coater. The coated NP aggregates were imaged at 4 kV using an in-lens detector at 9 mm working distance.

*Rheometer Shearing Assay -* A solution of platelet mimetics (5 mg/ml in 8% Polyvinylpyrrolidone solution) was sheared for 1 min using a 20 mm plate in a Rheometer (AR-G2 TA Instruments, DE). The solutions were then collected, filtered through a 0.45 micron filter (Millipore, MA) to remove NPs from large microscale aggregates and diluted 1:3 with water. The fluorescence intensity of these NP suspensions was measured using a spectrometer (Photon Technology International, NJ) and normalized relative to the highest shear level (1,000 dyne/cm²) value.

*Computational Fluid Dynamics (CFD) simulations -* CFD simulations for the microfluidic channels were performed using the software package Comsol 3.5 (Comsol, USA), based on a finite element method. We considered the flow to be steady and incompressible, and assumed a no-slip boundary condition at the walls and the fluid medium (PBS) to have a constant density of 1000 kg/m³ and viscosity of 1 mPa-sec. CFD simulations of IVUS reconstructed blood vessel were preformed as previously described (A. U. Coskun et al., Catheterization and cardiovascular interventions 60, 67 (2003)).

*Microfluidic Models of Vascular Stenosis-* Microchannels mimicking vascular constriction used for studies on microemboli formation were prepared from polydimethylsiloxane (PDMS) using conventional soft lithography (Y. Xia, G. M. Whitesides, Annual review of materials science 28, 153 (1998)). A master mold was prepared by aligning 80 micron layers designed using a CAD program and formed using a cutter plotter (CE5000, Graphtec, CA). The device contained a region (160 µm high x 400 µm wide x 10 mm long) with a 90% constriction relative to upstream and downstream channel regions (each: 640 µm high x 2 mm wide x 20 mm long). The PDMS channels were sealed with a glass microslide (170 µm thick) using plasma bonding. In some studies, solutions of platelet mimetics (5 ml, 100 µg/ml) were recirculated through microfluidic devices with 90% occlusion or without any constriction using a peristaltic pump (ISM 834C, Ismatec SA, Switzerland). Flow rate was adjusted to obtain a wall shear stress of 10 dyne/cm² at the unconstructed channels. The suspensions were collected after 20 minutes of flow and filtered through a sub-micron (0.45µm) filter. The fluorescent intensity of the collected NP suspensions was measured using a spectrometer (Photon Technology International, NJ) and normalized relative to the unconstricted channel value. For studies on release of NPs and their binding to endothelial cells in stenotic regions, the microfluidic devices were sterilized using oxygen plasma and coated with fibronectin (50µg/ml @ 30 min) to support cell adhesion. Bovine aortic endothelial cells (Lonza,MD) were introduced to the microchannel and allowed to adhere under static conditions (2 hr at 37°C). The devices were then placed in a tissue culture incubator and medium (EGM®-MV BulletKit, Lonza, MD) was infused (50 µL/hr) using a syringe pump (Braintree Scientific, Braintree, MA). The endothelial cells were cultured in the devices for 3- 4 day until a continuous cell monolayer was formed. A solution containing platelet mimetics (10 µg/ml) was then infused for 10 min through the device at a flow rate which produces a wall shear stress of ∼10 dyne/cm2 in the unconstructed channel. Unattached particles were flushed away by infusing water through the channels at the same flow rate for 5 min. Phase contrast and fluorescence microscopic images of cells and bound NPs in regions proximal upstream and downstream to the constriction were acquired using a Zeiss microscope. The averaged fluorescence intensity of cell-associated coumarin-loaded NPs obtained from these views was used to evaluate the difference in NP accumulation between pre- and post stenotic regions.

*Microfluidic Models of Vascular Embolism -* Microfluidic devices with a narrowed cross-sectional area (80µm high x 0.5 mm wide s 200 mm long) were fabricated using soft lithography as described above. Fibrin clots formed as described below that were infused into the main channel lodged and obstructed the flow in these smaller channels. A solution of t-PA or t-PA coated microaggregates was infused at a flow rate corresponding to a shear stress of 10 dyne/cm² in an unobstructed channel. Prior to infusion of the t-PA solutions, bovine plasminogen (Cell Sciences, MA) was added to a final concentration of 2.2 µM (S. L. Diamond, Annual review of biomedical engineering 1, 427 (1999)). During the fibrinolysis process, the fibrin clots size were monitored in real-time (images acquired every 30 sec) on an inverted Zeiss microscope.

*Experimental Fibrin Emboli-* Fibrin clots were formed by adding CaCl₂ (20 mM) and human α-thrombin (1 units/ml final concentrations, Enzyme Research Laboratories, IN) to human fibrinogen (5 mg/ml, Enzyme Research Laboratories, IN), as previously described (J. C. Murciano et al., Nature biotechnology 21, 891 (2003) and C. K. Lam, T. Yoo, B. Hiner, Z. Liu, J. Grutzendler, Nature 465, 478 (2010)). This solution was immediately added drop-wise to a solution of canola oil with Span-80 (0.05%). The emulsion was mixed at 350 rpm for 4 hr and centrifuged (500g, 5min), followed by repeated washing in ethanol and water. The diameter of the resulting fibrin beads was determined by optical microscopy to be ∼ 200 µm.

*Mouse Pulmonary Embolism Model* - Male 6-8 week-old C57BL/6 mice (Jackson Laboratory, Bar Harbor, ME) were weighed and anesthetized with Avertin (200 mg/kg IP). The trachea was incised via surgical tracheotomy, and cannulated with a blunted 22G stainless steel needle tip. The lungs were subsequently ventilated at a rate of 60 breaths/min, with a Peak Inspiratory Pressure (Pip) of 10 cm H₂O and a Positive End Expiratory Pressure (Peep) of 3 cm H₂O with compressed air using a mouse ventilator (VCM-R, Hugo Sachs Elektroniks, Germany). *Ex vivo* ventilation and perfusion of the mouse lung was performed using a IL1 *ex vivo* mouse lung ventilation-perfusion system (Harvard Apparatus, Natick, MA),(D. Huh et al., Science 328, 1662 (2010)). Following initiation of mechanical ventilation, the chest was opened via thoracotomy, and heparin (100 IU) was injected into the right ventricle. After 30 seconds, the thoracic aorta and superior vena cava were cut and the animal ex-sanguinated. A suture was placed around the pulmonary artery and aorta. Cannulae made from polyethylene tubing (PE90 (0.86 mm ID, 1.7 mm OD)) were placed in the pulmonary artery (PA) and left atrium (LA), and lungs were perfused with RPMI-1640 with 4% Bovine Albumin (ProbuminTM Reagent Grade, Billerica, MA) and 0.7 g NaCl/500 mL via a roller pump (ISM 834C, Ismatec SA, Switzerland) set at a constant flow rate of 0.5 ml/min in a recirculating system with a system volume of 6 mL. Perfusate and lung temperatures were maintained at 37°C by housing the entire *ex-vivo* ventilation perfusion system inside a standard cell incubator without CO₂ (Forma Scientific, Ohio). Humidity was maintained in the range of 90-95%. Pulmonary arterial and venous pressures and airway flow and pressures were recorded with dedicated Type 379 vascular pressure and DLP2.5 flow and MPX Type 399/2 airway pressure transducers and TAM-A amplifiers (Hugo Sachs Elektroniks, Germany). Vascular pressures were zeroed at the mid lung level prior to each experiment and recorded using Polyview16© software (Grass Technologies, West Warwick, RI) running on a desktop PC running Windows XP SP2 (Microsoft Corporation, Redmond, WA).

Prior to injection of experimental fibrin clots (prepared as above), the measured parameters were allowed to stabilize and remain stable for a period of more than 10 minutes. A solution of fibrin clots suspended in perfusion medium was infused at a flow rate of 0.1 ml/min and mixed with the regular perfusion line entering the pulmonary artery. The fibrin clot suspension was infused until the pulmonary artery pressure increased to ∼ three-fold higher than the baseline. The system was then allowed to equilibrate and remain stable for at least 10 minutes. Next, tPA-coated platelet mimetics or free soluble tPA was added to the main perfusion line and circulated through the perfusion system. As in the microfluidic device experiments, when tPA was perfused, plasminogen was added to obtain a final concentration of 2.2 µM. Pulmonary artery and vein pressures were acquired continuously during the perfusion period. At the end of each experiment, lungs were perfused with 4% paraformaldehyde, and prepared for sectioning by incubating in 4% paraformaldehyde, then sucrose and OCT. All experimental animal protocols were approved by the Institutional Animal Care and Use Committee at Children's Hospital Boston and Harvard Medical School.

*Adhesion of NPs and Microaggregates under Flow* - Microfluidic devices contain a narrowed channel (80 µm high x 2 mm wide x 200 mm long) were fabricated using soft lithography as described above. A glass slide coated with a thin dry layer of fibrin (< 1 µm thick) was bonded to the bottom of the channel. Fluorescent NPs (200nm) and microparticles (2µm) were coated with tPA as detailed above. A solution of the coated NPs or microaggregates (100mg/ml) were infused in the channel at a flow rate corresponding to a wall shear stress of 10 dyne/cm² for 15 min. At the end of the experiment the channel were washed with water at the same flow rate for >10 minute. Fluorescent microscopy images were taken and analyzed to evaluate the area covered by particles.

### Results and discussion

Disruption of normal blood flow to the heart, lung and brain is the leading cause of death and long-term adult disability in the western world (C. J. L. Murray, A. D. Lopez, The Lancet 349, 1269 (1997)). Current approaches to acute therapy for coronary infarction, pulmonary embolism and stroke require continuous delivery of thrombolytic drugs for several hours, which need to be administered systemically or through a catheter placed within the obstructed vessel, usually in a hospital setting ((T. J. Ingall et al., Stroke 35, 2418 (2004) and T. G. Kwiatkowski et al., New England Journal of Medicine 340, 1781 (1999)). To be effective, patients must receive therapy within a few hours after onset of symptoms, and doses of clot-lysing drugs that can be administered are limited by the potential risk of bleeding since the drug is free to distribute throughout the body. To overcome these limitations and to develop a therapeutic that potentially can be administered intravenously on site immediately after symptoms of acute vascular occlusion are observed, we set out to design a thrombolytic delivery system that releases drugs selectively at sites of flow obstruction.

Obstructed and stenotic blood vessels exhibit unique physical characteristics that distinguish them from neighboring normal vasculature in that fluid shear stress can increase locally by one to two orders of magnitude, from below approximately 70 dyne/cm² in normal vessels to greater than 1,000 dyne/cm² in highly constricted arteries (J. Strony et al., American Journal of Physiology-Heart and Circulatory Physiology 265, H1787 (1993); D. M. Wootton & D. N. Ku, Annual review of biomedical engineering 1, 299 (1999); and J. M. Siegel, C. P. Markou, D. N. Ku, S. Hanson, Journal of biomechanical engineering 116, 446 (1994)). Circulating platelets are locally activated by high shear stress in these regions and rapidly adhere to the adjacent surface lining of the narrowed vessels (Z. M. Ruggeri et al., Blood 108, 1903 (2006); W. S. Nesbitt et al., Nature medicine 15, 665 (2009); and S. Goto et al., Circulation 99, 608 (1999)). In contrast to drug targeting mechanisms that focus on expression of distinct molecular species that can vary between tissues or patients, shear stress increases as a function of narrowing of the lumen diameter in all patients, regardless of the cause or location of obstruction. Inspired by this natural physical mechanism of platelet targeting, we developed a strategy that uses local high shear stress as a generic mechanism to target drug delivery to regions of blood vessels that are constricted by clots, stenosis or developmental abnormalities.

The platelet mimetics of the invention are similar in size to natural platelets (1 to 5 µm in diameter); however, they are fabricated as aggregates of multiple smaller nanoparticles (NPs). The microscale aggregates remain intact when flowing in blood under physiological flow conditions, but breakup into individual nanoscale components when exposed to high local shear stress and rapidly adhere to the vessel surface. High concentrations of desired chemical or enzymatic activities can be delivered directly to sites of vascular occlusion by immobilizing relevant drugs or enzymes on the NPs that specifically deploy in these regions based on shear stress-dependent disruption of NP-NP adhesions.

The platelet mimetics were produced by spray-drying concentrated solutions of biocompatible, biodegradable poly lactic-co-glycolic acid (PLGA 50:50, MW 17kDa) to form micrometer-sized (3.8 ± 1.6 µm) aggregates of small (180 ± 70 nm) NPs. See Methods and Materials, and J. C. Sung, Pharma. Res., 26:1847 (2009) and N. Tsapis et al., Proc. Natl. Acad. Sci. USA, 99:12001 (2002), Fig. 2A), which are stable in aqueous solutions due to their hydrophobicity (R. C. Sonntag & W. B. Russel, Journal of colloid and interface science 113, 399 (1986); H. A. Stone, Annual Review of Fluid Mechanics 26, 65 (1994); and R. Gref et al., Science 263, 1600 (1994)). When exposed to mechanical forces that overcome the attractive forces holding the NPs together, such as hemodynamic shear stresses, the aggregates break apart (Fig. 2B) much like a wet ball of sand disperses into individual grains when rubbed in one's hands (R. C. Sonntag & W. B. Russel, Journal of colloid and interface science 113, 399 (1986) and H. A. Stone, Annual Review of Fluid Mechanics 26, 65 (1994)).

Stability and mechanical integrity of the aggregates are controlled by particle-particle molecular adhesion forces. PLGA based NPs are hydrophobic in nature and self aggregate in aqueous solutions (C. E. Astete and C. M. Sabliov, J. Biomater. Sci., Polymer Ed., 17:247 (2006)). In conventional applications of PLGA based NPs, sonication is used to destabilize and break these undesired aggregates (K. Avgoustakis, Current Drug Delivery, 1:321(2004)). The inventors leveraged this observation to design nano-carrier aggregates that break up when agitated by fluidic shear stress. When hydrodynamic forces overcome the attractive forces between the particles - the particles disperse.

To examine the shear-induced breakup of NP-aggregates, the inventors sheared suspensions of aggregates using a Rheometer at different levels of shear. Figure 2C shows the concentration of NPs in sheared solutions filtered through a sub-micron (0.45µm) filter. As the shear stress exceeded 10 dyne/cm², the concentration of NPs grew significantly. A 8- to 12-fold stress dependent increase in the concentration of released NPS was seen when the level of shear stress reached 100 dyne/cm² (Fig. 2C); a range that is relevant in many vascular diseases. These levels of shear stress correlate with shear stress at pathological stenotic sites as shown in computational fluid dynamic (CFD) modeling of stenosed vessel geometries. Figure 2E, shows the wall shear stress for a normal and stenosed right coronary artery (60% lumen obstruction), as obtained by CFD simulations of human patients intravascular ultrasound reconstructed geometries. For example, computational fluid dynamic (CFD) modeling of flow within normal and stenotic human left coronary arteries based on ultrasound imaging (see Methods and B. E. Figueroa et al., Stroke 29, 1202 (1998) and C. D. Murray, The Journal of General Physiology 9, 835 (1926)) revealed that the level of shear that induced NP release is similar to that generated by a 60% lumen obstruction (Fig. 2D,E) whereas normal coronary vessels experience a 5 fold lower level of shear stress (∼ 10 to 30 dyne/cm²).

To determine whether these platelet mimetics can release NPs under relevant hemodynamic flow conditions, the approach was tested under hemodynamic conditions in a three-dimensional (3D) microfluidic model of vascular narrowing fabricated from polydimethylsiloxane (PDMS) that were designed to mimic regions of living blood vessels with 90% lumen obstruction (Fig. 3A,B). Based on CFD modeling, such a constriction generates ∼100 fold increase in shear at the stenotic site (Fig. 3C). Perfusion of solutions of platelet mimetics (100 µg/ml in PBS) through these microfluidic devices resulted in a 16-fold increase in the release of free NPs, as measured in the solution flowing downstream of the obstruction compared to fluid flowing through unobstructed microfluidic channels of similar dimensions (Fig. 3D). Fluorescence microscopic imaging confirmed that released NPs accumulated in endothelial cells cultured on the inner surface of the artificial micro fluidic vessel just distal to the narrowed region whereas minimal uptake occurred in cells lining the channel prior to the constriction (Fig. 3E).

The inventors further tested a therapeutic strategy for thrombolysis utilizing the shear targeting strategy. Currently used thrombolytic therapy is based on local administration through a catheter that must be positioned precisely at the clot site in a hospital setting, or on systemic delivery of fibrinolytic agents, such as tissue plasmin activator (tPA) for several hours, which dissolve the clots. Unfortunately, a major concern and limitation with both of these treatments is serious side effects of bleeding (e.g., intracranial hemorrhage) and neuronal toxicity due to the circulating drug (T. G. Kwiatkowski et al., New England Journal of Medicine, 340:1781 (1999)). Administering thrombolytic drugs in a form that would have low levels of systemic activity, but still localize the treatment to the desired site can be highly valuable for such therapies. The invention provides a method to utilize abnormal elevated shear stress near clots that partially occlude vessels or during recanalization to locally enhance thrombolysis at these sites (Fig. 4). This approach can both significantly reduce the level of circulating drug as well as diminish extravascular leakage of the drug, e.g., diffusion to brain tissue.

In order to evaluate their functional potential, the inventors coated platelet mimetics containing fluorescent NPs with the FDA-approved thrombolytic drug, tissue plasminogen activator (tPA), and tested their ability to dissolve blood clots *in vitro* and *in vivo* (Fig. 5). Based on the shear targeting strategy (Fig. 5A), the platelet mimetics selectively disaggregate at regions where blood flow is abnormally constricted (Fig. 5B). Consequently, the disaggregated NPs containing thrombolytic drug accumulate at embolic sites and act locally to dissolve the obstruction. When artificial fibrin clots of defined size (250 ± 150 µm diameter) produced by a water-in-oil emulsion technique (R. Gref et al., Science 263, 1600 (1994) were injected into microfluidic channels with regions narrowed to a 80 µm high by 500 µm wide constriction, these fibrin emboli lodged in the devices and partially obstructed flow in the channels (Fig. 5C). When platelet mimetics (100 µg/ml) carrying tPA (50 ng/mL) were infused at physiological flow rates into these same devices, the microaggregates broke apart in the regions of the occlusion where shear was elevated causing releasing of the t-PA-coated NPs. Importantly, the fluorescent tPA-coated NPs accumulated at the surface of the artificial emboli, progressively dissolving the clots and reducing their size by half within less than an hour of treatment (Fig. 5C). In contrast, treatment with soluble t-PA enzyme at the same concentration and flow conditions had negligible effects (<5% reduction) on clot size (Fig. 5D).

Based on the above results, the inventors tested the therapeutic strategy to reverse the effects of acute pulmonary vascular occlusion caused by embolism in an *ex vivo* whole mouse lung ventilation-perfusion model. A solution containing pre-formed fibrin clots (1 x 10⁵/mL) similar in size to those tested in the microfluidic channel (Fig. 5C) were infused through the pulmonary artery of the perfused lung (0.1 mL/min for ~5 min). Occlusion of pulmonary microvessels by multiple microemboli (Fig. 5E) caused the pulmonary artery pressure to increase by about 3-fold compared to its normal value [30 versus 8 mm Hg] (Fig. 5H). We then perfused tPA-coated platelet mimetics (100 µg/ml platelet mimetics containing NPs coated with 50 ng/mL tPA) through the pulmonary artery at a physiological flow rate (0.5 mL/min). Fluorescence microscopic analysis of tissue sections confirmed that tPA-NPs were released selectively in regions of vascular occlusion, producing greater than a 25-fold increase in accumulation of NPs at these sites, and the fluorescent NPs bound at high density to the surfaces of the obstructing pulmonary emboli (Fig. 5E,F). Progressive lysis of the emboli by the tPA-NPs resulted in normalization of pulmonary artery pressure levels after a period of about 1 hour in this whole lung model (Fig. 5G,H). Importantly, perfusion of the same concentration (50ng/ml) of soluble tPA as that delivered on the injected tPA-coated NPs failed to produce any significant clinical response in this model (Fig. 5G). In fact, similar clot-lysing effects were only observed when we administered a hundred times higher concentration (5000 ng/ml) of soluble tPA under identical flow conditions (Fig. 5G). Hence, clot lysis and reversal of the physiological manifestations of pulmonary emboli were obtained with only a small fraction of the soluble tPA dose when administered using the platelet mimetics. Further, systemic perfusion of the same concentration of t-PA in the medium did not reduce the pressure - thus demonstrating the enhanced effectiveness of the therapeutic strategy described herein.

In humans, vascular obstruction due to pulmonary embolism is associated with high levels of morbidity and mortality (V. F. Tapson, New England Journal of Medicine 358, 1037 (2008)). Thrombolytic therapy is currently based on bolus injection and/or continuous delivery of fibrinolytic agents, such as soluble tPA, for several hours through intravascular catheters in a hospital setting, and major side effects include bleeding, intracranial hemorrhage and neuronal toxicity (T. G. Kwiatkowski et al., New England Journal of Medicine 340, 1781 (1999); S. G. Soriano & S. A. Lipton, Nature medicine 4, (1998); and B. E. Figueroa et al., Stroke 29, 1202 (1998)). This new shear-activated tPA targeting approach can significantly reduce the level of circulating drug due to targeting to regions where vessels are abnormally constricted due to occlusive emboli. The platelet mimetics also should greatly decrease extravascular leakage of the drug (e.g., diffusion to brain tissue) since the active agent is bound to NPs that are components of much larger (micrometer-sized) aggregates, which will not easily diffuse across the endothelial permeability barrier. Thus, this physics-based drug deployment strategy might improve the safety and effectiveness of therapies using tPA or other therapeutic agents.

The model used in this example, pulmonary embolism - a disease associated with high levels of morbidity and mortality (V. F. Tapson, New England Journal of Medicine, 358:1037 (2008)), represents just one of a potentially large number of therapeutic applications for the therapeutic strategy described herein. For example, stroke, acute coronary syndrome, myocardial infarction, atherosclerosis, peripheral vascular disease, vascular intimal hyperplasia, vasospasm, and developmental abnormalities, such as Moya Moya, all share vascular narrowing at the disease site as a common feature. Thus, the shear-activated drug delivery system can be useful for all of these diseases because the design of the living vasculature tends to restrain wall shear stress to a narrow and nearly constant range to minimize work under physiological conditions (C. D. Murray, The Journal of General Physiology 9, 835 (1926) and M. Zamir, The Journal of General Physiology 69, 449 (1977)). Tightly regulating this physical parameter is important for modulating endothelial cell phenotype, gene expression, aggregation of platelet and erythrocytes, arteriogenesis and vascular wall remodeling (N. Resnick et al., Endothelium 10, 197 (2003); J. J. Hathcock, Arteriosclerosis, thrombosis, and vascular biology 26, 1729 (2006); O. K. Baskurt, Biorheology 45, 629 (2008); and J. N. Topper & M. A. Gimbrone Jr, Molecular medicine today 5, 40 (1999)). Shear stress is tightly coupled to vessel diameter (Fig.1A), and thus, stenotic or obstructed regions create abnormal levels of shear stress that are orders of magnitude higher than any normal shear stress. In normal blood vessels, shear stress is well below 70 dyne/cm² (J. A. Champion, A. Walker, S. Mitragotri, Pharmaceutical research 25, 1815 (2008)), which does not activate our platelet mimetic. In contrast, shear stress significantly increases above this value at stenotic sites, sometimes reaching values as high as 3,000 dyne/cm² (J. Strony et al., American Journal of Physiology- Heart and Circulatory Physiology 265, H1787 (1993)). These elevated levels of shear disrupt NP-NP interactions within the microscale NP aggregates, and thereby 'activate' the platelet mimetic to release its NP components (Fig. 1A).

The present invention is directed to novel compositions and their use in methods for treatment and drug delivery based on elevated hemodynamic shear stress. This discovery opens up new opportunities in treatment of atherosclerosis, the leading cause of death in western world, in thrombolytic therapies and in treatment of other vascular pathologies such as: vaso-spasm, Moya-Moya in which stenotic region exists. The compositions and methods described herein are also valuable for minimizing shear induced clotting events in extracorporeal devices (such as hemodialysis) and can be used in targeting internal hemorrhage in cases where blood infiltrates through small tears in large damaged vessels.

As a driving force, shear can cause morphological and structural changes in single and collective elements and varying length scales. The interaction between shear stress and different forms of potential drug carriers including: nano/microspheres, microcapsules and microgels have been extensively studied. As shear increases single particles deform and eventually break. Shear triggered breakup of microcapsule/nanocapsule is being successfully employed in cosmetic products for active ingredient release upon rubbing against the skin. However, these or alternative approaches have not been suggested or developed for medical applications.

In one aspect, the present invention is directed to micron size aggregates which break into nanoparticles when flowing through a stenotic region while staying intact under normal physiological flow (Fig. 1A); thus allowing a localized delivery mechanism driven by the physiological abnormality - the locally elevated hemodynamic shear stress in the stenotic or partially occluded region. The targeting strategy is based on three steps. In the first step, elevated shear stress induces breakup of the aggregate into nanoparticles (Fig. 1A). Shear stress (τ) is a strong function of the vessel diameter (d), under Hagen-Poiseuille flow conditions *τ* ≈ *d³*; thus stenosis sites create abnormal levels of shear stress which are order of magnitude higher than any normal stress (J. Strony, et al., American Journal of Physiology- Heart and Circulatory Physiology 265, H1787 (1993)). These elevated levels of shear stress break apart NP aggregates thus activating the "platelet mimetics." In the second step, once the aggregates disperse, the dispersed nanoparticles exhibit enhanced adhesion and uptake at the target site. Under flow, due to the low hydrodynamic force (F_{hydro}) applied on them (*F_{hydro}* ≈ *r²*), NPs adhere to the target area while micron sized particles do not (Fig. 1B). Also see O. C. Farokhzad et al., Anal. Chem., 77:5453 (2005). Moreover, NPs easily undergo endocytosis thus further increasing their effective homing to cellular components. In the third step, drug is locally delivered in a controlled manner governed by the nanosized particles accumulating at the target site and NPs release kinetics (Fig. 1C). Breakup of the micron sized aggregates to NPs increases the surface area (S, *S* ≈ *r²*) and consequently enables enhanced drug release. Moreover, NPs release kinetics can be tuned: fast or active release for acute intervention or passive sustained release for prolonged treatments. Altogether, this enables localized delivery of drugs or imaging agents at defined prescriptions to stenosis sites.

The platelet mimetics also offer a number of other advantages. For example, due to their smaller size, the NPs experience lower hydrodynamic forces (F_{hydro}) (Fig. 1B) and hence, they adhere rapidly to the neighboring endothelial surface or vessel wall, whereas larger micrometer-sized particles tend to continue to move with the flow (Fig. 1B) (O. C. Farokhzad et al., Anal. Chem 77, 5453 (2005)). The smaller NPs are also more readily engulfed than larger particles, and so they can be harnessed to selectively deliver drugs intracellularly into the surrounding endothelium (R. Wiewrodt et al., Blood 99, 912 (2002)). Although the surface of the microscale aggregates was coated with drug in the present study, the spatial selectivity and activity of the drug deployed locally in high shear also might be further enhanced by coating individual NPs with drug before forming the larger aggregates, which would provide a large net increase in the total area available for drug loading (Fig. 1C).

Micron sized nanoparticle aggregates of PLGA or PEG-PLGA nano-particles can be produced by spontaneous self-aggregation of concentrated PLGA nanoparticle solutions followed by filtration to oversized aggregates (>5 micron). PLGA based nano-particles are hydrophobic in nature and self aggregate in aqueous solutions. Stability and mechanical integrity of the aggregates are controlled by particle-particle molecular adhesion forces. In conventional applications of PLGA based nanoparticles, sonication is used to destabilize and break these undesired aggregates. Similarly, agitation by fluidic shear stress can also drive disaggregation of nano-carrier aggregates.

Without wishing to be bound by a theory, the abnormal shear stress in stenotic sites can break up micron sized aggregates and disperse nanoparticles. The inventors infused a solution of nano-aggregates through microfluidic devices mimicking vascular stenosis conditions. Figure 7 shows the fluorescence intensity of solutions filtered through a 0.22 micron filter; thus correlating with the concentration of nano-particles in the solution. Figure 7 shows that flow through a stenosis mimicking site significantly increases the concentration of nanoparticles compared to the control and to flow under normal physiological conditions.

The second step, once the aggregates disperse, utilizes the enhanced ability of nanoparticles to adhere and be up taken at the target site. Under flow, due to the low hydrodynamic force applied on them, nanoparticles adhere to the targeted area while micron sized particles do not (Fig. 1B). Additionally, nanoparticles easily perform endocytosis thus further increasing their effective targeting. Moreover, this approach is based on PLGA nanoparticles, which can be coated with targeting peptide to further selectively facilitate adhesion to a defined site. Thus, the inventors have demonstrated multi-functional targeting particles combining the shear mechanism with other targeting approaches.

In the third step, once the nanoparticles settled, drug is delivered locally by the nanoparticle release kinetics. Breakup of the micron sized aggregates to nanoparticles increases the surface area and consequently enables enhanced drug release at the required site. The drug then can be taken up by cells present at the site, and/or the drug can coat the surface of the site without being taken up by the cells.

Alternatively, or in addition, the nanoparticles themselves can have therapeutic or biological activity. For example, the nanoparticles can be thrombogenic (e.g. nanoparticles coated with thrombogenic agents, such as collagen-coated particles, or nanoparticles coated with or encapsulating pro-coagulant enzymes/proteins). On break up, aggregates would release the pro-coagulants only at bleed sites (i.e. where shear is high due to high volume going through a small hole in vessel wall). But otherwise they would be stealth and cleared out in bile or in urine if biodegradable.

Thus, the inventors have discovered that shear induced disaggregation of nanoparticle aggregates and the biophysical properties of nanoparticles can be used to create a novel strategy for multi-functional targeting of stenotic blood vessels. This novel strategy provides an entirely an entirely new therapeutic approach for non-invasive treatment of atherosclerosis, stroke, pulmonary embolism and other hemodynamic related disorders that involve narrowing or occlusion of blood vessels or other hollow fluid-filled channels in the body.

The results described herein provide a compelling example of how engineering approaches inspired by pathophysiological mechanisms can be used to develop biomimetic therapeutic systems. The targeted drug delivery system described herein is distinct from conventional biochemical targeting approaches (e.g., using specific cell surface antigens) as it is based on increased local shear stress, which is a physical characteristic of narrowed vessels regardless of the cause or location of obstruction. The shear-activated delivery technology involves fabrication of microaggregates of NPs that remain intact under fluid shear stress levels experienced in normal small and large vessels, but disaggregate and deploy their active NP components in diseased regions characterized by high shear (>100 dyne/ cm²), much like living platelets do. In contrast to microscale aggregates, the dispersed NPs rapidly adhere to the surface of the adjacent vessel wall due to smaller drag forces, and this can be further enhanced by surface coatings on the NPs (e.g., tPA in the present study) that bind to endothelial cells or relevant vessel targets, such as fibrin clots. As a result, high local concentrations of drugs administered intravenously can be selectively delivered to multiple regions of vascular obstruction simultaneously, and a similar approach could be used to concentrate imaging agents to these stenotic sites. This biomimetic strategy provides a new path for therapeutic intervention in which life-saving therapies can be administered to patients with pulmonary embolism, stroke, or other acutely occluded blood vessels through systemic injection by emergency medical technicians or physicians immediately upon diagnosis, prior to transport of the patient to a hospital setting.

### Example 3: Shear stress controlled release from RBCs (reference)

Red blood cells ghosts were prepared using hypotonic hemolysis method. In brief, RBC were centrifuged from blood (2000g, 10min) and resuspended in calcium/magnesium free diluted PBS (PBS to DD water vol ratio of 1:10). The cells were allowed to incubate for 15 minutes at 4°C and then centrifuged (12,000g, 10min). This process was repeated four times. Afterwards the cells were loaded with FITC-dextran by incubating the cells with 5 mg/ml dextran in diluted PBS for 1 hour at 4°C. The cells were centrifuges, suspended in PBS buffer with Ca/Mg and allowed to reseal in a 37°C incubator for more than 2 hr. Following the resealing procedure the cells were washed in PBS for four times to remove any residuals in solution. **Figure 8** shows a fluorescence image of RBC ghosts loaded with FITC-dextran taken five days after preparation of FITC-dextran loaded ghosts.

A suspension of FITC-dextran loaded RBC ghosts was infused through a device without a stenosis region (640 micron height channel, wall shear stress 10 dyne/cm²) or with a stenosis region (80% stenosis, 80 micron in height). The suspension was then centrifuged and filtered through a 0.22 µm filter to remove RBCs and the fluorescence intensity was measured. As shown in **Fig. 9****,** the flow induced release was more than two fold higher with the stenosis compared to without the stenosis.

### Example 4: Shear stress controlled release from microcapsules (reference)

For nanocapsules, Pluronic/poly(ethylenimine) (F127/PEI) nanocapsules encapsulating rhodadmine dye was prepared by emulsification/solvent evaporation with slight modification from a previously reported method (S.H. Choi, S.H. Lee & T.G., Park, Temperature-sensitive pluronic/poly(ethylenimine) nanocapsules for thermally triggered disruption of intracellular endosomal compartment, Biomacromolecules. 2006 Jun;7(6):1864-70). Briefly, Pluronic F127 was activated with p-nitrophenyl chloroformate in tolune for 24 hours at room temperature. The product was precipitated in ether and characterized by 1H NMR. To prepare the nanocapsules, 30% of activated F127 and a small amount of hydrophobic dye (rhodamine) was dissolved in dichloromethane (1ml) and then added dropwise into a 10 ml aqueous PEI solution (7.5 w/v, pH 9). The mixture was stirred at room temperature for about an hour to obtain nano/micro capsules and to evaporate the entrapped dichloromethane. The obtained microcapsules were then purified either by centrifugation or neutralized and dialyzed against water at pH 4.

A suspension of Pluronic-PEI microcapsules loaded with FICT dextran (70kDa) was infused through a device without a stenosis region (640 micron height channel, wall shear stress 10 dyne/cm²) or with a stenosis region (80% stenosis, 80 micron in height). The suspension was then centrifuged and filtered through a 0.22µm filter to remove the microcapsules and the fluorescence intensity was measured. As shown in Fig. 10, the flow induced release was more than two fold higher with the stenosis compared to without the stenosis.

### References

1. R. Langer, Nature 392, 5 (1998).
2. V. P. Torchilin, European Journal of Pharmaceutical Sciences 11, S81 (2000).
3. T. Mammoto, D. E. Ingber, Development 137, 1407 (2010).
4. N. Resnick et al., Endothelium 10, 197 (2003).
5. J. J. Hathcock, Arteriosclerosis, Thrombosis, and Vascular Biology 26, 1729 (2006).
6. O. K. Baskurt, Biorheology 45, 629 (2008).
7. J. N. Topper, M. A. Gimbrone Jr, Molecular Medicine Today 5, 40 (1999).
8. S. A. Berger, L. D. Jou, Annual Review of Fluid Mechanics 32, 347 (2000).
9. J. Strony, A. Beaudoin, D. Brands, B. Adelman, American Journal of Physiology-Heart and Circulatory Physiology 265, H1787 (1993).
10. Z. M. Ruggeri, J. N. Orje, R. Habermann, A. B. Federici, A. J. Reininger, Blood 108, 1903 (2006).
11. O. C. Farokhzad et al., Anal. Chem 77, 5453 (2005).
12. C. E. Astete, C. M. Sabliov, Journal of Biomaterials Science, Polymer Edition 17, 247 2006).
13. J. C. Sung et al., Pharmaceutical Research 26, 1847 (2009).
14. N. Tsapis, D. Bennett, B. Jackson, D. A. Weitz, D. A. Edwards, Proceedings of the National Academy of Sciences of the United States of America 99, 12001 (2002).
15. K. Avgoustakis, Current Drug Delivery 1, 321 (2004).
16. K. C. Koskinas et al., Circulation 121, 2092 (2010).
17. P. H. Stone et al., European Heart Journal 28, 705 (2007).
18. T. G. Kwiatkowski et al., New England Journal of Medicine 340, 1781 (1999).
19. J. C. Murciano et al., Nature Biotechnology 21, 891 (2003).
20. V. F. Tapson, New England Journal of Medicine 358, 1037 (2008).
21. C. J. L. Murray, A. D. Lopez, The Lancet 349, 1269 (1997).
22. T. J. Ingall et al., Stroke 35, 2418 (2004).
23. T. G. Kwiatkowski et al., New England Journal of Medicine 340, 1781 (1999).
24. J. Strony, A. Beaudoin, D. Brands, B. Adelman, American Journal of Physiology-Heart and Circulatory Physiology 265, H1787 (1993).
25. D. M. Wootton, D. N. Ku, Annual review of biomedical engineering 1, 299 (1999).
26. J. M. Siegel, C. P. Markou, D. N. Ku, S. Hanson, Journal of biomechanical engineering 116, 446 (1994).
27. Z. M. Ruggeri, J. N. Orje, R. Habermann, A. B. Federici, A. J. Reininger, Blood 108, 1903 (2006).
28. W. S. Nesbitt et al., Nature medicine 15, 665 (2009).
29. S. Goto et al., Circulation 99, 608 (1999).
30. R. C. Sonntag, W. B. Russel, Journal of colloid and interface science 113, 399 (1986).
31. H. A. Stone, Annual Review of Fluid Mechanics 26, 65 (1994).
32. R. Gref et al., Science 263, 1600 (1994).
33. V. F. Tapson, New England Journal of Medicine 358, 1037 (2008).
34. S. G. Soriano, S. A. Lipton, Nature medicine 4, (1998).
35. B. E. Figueroa, R. F. Keep, A. L. Betz, J. T. Hoff, J. H. Garcia, Stroke 29, 1202 (1998).
36. C. D. Murray, The Journal of General Physiology 9, 835 (1926).
37. M. Zamir, The Journal of General Physiology 69, 449 (1977).
38. N. Resnick et al., Endothelium 10, 197 (2003).
39. J. J. Hathcock, Arteriosclerosis, thrombosis, and vascular biology 26, 1729 (2006).
40. O. K. Baskurt, Biorheology 45, 629 (2008).
41. J. N. Topper, M. A. Gimbrone Jr, Molecular medicine today 5, 40 (1999).
42. J. A. Champion, A. Walker, S. Mitragotri, Pharmaceutical research 25, 1815 (2008).
43. O. C. Farokhzad et al., Anal. Chem 77, 5453 (2005).
44. R. Wiewrodt et al., Blood 99, 912 (2002).
45. S.H. Choi, S.H. Lee & T.G., Park, Biomacromolecules, 7(6):1864-70 (2006).

## Claims

1. A pharmaceutical composition for use in the treatment of a stenosis and/or a stenotic lesion, and/or an occlusive lesion in a subject comprising an aggregate, the aggregate comprising a plurality of nanoparticles, the nanoparticles comprising copolymers of polylactic acid and polyglycolic acid, wherein:
the aggregate disaggregates above a predetermined shear stress when exposed to said predetermined shear stress at the site of stenosis or occlusion; and
the aggregate further comprises a molecule which is a therapeutic agent or
pharmaceutically acceptable salt thereof, comprising an antithrombotic and/or thrombolytic agent, and/or a vasodilator.

2. Use of a pharmaceutical composition for the imaging of a stenosis and/or a stenotic lesion, and/or an occlusive lesion, wherein the composition comprises an aggregate, the aggregate comprises a plurality of nanoparticles, the nanoparticles comprise copolymers of polylactic acid and polyglycolic acid, and wherein:
the aggregate disaggregates above a predetermined shear stress when exposed to said predetermined shear stress at the site of stenosis or occlusion; and
the aggregate further comprises a molecule which is an imaging agent.

3. The pharmaceutical composition for use according to claim 1 or use of the pharmaceutical composition according to claim 2, wherein the molecule is encapsulated in the nanoparticle, is absorbed/adsorbed on the surface of the nanoparticle, or is covalently linked to the nanoparticle.

4. The pharmaceutical composition for use according to claim 3 or use of the pharmaceutical composition according to claim 3, wherein the molecule is covalently linked to the nanoparticle via a linker.

5. The pharmaceutical composition for use according to any of claims 1, 3 or 4, wherein the antithrombotic and/or thrombolytic agent is selected from the group consisting of anticoagulants, pro-coagulant antagonists, antiplatelet agents, thrombolytic agents, anti-thrombolytic agent antagonists, fibrinolytic enzymes, and any combinations thereof.

6. Use of the pharmaceutical composition according to any of claims 2 to 4, wherein the imaging agent is selected from the group consisting of Alexa Fluor® dyes (InvitrogenCorp.; Carlsbad, Calif); fluorescein; fluorescein isothiocyanate (FITC); Oregon GreenTM; tetrarhodamine isothiocynate (TRITC), 5-carboxyfluorescein (FAM); 2'7'-dimethoxy-4'5'-dichloro-6-carboxyfluorescein (JOE); tetrachlorofluorescein (TET); 6-carboxyrhodamine (R6G); N,N,N,N'-tetramefhyl-6-carboxyrhodamine (TAMRA); 6-carboxy-X-rhodamine (ROX); naphthylamine dyes that have an amino group in the alpha or beta position, such as naphthylamino compounds including 1-dimethylamino-naphthyl-5-sulfonate, 1-anilino-8-naphthalene sulfonate, 2-p-toluidinyl-6-naphthalene sulfonate, and 5-(2'-aminoethyl)aminonaphthalene-1-sulfonic acid (EDANS)); coumarins, such as 3-phenyl-7-isocyanatocoumarin; acridines, such as 9-isothiocyanatoacridine and acridine orange; N-(p(2-benzoxazolyl)phenyl)maleimide; cyanines, such as Cy2, indodicarbocyanine 3 (Cy3), indodicarbocyanine 5 (Cy5), indodicarbocyanine 5.5 (Cy5.5), 3-(-carboxy-pentyl)-3'ethyl-5,5'-dimethyloxacarbocyanine (CyA); 1H,5H,11H, 15H-Xantheno[2,3,4-ij:5,6,7-i'j']diquinolizin-18-ium, 9-[2(or 4)-[[[6-[2,5-dioxo-1-pyrrolidinyl)oxy]-6-oxohexyl]amino]sulfonyl]-4(or 2)-sulfophenyl]-2,3,6,7,12,13,16,17octahydro-inner salt (TR or Texas Red); BODIPYTM dyes; benzoxadiazoles; stilbenes; pyrenes; fluorescent proteins, such as green fluorescent protein, enhanced green fluorescent protein (EGFP), destabilized EGFP, red fluorescent protein (e.g., DsRed), dsREd variants mRFPmars and mRFPruby, yellow fluorescent protein, cyan fluorescent protein, blue fluorescent protein, cerulean fluorescent proteins, and variants thereof; radioisotopes, such as ⁹⁹mTc, ⁹⁵Tc, ¹¹¹In, ⁶²Cu, ⁶⁴Cu, Ga, ⁶⁸Ga, ¹⁵³Gd, ¹⁸F, ¹²⁴I, ¹²⁵I, ¹³¹I, ⁴⁷Sc, ⁶⁴Cu, ⁶⁷Cu, ⁸⁹Sr, ⁸⁶Y, ⁸⁷Y, ⁹⁰Y, ¹⁰⁵Rh, ¹¹¹Ag, ¹¹¹In, ¹¹⁷mSn, ¹⁴⁹Pm, ¹⁵³Sm, ¹⁶⁶Ho, ¹⁷⁷Lu, ¹⁸⁶Re, ¹⁸⁸Re, ²¹¹At, ²¹²Bi; paramagnetic metal ions, such as Gd(III), Dy(III), Fe(III), and Mn(II); X-ray absorbers, such as, Re, Sm, Ho, Lu, Pm, Y, Bi, Pd, Gd, La, Au, Au, Yb, Dy, Cu, Rh, Ag, and Ir; and any combinations thereof.

7. The pharmaceutical composition for use according to any of claims 1, 3, 4 or 5, wherein the thrombolytic agent is selected from the group consisting of tissue-type plasminogen activator (t-PA), streptokinase (SK), prourokinase, urokinase (uPA), alteplase (also known as Activase®, Genentech, Inc.), reteplase (also known as r-PA or retavase®, Centocor, Inc.), tenecteplase (also known as TNKTM, Genentech, Inc.), Streptase® (AstraZeneca, LP), lanoteplase (Bristol-Myers Squibb Company), monteplase (Eisai Company, Ltd.), saruplase (also known as r-scu-PA and rescupaseTM, Grunenthal GmbH, Corp.), staphylokinase, and anisoylated plasminogen-streptokinase activator complex (also known as APSAC, Anistreplase and Eminase®, SmithKline Beecham Corp.), and any combinations thereof.

8. The pharmaceutical composition for use according to any of claims 1, 3, 4, 5 or 7, or use of the pharmaceutical composition according to any of claims 2, 3, 4 or 6, wherein the molecule is released at a higher rate and/or in higher amount from a disaggregated aggregate relative to a non-disaggregated aggregate.

9. The pharmaceutical composition for use according to any of claims 1, 3, 4, 5, 7 or 8, or use of the pharmaceutical composition according to any of claims 2, 3, 4, 6 or 8, wherein at least one nanoparticle in the plurality of nanoparticles comprises a ligand, and wherein the ligand is selected from the group consisting of:
(i) peptides, polypeptides, proteins, peptidomimetics, glycoproteins, lectins, nucleosides, nucleotides, nucleic acids (e.g., polynucleotides, oligonucleotides, genes, genes including control and termination regions, self-replicating systems such as viral or plasmid DNA, single-stranded and double-stranded siRNAs and other RNA interference reagents (RNAi agents or iRNA agents), short-hairpin RNAs (shRNA), antisense oligonucleotides, ribozymes, microRNAs, microRNA mimics, supermirs, aptamers, antimirs, antimirs, antagomirs, U1 adaptors, triplex-forming oligonucleotides, RNA activators, immuno-stimulatory oligonucleotides, and decoy oligonucleotides), monosaccharides, disaccharides, trisaccharides, oligosaccharides, polysaccharides, glycosaminoglycans, lipopolysaccharides, lipids, vitamins, steroids, hormones, cofactors, receptors, receptor ligands, and analogs and derivatives thereof;
(ii) polylysine (PLL), poly L-aspartic acid, poly L-glutamic acid, styrenemaleic acid anhydride copolymer, poly(L-lactide-co-glycolide) copolymer, divinyl ether-maleic anhydride copolymer, N-(2-hydroxypropyl)methacrylamide copolymer (HMPA), polyethylene glycol (PEG), polyvinyl alcohol (PVA), polyurethane, poly(2-ethylacryllic acid), N-isopropylacrylamide polymers, polyphosphazine, polyethylenimine, cspermine, spermidine, polyamine, pseudopeptide-polyamine, peptidomimetic polyamine, dendrimer polyamine, arginine, amidine, protamine, thyrotropin, melanotropin, lectin, surfactant protein A, mucin, transferrin, bisphosphonate, polyglutamate, polyaspartate, an aptamer, asialofetuin, hyaluronan, procollagen, insulin, transferrin, albumin, acridines, cross- psoralen, mitomycin C, TPPC4, texaphyrin, Sapphyrin, polycyclic aromatic hydrocarbons (e.g., phenazine, dihydrophenazine), bile acids, cholesterol, cholic acid, adamantane acetic acid, 1-pyrene butyric acid, dihydrotestosterone, 1,3-Bis-O(hexadecyl)glycerol, geranyloxyhexyl group, hexadecylglycerol, borneol, menthol, 1,3-propanediol, heptadecyl group, palmitic acid, myristic acid, O3-(oleoyl)lithocholic acid, O3-(oleoyl)cholenic acid, dimethoxytrityl, or phenoxazine), RGD peptide, radiolabeled markers, haptens, naproxen, aspirin, dinitrophenyl, HRP, AP, lectins, vitamin A, vitamin E, vitamin K, vitamin B, folic acid, B12, riboflavin, biotin, pyridoxal, taxon, vincristine, vinblastine, cytochalasin, nocodazole, japlakinolide, latrunculin A, phalloidin, swinholide A, indanocine, myoservin, tumor necrosis factor alpha (TNFalpha), interleukin-1 beta, gamma interferon, GalNAc, galactose, mannose, mannose-6P, clusters of sugars such as GalNAc cluster, mannose cluster, galactose cluster, an aptamer, integrin receptor ligands, chemokine receptor ligands, serotonin receptor ligands, PSMA, endothelin, GCPII, somatostatin, and any combinations thereof; and/or
(iii) SEQ ID NO: 1 (CREKA), SEQ ID NO: 2 (CRKRLDRNK), SEQ ID NO: 3 (CHVLWSTRC), SEQ ID NO: 4 (ALEALAEALEALAEA), SEQ ID NO: 5 (KFFKFFKFFK (Bacterial cell wall permeating peptide)), SEQ ID NO: 6 (AALEALAEALEALAEALEALAEAAAAGGC (GALA)), SEQ ID NO: 7 (ALAEALAEALAEALAEALAEALAAAAGGC (EALA)), SEQ ID NO: 8 (GLFEAIEGFIENGWEGMIWDYG (INF-7)), SEQ ID NO: 9 (GLFGAIAGFIENGWEGMIDGWYG (Inf HA-2)), SEQ ID NO: 10 (GLF EAI EGFI ENGW EGMI DGWYGC GLF EAI EGFI ENGW EGMI DGWYGC (diINF-7)), SEQ ID NO: 11 (GLF EAI EGFI ENGW EGMI DGGC GLF EAI EGFI ENGW EGMI DGGC (diINF-3)), SEQ ID NO: 12 (GLFGALAEALAEALAEHLAEALAEALEALAAGGSC (GLF)), SEQ ID NO: 13 (GLFEAIEGFIENGWEGLAEALAEALEALAAGGSC (GALA-INF3)), SEQ ID NO: 14 (GLF EAI EGFI ENGW EGnI DG K GLF EAI EGFI ENGW EGnI DG (INF-5, n is norleucine)), SEQ ID NO: 15 (RQIKIWFQNRRMKWKK (penetratin)), SEQ ID NO: 16 (GRKKRRQRRRPPQC (Tat fragment 48-60)), SEQ ID NO: 17 (GALFLGWLGAAGSTMGAWSQPKKKRKV (signal sequence based peptide)), SEQ ID NO: 18 (LLIILRRRIRKQAHAHSK (PVEC)), SEQ ID NO: 19 (WTLNSAGYLLKINLKALAALAKKIL (transportan)), SEQ ID NO: 20 (KLALKLALKALKAALKLA (amphiphilic model peptide)), SEQ ID NO: 21 (RRRRRRRRR (Arg9)), SEQ ID NO: 22 (LLGDFFRKSKEKIGKEFKRIVQRIKDFLRNLVPRTES (LL-37)), SEQ ID NO: 23 (SWLSKTAKKLENSAKKRISEGIAIAIQGGPR (cecropin P1)), SEQ ID NO: 24 (ACYCRIPACIAGERRYGTCIYQGRLWAFCC (a-defensin)), SEQ ID NO: 25 (DHYNCVSSGGQCLYSACPIFTKIQGTCYRGKAKCCK (β-defensin)), SEQ ID NO: 26 (RRRPRPPYLPRPRPPPFFPPRLPPRIPPGFPPRFPPRFPGKR-NH2 (PR-39)), SEQ ID NO: 27 ILPWKWPWWPWRR-NH2 (indolicidin)), SEQ ID NO: 28 (AAVALLPAVLLALLAP (RFGF)), SEQ ID NO: 29 (AALLPVLLAAP (RFGF analogue)), SEQ ID NO: 30 (RKCRIVVIRVCR (bactenecin)), cecropins, lycotoxins, paradaxins, buforin, CPF, bombinin-like peptide (BLP), cathelicidins, ceratotoxins, S. *clava* peptides, hagfish intestinal antimicrobial peptides (HFIAPs), magainines, brevinins-2, dermaseptins, melittins, pleurocidin, H₂A peptides, Xenopus peptides, esculentinis-1, caerins, and any analogs and derivatives thereof.

10. The pharmaceutical composition for use according to claim 9 or use of the pharmaceutical composition according to claim 9, wherein the ligand decreases the rate of disaggregation by at least 10% relative to a control, and/or the aggregate disaggregates by at least 5% in a stenosis region relative to a non-stenosis region.

11. The pharmaceutical composition for use according to any of claims 1, 3, 4, 5, 7, 8, 9 or 10, or use of the pharmaceutical composition according to any of claims 2, 3, 4, 6, 8, 9 or 10, wherein surface of the nanoparticles is modified to modulate intermolecular electrostatic interactions, hydrogen bonding interactions, dipole-dipole interactions, hydrophilic interaction, hydrophobic interactions, van der Waal's forces, and any combinations thereof between two or more nanoparticles.

12. The pharmaceutical composition for use according to any of claims 1, 3, 4, 5, 7, 8, 9, 10 or 11, or use of the pharmaceutical composition according to any of claims 2, 3, 4, 6, 8, 9, 10 or 11, wherein the aggregate is of size ≤ 50µm, preferably wherein the aggregate is 1µm to 20µm in size.

13. The pharmaceutical composition for use according to any of claims 1, 3, 4, 5, 7, 8, 9, 10, 11 or 12, or use of the pharmaceutical composition according to any of claims 2, 3, 4, 6, 8, 9, 10, 11 or 12, wherein the nanoparticle comprises at least one moiety that increases the *in vivo* lifetime of the aggregate, preferably the at least one moiety is polyethylene glycol.

14. The pharmaceutical composition for use according to any of claims 1, 3, 4, 5, 7, 8, 9, 10, 11, 12 or 13, or use of the pharmaceutical composition according to any of claims 2, 3, 4, 6, 8, 9, 10, 11, 12 or 13, wherein the stenosis, stenotic, or occlusive lesion is selected from the group consisting of intermittent claudication (peripheral artery stenosis), angina (coronary artery stenosis) or myocardial infarction, carotid artery stenosis, aortic stenosis, buttonhole stenosis, calcific nodular stenosis, coronary ostial stenosis, double aortic stenosis, fish-mouth mitral stenosis, idiopathic hypertrophic subaortic stenosis, infundibular stenosis, mitral stenosis, muscular subaortic stenosis, pulmonary stenosis, pulmonary embolism, pulmonary hypertension, subaortic stenosis, subvalvar stenosis, supravalvar stenosis, tricuspid stenosis, renal artery stenosis, vascular hypertension, sickle cell anemia, and any combinations thereof; and/or results from trauma or injury, atherosclerosis, birth defects, diabetes, iatrogenic, infection, inflammation, ischemia, neoplasm, vasospasm, coronary vasospasm, Raynaud's phenomenon, stroke, blood clotting, Moyamoya disease, Takayasu's disease, polyarteritis nodosa, disseminated lupus erythematous, rheumatoid arthritis, tumors of the spine, Paget's disease of bone, fluorosis, hemodialysis, and any combinations thereof.

15. The pharmaceutical composition for use according to any of claims 1, 3, 4, 5, 7, 8, 9, 10, 11, 12, 13 or 14, or use of the pharmaceutical composition according to any of claims 2, 3, 4, 6, 8, 9, 10, 11, 12, 13 or 14 in combination with embolization treatments.

16. The pharmaceutical composition for use according to claim 1, wherein the vasodilator is selected from the group consisting of prazosin, terazosin, doxazosin, trimazosin, phentolamine, phenoxybenzamine, benazepril, captopril, enalapril, fosinopril, lisinopril, moexipril, quinapril, ramipril, candesartan, eprosartan, irbesartan, losartan, olmesartan, telmisartan, valsartan, Epinephrine, Norepinephrine, Dopamine, Dobutamine, Isoproterenol, amlodipine, felodipine, isradipine, nicardipine, nifedipine, nimodipine, nitrendipine, clonidine, guanabenz, guanfacine, α-methyldopa, hydralazine, Bosentan, trimethaphan camsylate, isosorbide dinitrate, isosorbide mononitrate, nitroglycerin, erythrityl tetranitrate, pentaerythritol tetranitrate, sodium nitroprusside, milrinone, inamrinone (formerly amrinone), cilostazol, sildenafil, tadalafil, minoxidil, aliskiren, and pharmaceutically acceptable salts thereof.

17. The pharmaceutical composition for use according to claim 16, wherein the vasodilator is nitroglycerin.

## Patentansprüche

1. Pharmazeutische Zusammensetzung zur Verwendung in der Behandlung einer Stenose und/oder stenotischen Läsion und/oder einer okklusiven Läsion bei einem Subjekt, welche ein Aggregat umfasst, wobei das Aggregat eine Vielzahl von Nanopartikeln umfasst, wobei die Nanopartikel Copolymere von Polymilchsäure und Polyglykolsäure umfassen, wobei:
das Aggregat oberhalb einer vorgegebenen Scherbeanspruchung disaggregiert, wenn es der vorgegebenen Scherbeanspruchung an dem Situs der Stenose oder Okklusion ausgesetzt wird; und
das Aggregat ferner ein Molekül umfasst, welches ein therapeutisches Mittel oder pharmazeutisch annehmbares Salz davon ist, welches ein antithrombotisches und/oder thrombolytisches Mittel und/oder einen Vasodilatator umfasst.

2. Verwendung einer pharmazeutischen Zusammensetzung zur Bildgebung einer Stenose und/oder stenotischen Läsion und/oder einer okklusiven Läsion, wobei die Zusammensetzung ein Aggregat umfasst, das Aggregat eine Vielzahl von Nanopartikeln umfasst, die Nanopartikel Copolymere von Polymilchsäure und Polyglykolsäure umfassen, und wobei:
das Aggregat oberhalb einer vorgegebenen Scherbeanspruchung disaggregiert, wenn es der vorgegebenen Scherbeanspruchung an dem Situs der Stenose oder Okklusion ausgesetzt wird; und
das Aggregat ferner ein Molekül umfasst, das ein Bildgebungsmittel ist.

3. Pharmazeutische Zusammensetzung zur Verwendung nach Anspruch 1 oder Verwendung der pharmazeutischen Zusammensetzung nach Anspruch 2, wobei das Molekül in dem Nanopartikel verkapselt ist, auf der Oberfläche des Nanopartikels absorbiert/adsorbiert ist, oder kovalent mit dem Nanopartikel verknüpft ist.

4. Pharmazeutische Zusammensetzung zur Verwendung nach Anspruch 3 oder Verwendung der pharmazeutischen Zusammensetzung nach Anspruch 3, wobei das Molekül über einen Linker kovalent mit dem Nanopartikel verknüpft ist.

5. Pharmazeutische Zusammensetzung zur Verwendung nach einem der Ansprüche 1, 3 oder 4, wobei das antithrombotische und/oder thrombolytische Mittel ausgewählt ist aus der Gruppe bestehend aus Antikoaguantien, Antagonisten von Prokoagulantien, Thrombozytenaggregationshemmern, thrombolytischen Mitteln, Antagonisten von antithrombolytischen Mitteln, fibrinolytischen Enzymen und jeglichen Kombinationen davon.

6. Verwendung der pharmazeutischen Zusammensetzung nach einem der Ansprüche 2 bis 4, wobei das bildgebende Mittel ausgewählt ist aus der Gruppe bestehend aus Alexa Fluor® Farbstoffen (InvitrogenCorp.; Carlsbad, Californien, USA); Fluorescein; Fluoresceinisothiocyanat (FITC); Oregon Green™; Tetrarhodaminisothiocyanat (TRITC), 5-Carboxyfluorescein (FAM); 2',7'-Dimethoxy-4',5'-dichlor-6-carboxyfluorescein (JOE); Tetrachlorfluorescein (TET); 6-Carboxyrhodamin (R6G); N,N,N,N'-Tetramethyl-6-carboxyrhodamin (TAMRA); 6-Carboxy-X-rhodamin (ROX); Naphthylaminfarbstoffen, die eine Aminogruppe in der alpha- oder beta-Position aufweisen, wie Naphthylaminoverbindungen, einschließlich 1-Dimethylaminonaphthyl-5-sulfonat, 1-Anilino-8-naphthalinsulfonat, 2-p-Toluidinyl-6-naphthalinsulfonat und 5-(2'-Aminoethyl)aminonaphthalin-1-sulfonsäure (EDANS)); Cumarinen, wie 3-Phenyl-7-isocyanatocumarin; Acridinen, wie 9-Isothiocyanatoacridin und Acridinorange; N-(p(2-Benzoxazolyl)phenyl)maleimid; Cyaninen, wie Cy2, Indodicarbocyanin 3 (Cy3), Indodicarbocyanin 5 (Cy5), Indodicarbocyanin 5.5 (Cy5.5), 3-(ε-Carboxypentyl)-3'-ethyl-5,5'-dimethyloxacarbocyanin (CyA); 1H,5H,1 1H,15H-Xantheno[2,3,4-ij:5,6,7-i'j']dichinolizin-18-ium, 9-[2(oder 4)-[[[6-[2,5-Dioxo-1-pyrrolidinyl)oxy]-6-oxohexyl]amino]sulfonyl]-4(oder 2)-sulfophenyl]-2,3,6,7,12,13,16, 17-octahydroinnerem Salz (TR oder Texas Red); BODIPY™-Farbstoffen; Benzoxadiazolen; Stilbenen; Pyrenen; fluoreszierenden Proteinen, wie grün fluoreszierendem Protein, weiterentwickeltem ("enhanced") grün fluoreszierendem Protein (EGFP), destabilisiertem EGFP, rot fluoreszierendem Protein (z.B. DsRed), den dsREd-Varianten mRFPmars und mRFPruby, gelb fluoreszierendem Protein, cyan fluoreszierendem Protein, blau fluoreszierendem Protein, himmelblau fluoreszierenden Proteinen und Varianten davon; Radioisotopen, wie ⁹⁹mTc, ⁹⁵Tc, ¹¹¹In, ⁶²Cu, ⁶⁴Cu, Ga, ⁶⁸Ga, ¹⁵³Gd, ¹⁸F, ¹²⁴I, ¹²⁵I, ¹³¹I, ⁴⁷Sc, ⁶⁴Cu, ⁶⁷Cu, ⁸⁹Sr, ⁸⁶Y, ⁸⁷Y, ⁹⁰Y, ¹⁰⁵Rh, ¹¹¹Ag, ¹¹¹In, ¹¹⁷mSn, ¹⁴⁹Pm, ¹⁵³Sm, ¹⁶⁶Ho, ¹⁷⁷Lu, ¹⁸⁶Re, ¹⁸⁸Re, ²¹¹At, ²¹²Bi; paramagnetischen Metallionen, wie Gd(III), Dy(III), Fe(III) und Mn(II); Röntgenstrahlen absorbierenden Substanzen, wie Re, Sm, Ho, Lu, Pm, Y, Bi, Pd, Gd, La, Au, Au, Yb, Dy, Cu, Rh, Ag und Ir; und jeglichen Kombinationen davon.

7. Pharmazeutische Zusammensetzung zur Verwendung nach einem der Ansprüche 1, 3, 4 oder 5, wobei das thrombolytische Mittel ausgewählt ist aus gewebespezifischem Plasminogenaktivator (t-PA), Streptokinase (SK), Prourokinase, Urokinase (uPA), Alteplase (auch bekannt als Activase®, Genentech, Inc.), Reteplase (auch bekannt als r-PA oder Retavase®, Centocor, Inc.), Tenecteplase (auch bekannt als TNKTM, Genentech, Inc.), Streptase® (AstraZeneca, LP), Lanoteplase (Bristol-Myers Squibb Company), Monteplase (Eisai Company, Ltd.), Saruplase (auch bekannt als r-scu-PA und Rescupase™, Grünenthal GmbH, Corp.), Staphylokinase und anisoyliertem Plasminogen-Streptokinase-Aktivatorkomplex (auch bekannt als APSAC, Anistreplase und Eminase®, SmithKline Beecham Corp.) und jeglichen Kombinationen davon.

8. Pharmazeutische Zusammensetzung zur Verwendung nach einem der Ansprüche 1, 3, 4, 5 oder 7, oder Verwendung der pharmazeutischen Zusammensetzung nach einem der Ansprüche 2, 3, 4 oder 6, wobei das Molekül von einem disaggregiertem Aggregat mit einer höheren Rate und/oder in einer höheren Menge freigesetzt wird als von einem nicht-disaggregiertem Aggregat.

9. Pharmazeutische Zusammensetzung zur Verwendung nach einem der Ansprüche 1, 3, 4, 5, 7 oder 8, oder Verwendung der pharmazeutischen Zusammensetzung nach einem der Ansprüche 2, 3, 4, 6 oder 8, wobei mindestens ein Nanopartikel in der Vielzahl der Nanopartikel einen Liganden umfasst, und wobei der Ligand ausgewählt ist aus der Gruppe bestehend aus:
(i) Peptiden, Polypeptiden, Proteinen, Peptidomimetika, Glycoproteinen, Lectinen, Nucleosiden, Nucleotiden, Nucleinsäuren (z. B. Polynucleotiden, Oligonucleotiden, Genen, Genen, die Kontroll- und Terminierungsregionen einschließen, selbstreplizierenden Systemen, wie viraler oder Plasmid-DNA, einsträngigen und doppelsträngigen siRNAs und anderen RNA-Interferenzreagentien (RNAi-Mitteln oder iRNA-Mitteln), kurzen Haarnadel-RNAs (shRNA), Antisense-Oligonucleotiden, Ribozymen, MikroRNAs, MikroRNA-Mimetika, Super-miRs, Aptamers, Anti-miRs, Anti-miRs, Antago-miRs, U1-Adaptoren, triplexbildenden Oligonucleotiden, RNA-Aktivatoren, immunstimulierenden Oligonucleotiden und Decoy-Oligonucleotiden), Monosacchariden, Disacchariden, Trisacchariden, Oligosacchariden, Polysacchariden, Glycosaminoglycanen, Lipopolysacchariden, Lipiden, Vitaminen, Steroiden, Hormonen, Cofaktoren, Rezeptoren, Rezeptorliganden und Analoga und Derivaten davon;
(ii) Polylysin (PLL), Poly-L-asparaginsäure, Poly-L-glutaminsäure, Styrol-Maleinsäureanhydrid-Copolymer, Poly(L-lactid-co-glykolid)-Copolymer, Divinylether-Maleinsäureanhydrid-Copolymer, N-(2-Hydroxypropyl)methacrylamid-Copolymer (HMPA), Polyethylenglykol (PEG), Polyvinylalkohol (PVA), Polyurethan, Poly(2-ethylacrylsäure), N-Isopropylacrylamidpolymeren, Polyphosphazin, Polyethylenimin, Cspermin, Spermidin, Polyamin, Pseudopeptidpolyamin, peptidomimetischem Polyamin, Dendrimerpolyamin, Arginin, Amidin, Protamin, Thyrotropin, Melanotropin, Lectin, Surfactant-Protein A, Mucin, Transferrin, Bisphosphonat, Polyglutamat, Polyaspartat, einem Aptamer, Asialofetuin, Hyaluronan, Procollagen, Insulin, Transferrin, Albumin, Acridinen, Cross-Psoralen, Mitomycin C, TPPC4, Texaphyrin, Sapphyrin, polycyclischen aromatischen Kohlenwasserstoffen (z. B. Phenazin, Dihydrophenazin), Gallensäuren, Cholesterol, Cholsäure, Adamantanessigsäure, 1-Pyrenbuttersäure, Dihydrotestosteron, 1,3-Bis-O(hexadecyl)glycerin, Geranyloxyhexylgruppe, Hexadecylglycerin, Borneol, Menthol, 1,3-Propandiol, Heptadecylgruppe, Palmitinsäure, Myristinsäure, O3-(Oleoyl)lithocholsäure, O3-(Oleoyl)cholensäure, Dimethoxytrityl oder Phenoxazin), RGD-Peptid, radiomarkierten Markern, Haptenen, Naproxen, Aspirin, Dinitrophenyl, HRP, AP, Lectinen, Vitamin A, Vitamin E, Vitamin K, Vitamin B, Folsäure, B12, Riboflavin, Biotin, Pyridoxal, Taxon, Vincristin, Vinblastin, Cytochalasin, Nocodazol, Japlakinolid, Latrunculin A, Phalloidin, Swinholid A, Indanocin, Myoservin, Tumornekrosefaktor alpha (TNFalpha), Interleukin-1 beta, gamma-Interferon, GalNAc, Galactose, Mannose, Mannose-6P, Cluster von Zuckern, wie GalNAc-Cluster, Mannose-Cluster, Galactose-Cluster, einem Aptamer, Integrin-Rezeptorliganden, Chemokin-Rezeptorliganden, Serotonin-Rezeptorliganden, PSMA, Endothelin, GCPII, Somatostatin und jeglichen Kombinationen davon; und/oder
(iii) SEQ ID NO: 1 (CREKA), SEQ ID NO: 2 (CRKRLDRNK), SEQ ID NO: 3 (CHVLWSTRC), SEQ ID NO: 4 (ALEALAEALEALAEA), SEQ ID NO: 5 (KFFKFFKFFK (bakterielle Zellwand permeierendem Peptid)), SEQ ID NO: 6 (AALEALAEALEALAEALEALAEAAAAGGC (GALA)), SEQ ID NO: 7 (ALAEALAEALAEALAEALAEALAAAAGGC (EALA)), SEQ ID NO: 8 (GLFEAIEGFIENGWEGMIWDYG (INF-7)), SEQ ID NO: 9 (GLFGAIAGFIENGWEGMIDGWYG (Inf HA-2)), SEQ ID NO: 10 (GLF EAI EGFI ENGW EGMI DGWYGC GLF EAI EGFI ENGW EGMI DGWYGC (diINF-7)), SEQ ID NO: 11 (GLF EAI EGFI ENGW EGMI DGGC GLF EAI EGFI ENGW EGMI DGGC (diINF-3)), SEQ ID NO: 12 (GLFGALAEALAEALAEHLAEALAEALEALAAGGSC (GLF)), SEQ ID NO: 13 (GLFEAIEGFIENGWEGLAEALAEALEALAAGGSC (GALA-INF3)), SEQ ID NO: 14 (GLF EAI EGFI ENGW EGnI DG K GLF EAI EGFI ENGW EGnI DG (INF-5, n ist Norleucin)), SEQ ID NO: 15 (RQIKIWFQNRRMKWKK (Penetratin)), SEQ ID NO: 16 (GRKKRRQRRRPPQC (Tat-Fragment 48-60)), SEQ ID NO: 17 (GALFLGWLGAAGSTMGAWSQPKKKRKV (auf Signalsequenz basierendem Peptid)), SEQ ID NO: 18 (LLIILRRRIRKQAHAHSK (PVEC)), SEQ ID NO: 19 (WTLNSAGYLLKINLKALAALAKKIL (Transportan)), SEQ ID NO: 20 (KLALKLALKALKAALKLA (amphiphilem Modellpeptid)), SEQ ID NO: 21 (RRRRRRRRR (Arg9)), SEQ ID NO: 22 (LLGDFFRKSKEKIGKEFKRIVQRIKDFLRNLVPRTES (LL-37)), SEQ ID NO: 23 (SWLSKTAKKLENSAKKRISEGIAIAIQGGPR (Cecropin P1)), SEQ ID NO: 24 (ACYCRIPACIAGERRYGTCIYQGRLWAFCC (α-Defensin)), SEQ ID NO: 25 (DHYNCVSSGGQCLYSACPIFTKIQGTCYRGKAKCCK (β-Defensin)), SEQ ID NO: 26 (RRRPRPPYLPRPRPPPFFPPRLPPRIPPGFPPRFPPRFPGKR-NH₂ (PR-39)), SEQ ID NO: 27 ILPWKWPWWPWRR-NH₂ (Indolicidin)), SEQ ID NO: 28 (AAVALLPAVLLALLAP (RFGF)), SEQ ID NO: 29 (AALLPVLLAAP (RFGF-Analogon)), SEQ ID NO: 30 (RKCRIVVIRVCR (Bactenecin)), Cecropinen, Lycotoxinen, Paradaxinen, Buforin, CPF, bombinin-artigem Peptid (BLP), Cathelicidinen, Ceratotoxinen, *S. clava*-Peptiden, antimikrobiellen Peptiden aus dem Magen-DarmTrakt des Ingers (HFIAPs), Magaininen, Brevininen-2, Dermaseptinen, Melittinen, Pleurocidin, H₂A-Peptiden, Xenopus-Peptiden, Esculentinis-1, Caerinen und jeglichen Analoga und Derivaten davon.

10. Pharmazeutische Zusammensetzung zur Verwendung nach Anspruch 9 oder Verwendung der pharmazeutischen Zusammensetzung nach Anspruch 9, wobei der Ligand die Disaggregationsrate um mindestens 10 % relativ zu einer Kontrolle verringert, und/oder das Aggregat in einer Stenoseregion relativ zu einer Nicht-Stenoseregion um mindestens 5 % disaggregiert.

11. Pharmazeutische Zusammensetzung zur Verwendung nach einem der Ansprüche 1, 3, 4, 5, 7, 8, 9 oder 10, oder Verwendung der pharmazeutischen Zusammensetzung nach einem der Ansprüche 2, 3, 4, 6, 8, 9 oder 10, wobei die Oberfläche der Nanopartikel modifiziert ist, um intermolekulare elektrostatische Wechselwirkungen, Wechselwirkungen über Wasserstoffbrückenbindungen, Dipol-Dipol-Wechselwirkungen, hydrophile Wechselwirkung, hydrophobe Wechselwirkungen, van der Waals-Kräfte und jegliche Kombinationen davon zwischen zwei oder mehr Nanopartikeln zu modulieren.

12. Pharmazeutische Zusammensetzung zur Verwendung nach einem der Ansprüche 1, 3, 4, 5, 7, 8, 9, 10 oder 11, oder Verwendung der pharmazeutischen Zusammensetzung nach einem der Ansprüche 2, 3, 4, 6, 8, 9, 10 oder 11, wobei das Aggregat eine Größe ≤ 50 µm aufweist, wobei das Aggregat vorzugsweise eine Größe von 1 µm bis 20 µm aufweist.

13. Pharmazeutische Zusammensetzung zur Verwendung nach einem der Ansprüche 1, 3, 4, 5, 7, 8, 9, 10, 11 oder 12, oder Verwendung der pharmazeutischen Zusammensetzung nach einem der Ansprüche 2, 3, 4, 6, 8, 9, 10, 11 oder 12, wobei das Nanopartikel mindestens eine Komponente umfasst, die die *in vivo*-Lebensdauer des Aggregats erhöht, wobei die mindestens eine Komponente vorzugsweise Polyethylenglykol ist.

14. Pharmazeutische Zusammensetzung zur Verwendung nach einem der Ansprüche 1, 3, 4, 5, 7, 8, 9, 10, 11, 12 oder 13, oder Verwendung der pharmazeutischen Zusammensetzung nach einem der Ansprüche 2, 3, 4, 6, 8, 9, 10, 11, 12 oder 13, wobei die Stenose, stenotische oder okklusive Läsion ausgewählt ist aus der Gruppe bestehend aus Claudicatio intermittens (peripherer Arterienstenose), Angina (Koronararterienstenose) oder Myokardinfarkt, Karotisarterienstenose, Aortenstenose, Knopflochstenose, kalzifizierter knotenförmiger Stenose, koronarer Ostiumstenose, doppelter Aortenstenose, Fischmund-Mitralstenose, idiopathischer hypertropher Subaortenstenose, infundibulärer Stenose, Mitralstenose, muskulärer Subaortenstenose, Pulmonarstenose, Lungenembolie, Lungenhochdruck, Subaortenstenose, subvalvulärer Stenose, supravalvulärer Stenose, Trikuspidalstenose, Nierenarterienstenose, vaskulärer Hypertonie, Sichelzellanämie und jeglichen Kombinationen davon; und/oder Resultaten von Trauma oder Verletzung, Atherosklerose, Geburtsfehlern, Diabetes, iatrogener Ursache, Infektion, Entzündung, Ischämie, Neoplasmen, Vasospasmen, koronaren Vasospasmen, Morbus Raynaud, Schlaganfall, Blutgerinnung, Moyamoya-Erkrankung, Takayasu-Erkrankung, Polyarteriitis nodosa, disseminiertem Lupus erythematous, rheumatoider Arthritis, Tumoren des Rückenmarks, Morbus Paget der Knochen, Fluorose, Hämodialyse und jeglichen Kombinationen davon.

15. Pharmazeutische Zusammensetzung zur Verwendung nach einem der Ansprüche 1, 3, 4, 5, 7, 8, 9, 10, 11, 12, 13 oder 14, oder Verwendung der pharmazeutischen Zusammensetzung nach einem der Ansprüche 2, 3, 4, 6, 8, 9, 10, 11, 12, 13 oder 14 in Kombination mit Embolisierungsbehandlungen.

16. Pharmazeutische Zusammensetzung zur Verwendung nach Anspruch 1, wobei der Vasodilatator ausgewählt ist aus der Gruppe bestehend aus Prazosin, Terazosin, Doxazosin, Trimazosin, Phentolamin, Phenoxybenzamin, Benazepril, Captopril, Enalapril, Fosinopril, Lisinopril, Moexipril, Quinapril, Ramipril, Candesartan, Eprosartan, Irbesartan, Losartan, Olmesartan, Telmisartan, Valsartan, Epinephrin, Norepinephrin, Dopamin, Dobutamin, Isoproterenol, Amlodipin, Felodipin, Isradipin, Nicardipin, Nifedipin, Nimodipin, Nitrendipin, Clonidin, Guanabenz, Guanfacin, α-Methyldopa, Hydralazin, Bosentan, Trimethaphan-Camsylat, Isosorbiddinitrat, Isosorbidmononitrat, Nitroglycerin, Erythrityltetranitrat, Pentaerythrittetranitrat, Nitroprussid-Natrium, Milrinon, Inamrinon (zuvor Amrinon), Cilostazol, Sildenafil, Tadalafil, Minoxidil, Aliskiren und pharmazeutisch annehmbaren Salzen davon.

17. Pharmazeutische Zusammensetzung zur Verwendung nach Anspruch 16, wobei der Vasodilatator Nitroglycerin ist.

## Revendications

1. Composition pharmaceutique pour utilisation dans le traitement d'une sténose, d'une lésion sténosée et/ou d'une lésion occlusive chez un sujet comprenant un agrégat, l'agrégat comprenant une pluralité de nanoparticules, les nanoparticules comprenant des copolymères d'acide polylactique et d'acide polyglycolique, dans laquelle :
l'agrégat se désagrège au-dessus d'une contrainte de cisaillement prédéterminée lorsqu'il est exposé à ladite contrainte de cisaillement prédéterminée au site d'une sténose ou d'une occlusion ; et
l'agrégat comprend en outre une molécule qui est un agent thérapeutique ou un sel pharmaceutiquement acceptable de celui-ci, comprenant un agent antithrombotique et/ou thrombolytique, et/ou un vasodilatateur.

2. Utilisation d'une composition pharmaceutique pour l'imagerie d'une sténose, d'une lésion sténosée et/ou d'une lésion occlusive, dans laquelle la composition comprend un agrégat, l'agrégat comprenant une pluralité de nanoparticules, les nanoparticules comprenant des copolymères d'acide polylactique et d'acide polyglycolique, et dans laquelle :
l'agrégat se désagrège au-dessus d'une contrainte de cisaillement prédéterminée lorsqu'il est exposé à ladite contrainte de cisaillement prédéterminée au site d'une sténose ou d'une occlusion ; et
l'agrégat comprend en outre une molécule qui est un agent d'imagerie.

3. Composition pharmaceutique pour utilisation selon la revendication 1, ou utilisation de la composition pharmaceutique selon la revendication 2, dans laquelle la molécule est encapsulée dans la nanoparticule, est absorbée/adsorbée sur la surface de la nanoparticule ou est liée de manière covalente à la nanoparticule.

4. Composition pharmaceutique pour utilisation selon la revendication 3, ou utilisation de la composition pharmaceutique selon la revendication 3, dans laquelle la molécule est liée de manière covalente à la nanoparticule par un lieur.

5. Composition pharmaceutique pour utilisation selon l'une quelconque des revendications 1, 3 ou 4, dans laquelle l'agent antithrombotique et/ou thrombolytique est sélectionné parmi un groupe constitué d'anticoagulants, d'antagonistes procoagulants, d'agents antiplaquettaires, d'agents thrombolytiques, d'antagonistes d'agents anti-thrombolytiques, d'enzymes fibrinolytiques et toute combinaison de ceux-ci.

6. Utilisation de la composition pharmaceutique selon l'une quelconque des revendications 2 à 4, dans laquelle l'agent d'imagerie est sélectionné parmi le groupe constitué des produits suivants : colorants Alexa Fluor® (InvitrogenCorp. ; Carlsbad, Calif) ; fluorescéine ; isothiocyanate de fluorescéine (FITC) ; Oregon GreenTM ; isothiocynate de tétrarhodamine (TRITC), 5-carboxyfluorescéine (FAM) ; 2'7'-diméthoxy-4'5'-dichloro-6-carboxyfluorescéine (JOE) ; tétrachlorofluorescéine (TET) ; 6-carboxyrhodamine (R6G) ; N,N,N,N'-tétraméthyl-6-carboxyrhodamine (TAMRA) ; 6-carboxy-X-rhodamine (ROX) ; colorants de naphtylamine qui ont un groupe amino dans la position alpha ou bêta, comme des composés naphtylamino y compris 1-diméthylamino-naphtyl-5-sulfonate, sulfonate de l-anilino-8-naphtalène, sulfonate de 2-p-toluidinyl-6-naphthalène et acide 5-(2'-aminoéthyl)aminonaphtalène-1-sulfonique (EDANS); coumarines comme 3-phényl-7-isocyanatocoumarine ; acridines comme 9-isothiocyanatoacridine et acridine orange ; N-(p(2-benzoxazolyl)phényl)maléimide ; cyanines comme Cy2, indodicarbocyanine 3 (Cy3), indodicarbocyanine 5 (Cy5), indodicarbocyanine 5.5 (Cy5.5), 3-(-carboxy-pentyl)-3'éthyl-5,5'-diméthyloxacarbocyanine (CyA) ; 1H,5H,11H, 15H-xanthéno[2,3,4-ij:5,6,7-i'j']diquinolizin-18-ium, 9-[2(ou 4)-[[[6-[2,5-dioxo-1-pyrrolidinyl)oxy]-6-oxohexyl]amino]sulfonyl]-4(ou 2)-sulfophényl]-2,3,6,7,12,13,16,17octahydro-sel interne (TR ou Rouge Texas) ; colorants BODIPYTM ; benzoxadiazoles ; stilbènes ; pyrènes ; protéines fluorescentes, comme protéine verte fluorescente, protéine verte fluorescente améliorée (EGFP), EGFP déstabilisée, protéine rouge fluorescente (p. ex., DsRed), variants dsREd de mRFPmars et de mRFPruby, protéine jaune fluorescente, protéine fluorescente cyan, protéine fluorescente bleue, protéine fluorescente réculéenne, et des variants de celles-ci; radioisotopes comme ⁹⁹mTc, ⁹⁵Tc, ¹¹¹In, ⁶²Cu, ⁶⁴Cu, Ga, ⁶⁸Ga, ¹⁵³Gd, ¹⁸F, ¹²⁴I, ¹²⁵I, ¹³¹I, ⁴⁷Sc, ⁶⁴Cu, ⁶⁷Cu, ⁸⁹Sr, ⁸⁶Y, ⁸⁷Y, ⁹⁰Y, ¹⁰⁵Rh, ¹¹¹Ag, ¹¹¹In, ¹¹⁷mSn, ¹⁴⁹Pm, ¹⁵³Sm, ¹⁶⁶Ho, ¹⁷⁷Lu, ¹⁸⁶Re, ¹⁸⁸Re, ²¹¹At, ²¹²Bi ; ions métalliques paramagnétiques, comme Gd(III), Dy(III), Fe(III) et Mn(II) ; absorbeurs de rayons X, comme Re, Sm, Ho, Lu, Pm, Y, Bi, Pd, Gd, La, Au, Au, Yb, Dy, Cu, Rh, Ag et Ir ; et toutes combinaisons de ceux-ci.

7. Composition pharmaceutique pour utilisation selon l'une quelconque des revendications 1, 3, 4 ou 5, dans laquelle l'agent thrombolytique est sélectionné parmi le groupe constitué d'un activateur de plasminogène de type tissu (t-PA), de la streptokinase (SK), de la prourokinase, de l'urokinase (uPA), de l'altéplase (également appelée Activase®, Genentech, Inc.), de la retéplase (également appelée r-PA ou rétavase®, Centocor, Inc.), de la ténectéplase (également appelée TNKTM, Genentech, Inc.), de la Streptase® (AstraZeneca, LP), de la lanotéplase (Bristol-Myers Squibb Company), de la montéplase (Eisai Company, Ltd.), de la saruplase (également appelée r-scu-PA et rescupaseTM, Grunenthal GmbH, Corp.), de la staphylokinase et du complexe activateur plasminogène-streptokinase anisolée (également appelé APSAC, Anistreplase et Eminase®, SmithKline Beecham Corp.), et toutes combinaisons de celles-ci.

8. Composition pharmaceutique pour utilisation selon l'une quelconque des revendications 1, 3, 4, 5 ou 7, ou utilisation de la composition pharmaceutique selon l'une quelconque des revendications 2, 3, 4 ou 6, dans laquelle la molécule est libérée à un taux supérieur et/ou en une quantité supérieure d'un agrégat désagrégé par rapport à un agrégat non désagrégé.

9. Composition pharmaceutique pour utilisation selon l'une quelconque des revendications 1, 3, 4, 5, 7 ou 8, ou utilisation de la composition pharmaceutique selon l'une quelconque des revendications 2, 3, 4, 6 ou 8, dans laquelle au moins une nanoparticule dans la pluralité de nanoparticules comprend un ligand, et dans laquelle le ligand est sélectionné parmi le groupe constitué de :
(i) peptides, polypeptides, protéines, peptidomimétiques, glycoprotéines, lectines, nucléosides, nucléotides, acides nucléiques (p. ex., polynucléotides, oligonucléotides, gènes, gènes comprenant des régions de contrôle et de terminaison, systèmes s'auto-répliquant tel que de l'ADN viral ou plasmidique, ARNsi à simple brin ou à double brin et autres réactifs d'interférence d'ARN (agents ARNi ou agents iARN), ARN courts en épingle (ARNsh), oligonucléotides antisens, ribozymes, microARN, imitations de microARN, supermiR, aptamères, antimiR, antimiR, antagomiR, adaptateurs U1, oligonucléotides formant des triplex, activateurs d'ARN, oligonucléotides immuno-stimulants et oligonucléotides leurres), monosaccharides, disaccharides, trisaccharides, oligosaccharides, polysaccharides, glycosaminoglycanes, lipopolysaccharides, lipides, vitamines, stéroïdes, hormones, cofacteurs, récepteurs, ligands récepteurs et analogues et dérivés de ceux-ci ;
(ii) polylysine (PLL), acide poly-L-aspartique, acide poly-L-glutamique, copolymère de styrène-anhydride d'acide maléique, copolymère de poly(L-lactide-co-glycolide), copolymère d'éther divinylique-anhydride maléique, copolymère de N-(2-hydroxypropyl)méthacrylamide (HMPA), polyéthylèneglycol (PEG), alcool polyvinylique (PVA), polyuréthane, acide poly(2-éthylacryllique), polymères de N-isopropylacrylamide, polyphosphazine, polyéthylénimine, cspermine, spermidine, polyamine, pseudopeptide-polyamine, polyamine peptidominétique, polyamine dendrimère, arginine, amidine, protamine, thyrotropine, mélanotropine, lectine, protéine tensioactive A, mucine, transferrine, bisphosphonate, polyglutamate, polyaspartate, un aptamère, asialofétuine, hyaluronane, procollagène, insuline, transferrine, albumine, acridines, psoralène croisée, mitomycine C, TPPC4, texaphyrine, saphirine, hydrocarbures polycycliques aromatiques (p. ex., phénazine, dihydrophénazine), acides biliaires, cholestérol, acide cholique, acide acétique d'adamantane, acide 1-pyrène butyrique, dihydrotestostérone, 1,3-bis-O(hexadécyl)glycérol, groupe géranyloxyhexyle, hexadécylglycérol, bornéol, menthol, 1,3-propanediol, groupe heptadécyle, acide palmitique, acide myristique, acide O3-(oléoyl)lithocholique, acide O3-(oléoyl)cholénique, diméthoxytrityle ou phénoxazine), peptide RGD, marqueurs radioactifs, haptènes, naproxène, aspirine, dinitrophényle, HRP, AP, lectines, vitamine A, vitamine E, vitamine K, vitamine B, acide folique, B12, riboflavine, biotine, pyridoxal, taxon, vincristine, vinblastine, cytochalasine, nocodazole, japlakinolide, latrunculine A, phalloïdine, swinholide A, indanocine, myoservine, facteur de nécrose tumorale alpha (TNFalpha), interleukine-1 bêta, interféron gamma, GalNAc, galactose, mannose, mannose-6P, agrégats de sucres comme un agrégat de GalNAc, un agrégat de mannose, un agrégat de galactose, un aptamère, ligands de récepteur d'intégrine, ligands de récepteur de chimiokine, ligands de récepteur de sérotonine, PSMA, endothéline, GCPII, somatostatine, et toute combinaison de ceux-ci ; et/ou
(iii) SEQ ID NO: 1 (CREKA), SEQ ID NO: 2 (CRKRLDRNK), SEQ ID NO: 3 (CHVLWSTRC), SEQ ID NO: 4 (ALEALAEALEALAEA), SEQ ID NO: 5 (KFFKFFKFFK (peptide à perméation de paroi cellulaire bactérienne)), SEQ ID NO: 6 (AALEALAEALEALAEALEALAEAAAAGGC (GALA)), SEQ ID NO: 7 (ALAEALAEALAEALAEALAEALAAAAGGC (EALA)), SEQ ID NO: 8 (GLFEAIEGFIENGWEGMIWDYG (INF-7)), SEQ ID NO: 9 (GLFGAIAGFIENGWEGMIDGWYG (Inf HA-2)), SEQ ID NO: 10 (GLF EAI EGFI ENGW EGMI DGWYGC GLF EAI EGFI ENGW EGMI DGWYGC (diINF-7)), SEQ ID NO: 11 (GLF EAI EGFI ENGW EGMI DGGC GLF EAI EGFI ENGW EGMI DGGC (diINF-3)), SEQ ID NO: 12 (GLFGALAEALAEALAEHLAEALAEALEALAAGGSC (GLF)), SEQ ID NO: 13 (GLFEAIEGFIENGWEGLAEALAEALEALAAGGSC (GALA-INF3)), SEQ ID NO: 14 (GLF EAI EGFI ENGW EGnI DG K GLF EAI EGFI ENGW EGnI DG (INF-5, n est la norleucine)), SEQ ID NO: 15 (RQIKIWFQNRRMKWKK (pénétratine)), SEQ ID NO: 16 (GRKKRRQRRRPPQC (fragment Tat 48-60)), SEQ ID NO: 17 (GALFLGWLGAAGSTMGAWSQPKKKRKV (peptide à base d'une séquence de signal)), SEQ ID NO: 18 (LLIILRRRIRKQAHAHSK (PVEC)), SEQ ID NO: 19 (WTLNSAGYLLKINLKALAALAKKIL (transportan)), SEQ ID NO: 20 (KLALKLALKALKAALKLA (peptide modèle amphilique)), SEQ ID NO: 21 (RRRRRRRRR (Arg9)), SEQ ID NO: 22 (LLGDFFRKSKEKIGKEFKRIVQRIKDFLRNLVPRTES (LL-37)), SEQ ID NO: 23 (SWLSKTAKKLENSAKKRISEGIAIAIQGGPR (cécropine P1)), SEQ ID NO: 24 (ACYCRIPACIAGERRYGTCIYQGRLWAFCC (α-défensine)), SEQ ID NO: 25 (DHYNCVSSGGQCLYSACPIFTKIQGTCYRGKAKCCK (β-défensine)), SEQ ID NO: 26 (RRRPRPPYLPRPRPPPFFPPRLPPRIPPGFPPRFPPRFPGKR-NH2 (PR-39)), SEQ ID NO: 27 ILPWKWPWWPWRR-NH2 (indolicidine)), SEQ ID NO: 28 (AAVALLPAVLLALLAP (RFGF)), SEQ ID NO: 29 (AALLPVLLAAP (analogue de RFGF)), SEQ ID NO: 30 (RKCRIVVIRVCR (bacténécine)), cécropines, lycotoxines, paradaxines, buforine, CPF, peptide de type bombinine (BLP), cathélicidines, cératotoxines, peptides *S. clava,* peptides antimicrobiens intestinaux de myxine (HFIAP), magainines, brevinines-2, dermaseptines, mélittines, pleurocidine, peptides H₂A, peptides Xenopus, esculentinis-1, caérines et tout analogue et dérivé de ceux-ci.

10. Composition pharmaceutique pour utilisation selon la revendication 9, ou utilisation de la composition pharmaceutique selon la revendication 9, dans laquelle le ligand réduit le taux de désagrégation d'au moins 10 % par rapport à un témoin, et/ou l'agrégat se désagrège d'au moins 5 % dans une région de sténose par rapport à une région de non-sténose.

11. Composition pharmaceutique pour utilisation selon l'une quelconque des revendications 1, 3, 4, 5, 7, 8, 9 ou 10, ou utilisation de la composition pharmaceutique selon l'une quelconque des revendications 2, 3, 4, 6, 8, 9 ou 10, dans laquelle une surface des nanoparticules est modifiée pour moduler les interactions électrostatiques intermoléculaires, les interactions de liaison hydrogène, les interactions dipôle-dipôle, les interactions hydrophiles, les interactions hydrophobes, les forces de van der Waal et toute combinaison de celles-ci entre deux ou plusieurs particules.

12. Composition pharmaceutique pour utilisation selon l'une quelconque des revendications 1, 3, 4, 5, 7, 8, 9, 10 ou 11, ou utilisation de la composition pharmaceutique selon l'une quelconque des revendications 2, 3, 4, 6, 8, 9, 10 ou 11, dans laquelle l'agrégat est d'une taille de ≤ 50 µm, de préférence dans laquelle la taille de l'agrégat varie de 1 µm à 20 µm.

13. Composition pharmaceutique pour utilisation selon l'une quelconque des revendications 1, 3, 4, 5, 7, 8, 9, 10, 11 ou 12, ou utilisation de la composition pharmaceutique selon l'une quelconque des revendications 2, 3, 4, 6, 8, 9, 10, 11 ou 12, dans laquelle la nanoparticule comprend au moins une fraction qui augmente la durée de vie *in vivo* de l'agrégat, de préférence la au moins une fraction est du polyéthylèneglycol.

14. Composition pharmaceutique pour utilisation selon l'une quelconque des revendications 1, 3, 4, 5, 7, 8, 9, 10, 11, 12 ou 13, ou utilisation de la composition pharmaceutique selon l'une quelconque des revendications 2, 3, 4, 6, 8, 9, 10, 11, 12 ou 13, dans laquelle la sténose, la lésion sténosée ou occlusive est sélectionnée parmi le groupe constitué de : claudication intermittente (sténose de l'artère périphérique), angine (sténose de l'artère coronaire) ou infarctus du myocarde, sténose de l'artère carotide, sténose aortique, sténose de la boutonnière, sténose nodulaire calcique, sténose ostiale coronarienne, sténose aortique double, sténose mitrale en bouche de poisson, sténose sous-aortique hypertrophique idiopathique, sténose infundibulaire, sténose mitrale, sténose sous-aortique musculaire, sténose pulmonaire, embolisme pulmonaire, hypertension pulmonaire, sténose sous-aortique, sténose sous-valvulaire, sténose supra-valvulaire, sténose tricuspide, sténose de l'artère rénale, hypertension vasculaire, drépanocytose, et toute combinaison de ceux-ci ; et/ou résultats d'un(e) : traumatisme ou blessure, athérosclérose, anomalies congénitales, diabètes, iatrogène, infection, inflammation, ischémie, néoplasme, vasospasme, vasospasme coronaire, phénomène de Raynaud, accident vasculaire cérébral, coagulation du sang, maladie de Moya-moya, maladie de Takayasu, périartérite noueuse, lupus érythémateux disséminé, arthrite rhumatoïde, tumeurs de la colonne, maladie osseuse de Paget, fluorose, hémodialyse et toute combinaison de ceux-ci.

15. Composition pharmaceutique pour utilisation selon l'une quelconque des revendications 1, 3, 4, 5, 7, 8, 9, 10, 11, 12, 13 ou 14, ou utilisation de la composition pharmaceutique selon l'une quelconque des revendications 2, 3, 4, 6, 8, 9, 10, 11, 12, 13 ou 14 en combinaison avec des traitements d'embolisation.

16. Composition pharmaceutique pour utilisation selon la revendication 1, dans laquelle le vasodilatateur est sélectionné parmi le groupe constitué de : prazosine, térazosine, doxazosine, trimazosine, phentolamine, phénoxybenzamine, bénazépril, captopril, énalapril, fosinopril, lisinopril, moexipril, quinapril, ramipril, candésartan, éprosartan, irbesartan, losartan, olmésartan, telmisartan, valsartan, épinéphrine, norépinéphrine, dopamine, dobutamine, isoprotérénol, amlodipine, félodipine, isradipine, nicardipine, nifédipine, nimodipine, nitrendipine, clonidine, guanabenz, guanfacine, α-méthyldopa, hydralazine, bosentan, camsylate de triméthaphan camsylate, dinitrate d'isosorbide, mononitrate d'isosorbide, nitroglycérine, tétranitrate d'érythrityle, tétranitrate de pentaérythritol, nitroprussiate de sodium, milrinone, inamrinone (anciennement amrinone), cilostazol, sildénafil, tadalafil, minoxidil, aliskirène et des sels pharmaceutiquement acceptables de ceux-ci.

17. Composition pharmaceutique pour utilisation selon la revendication 16, dans laquelle le vasodilatateur est la nitroglycérine.
